(19) Europäisches Patentamt
European Patent Office
Office européen des brevets

(11) EP 2 794 909 B1

(12) **EUROPÄISCHE PATENTSCHRIFT**

(45) Veröffentlichungstag und Bekanntmachung des Hinweises auf die Patenterteilung:
**16.01.2019 Patentblatt 2019/03**

(21) Anmeldenummer: **12805708.0**

(22) Anmeldetag: **20.12.2012**

(51) Int Cl.:
***C12Q 1/54*** *(2006.01)* ***G01N 33/52*** *(2006.01)*
***A61B 5/145*** *(2006.01)*

(86) Internationale Anmeldenummer:
**PCT/EP2012/076361**

(87) Internationale Veröffentlichungsnummer:
**WO 2013/092845 (27.06.2013 Gazette 2013/26)**

# (54) VERFAHREN ZUR BESTIMMUNG EINER ANALYTKONZENTRATION

METHOD FOR DETERMINING THE CONCENTRATION OF AN ANALYTE

PROCÉDÉ DE DÉTERMINATION D'UNE CONCENTRATION D'ANALYTE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **22.12.2011 EP 11195083**

(43) Veröffentlichungstag der Anmeldung:
**29.10.2014 Patentblatt 2014/44**

(73) Patentinhaber:
- **Roche Diabetes Care GmbH**
  **68305 Mannheim (DE)**
  Benannte Vertragsstaaten:
  **DE**
- **F. Hoffmann-La Roche AG**
  **4070 Basel (CH)**
  Benannte Vertragsstaaten:
  **AL AT BE BG CH CY CZ DK EE ES FI FR GB GR HR HU IE IS IT LI LT LU LV MC MK MT NL NO PL PT RO RS SE SI SK SM TR**

(72) Erfinder:
- **HORN, Carina**
  **68647 Biblis (DE)**
- **UNKRIG, Volker**
  **68526 Ladenburg (DE)**

(74) Vertreter: **Herzog, Fiesser & Partner**
**Patentanwälte PartG mbB**
**Dudenstrasse 46**
**68167 Mannheim (DE)**

(56) Entgegenhaltungen:
**US-A- 4 250 257**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 794 909 B1

Printed by Jouve, 75001 PARIS (FR)

## Beschreibung

Gebiet der Erfindung

Die Erfindung betrifft ein Verfahren, eine Vorrichtung und ein Testelement zum Nachweis mindestens eines Analyten in einer Probe. Derartige Verfahren, Vorrichtungen und Testelemente werden insbesondere zum Nachweis eines oder mehrerer Analyten wie beispielsweise Metaboliten in Körperflüssigkeiten eingesetzt. Ein Schwerpunkt der folgenden Erfindung liegt auf dem Nachweis von Glucose in einer Körperflüssigkeit, insbesondere Blut und/oder interstitieller Flüssigkeit. Grundsätzlich sind jedoch auch andere Arten von Proben einsetzbar, beispielsweise andere Arten von Körperflüssigkeiten, wie Urin oder Speichel, sowie andere Arten von Analyten, wie Cholesterin, Lactat oder ähnliches. Neben einem Einsatzgebiet in der medizinischen Diagnostik ist grundsätzlich der Einsatz der erfindungsgemäßen Verfahren, Vorrichtungen und Testelemente in anderen Bereichen möglich, beispielsweise in anderen Bereichen der Naturwissenschaften und Technik, insbesondere in der chemischen Analytik.

Stand der Technik

In vielen Bereichen der Naturwissenschaften und Technik müssen zuverlässig und schnell ein oder mehrere Analyte qualitativ und/oder quantitativ in einer Probe, beispielsweise einer flüssigen und/oder gasförmigen Probe, nachgewiesen werden. Ein Schwerpunkt der vorliegenden Erfindung, auf welche die Erfindung jedoch nicht beschränkt ist, liegt auf Analysen in der medizinischen Diagnostik, insbesondere in der Diabetesvorsorge und der Diabetesbehandlung. Im Rahmen einer Diabetesvorsorge und/oder einer Diabetesbehandlung ist es in der Regel erforderlich, den Blutzuckergehalt mindestens einmal am Tag, in der Regel mehrfach, schnell und zuverlässig zu bestimmen, um bei Abweichungen vom Normwert bzw. einem Normbereich entsprechende Gegenmaßnahmen ergreifen zu können. Um den Tagesablauf des Patienten durch diese Messungen nicht mehr als notwendig zu beeinträchtigen, wurden Vorrichtungen und Verfahren entwickelt, welche Blutzuckerbestimmungen nicht nur in einem klinischen Umfeld, sondern beispielsweise auch am Arbeitsplatz, zu Hause oder bei Freizeitbeschäftigungen ermöglichen. Daneben sind auch Vorrichtungen und Verfahren bekannt, welche im klinischen Umfeld eingesetzt werden. Derartige Vorrichtungen und Verfahren beruhen in der Regel auf der Verwendung eines oder mehrerer Testelemente. Diese Testelemente sind in unterschiedlichen Formen bekannt und erhältlich, auf welche die vorliegende Erfindung insgesamt anwendbar, jedoch nicht eingeschränkt ist. So sind beispielsweise Testelemente in Form von Teststreifen, Testbändern, Testscheiben, Testnadeln oder in anderen Formen bekannt. Im Folgenden wird die Erfindung im Wesentlichen unter Bezugnahme auf Teststreifen beschrieben, wobei jedoch auch andere Ausgestaltungen grundsätzlich möglich sind.

Testelemente umfassen in der Regel ein oder mehrere Testfelder mit mindestens einem analytspezifischen Nachweisreagens, welches im Folgenden auch als Testchemie bezeichnet wird. Dieses Nachweisreagens ist ausgewählt und ausgestaltet, um bei Anwesenheit des nachzuweisenden Analyten eine nachweisbare Reaktion durchzuführen, vorzugsweise eine Analyt-spezifische Reaktion. Unter einer nachweisbaren Reaktion wird dabei im Folgenden eine Reaktion verstanden, welche mittels mindestens eines physikalischen und/oder chemischen Nachweisverfahrens detektierbar ist, vorzugsweise mittels eines physikalischen Nachweisverfahrens. Beispielsweise sind Reaktionen bekannt, die auf optischem und/oder elektrochemischem Wege nachgewiesen werden können. Beispielsweise lassen sich dabei Reaktionen einsetzen, bei denen mindestens ein Nachweisstoff gebildet wird, welcher optisch und/oder elektrochemisch nachgewiesen werden kann. Solche Testelemente sind beispielsweise unter dem Namen Accu Chek® Aviva, Accu Chek® Performa, Accu Chek® Active, Accu Chek® Go oder Accu Chek® Mobile Testcassette mit geeigneten Testgeräten wie Accu Chek® Aviva, Active, Go und Mobile von Roche Diagnostics GmbH erhältlich.

So beschreibt beispielsweise die WO 2010/052306 ein diagnostisches Testelement zum Nachweis eines Analyten in einer Probe einer Körperflüssigkeit, wobei ein Testfeld ein Nachweisreagens aufweist, welches dazu eingerichtet ist, um bei Anwesenheit des Analyten eine nachweisbare Veränderung zu durchlaufen, insbesondere eine optische Veränderung. Das Testfeld weist mindestens eine das Nachweisreagens umfassende Nachweisschicht auf, welche Partikel aufweist, wobei mindestens 90 % sämtlicher Partikel der Nachweisschicht eine tatsächliche Partikelgröße von weniger als 10 $\mu$m aufweisen.

In WO 2010/052307 wird ein Testelement zum Nachweis eines Analyten in einer Probe beschrieben. Das Testelement umfasst mindestens ein Testfeld mit einer Testfeldoberfläche, wobei das Testfeld ein Nachweisreagens aufweist, welches eingerichtet ist, um bei Anwesenheit des Analyten eine nachweisbare Reaktion durchzuführen. Weiterhin weist das Testelement mindestens ein Verteilerelement auf, welches mindestens eine der Testfeldoberfläche zuweisende Verteileroberfläche aufweist, wobei zwischen der Verteileroberfläche und der Testfeldoberfläche mindestens ein Kapillarspalt ausgebildet ist.

In Testelementen können beispielsweise ein oder mehrere verschiedene Testchemien, insbesondere Enzymsysteme, eingesetzt werden, die den Analyten, insbesondere Glucose, umsetzen. Unter einer Umsetzung ist dabei eine Reaktion zu verstehen, an welcher der nachzuweisende Analyt beteiligt ist und/oder bei welcher der nachzuweisende

Analyt chemisch verändert wird. Dabei kann die Testchemie die Spezifität des Testelements sicherstellen. Beispielsweise kann die Detektion über eine Testchemie erfolgen, die ein Enzym sowie ein Coenzym enthält, indem beispielsweise von der Glucose übertragenen Redoxäquivalente auf das Coenzym von einem Mediator, wie beispielsweise Braunstein, übertragen werden. Dieses Redoxäquivalent kann beispielsweise für photometrische und/oder optische Konzentrationsmessungen des Analyten mit einem Indikator umgesetzt werden.

Um für den Benutzer eine handhabbare Form der Testchemie, insbesondere des vorbeschriebenen Enzymsystems bereitzustellen, wird beispielsweise das Enzymsystem als mindestens ein Teil der Testchemie ggf. samt der weiteren benötigten Reaktivstoffe, wie beispielsweise einem Mediator, in Schichten, die meist trocken und fest sind, auf dem Testelement eingebracht oder immobilisiert, so dass ein Benutzer nur noch seine Probe auf die Schicht auftragen muss, um kurze Zeit danach ein Messergebnis zu erhalten.

Nachdem die Probe, beispielsweise das Blut, auf die Schicht des Testelementes aufgetragen wird, kann die Analyt-spezifische Nachweisreaktion sowohl in der Schicht ablaufen als auch ganz oder teilweise in der Probe. So können sich beispielsweise ein oder mehrere Reaktivstoffe, beispielsweise in Form des Nachweisreagens oder Bestandteilen desselben, in der Probe lösen, was beispielsweise bis zu einem Endpunkt der Reaktion beobachtet werden kann. Bei diesen Vorgängen können beispielsweise auch Diffusionsprozesse beteiligt sein, bei denen die flüssige Probe den Analyten beispielsweise zu den Reaktivstoffen transportiert und/oder der gebildete Nachweisstoff in eine oder aus einer Nachweisschicht diffundiert. Beispielsweise kann der Endpunkt der Reaktion in photometrischen oder elektrochemischen Systemen bestimmt werden. Hierbei kann die Geschwindigkeit der Bildung beispielsweise eines Nachweisstoffes gleich der Geschwindigkeit des Diffundierens des gebildeten Nachweisstoffes sein. Alternativ kann zu einem bestimmten Zeitpunkt nach Aufgabe der Probe auf das Testfeld ein Messsignal bestimmt werden, aus dem die Konzentration des Analyten abgeleitet werden kann.

Solche Diffusionsprozesse, wie auch enzymkatalysierte Reaktionen, können temperaturabhängig sein. Weiterhin können weitere Bestandteile der Probe, wie beispielsweise zelluläre und/oder partikuläre Bestandteile, wie Hämatokrit, einen Einfluss auf die Diffusions- und Auflöseprozesse in dem Teststreifen haben. Hieraus kann eine starke Abhängigkeit der ermittelten Analytkonzentration von der Umgebungstemperatur, bei der die Messung durchgeführt wird, sowie dem Hämatokrit der Probe, welche auf das Testelement aufgetragen wird, resultieren. So könnte grundsätzlich die Gefahr bestehen, dass Analytwerte beeinflusst werden, was wiederum zu einer Herausforderung bei Dosierungen von Medikamenten wie beispielsweise Insulin führen kann. Besonders in niedrigen Konzentrationsbereichen der Blutglucose bei einem Diabetiker kann ein fälschlich zu hoch gemessener Blutglucosewert dazu führen, dass zu viel Insulin verabreicht wird. Daher kann es sehr wichtig sein, die Temperaturabhängigkeit und/oder Hämatokritabhängigkeit des jeweiligen Testelementsystems zu korrigieren.

Beispielsweise kann die Temperatur durch einen Sensor, der im Messgerät untergebracht ist, gemessen und zur Korrektur herangezogen werden. Dies kann jedoch wiederum zu fehlerhaften Korrekturen führen, da in der Regel die Temperatur nicht an der eigentlichen Position der chemischen Umsetzung des Analyten gemessen wird. Folglich kann die zur Korrektur herangezogene Temperatur sich deutlich von der Temperatur des Umsatzortes des Analyten unterscheiden, so dass diese Korrektur wiederum zu Fehlern führen kann.

Des Weiteren ist es insbesondere bei photometrischen Systemen schwierig, eine Korrektur des Hämatokrits durchzuführen, da in der Regel keine rein hämatokritabhängigen Messwerte, die zur Korrektur des Analytmesssignals herangezogen werden könnten, existieren. Bei elektrochemischen Messverfahren ist eine Korrektur des Messsignals bezüglich des Hämatokrits zwar prinzipiell möglich, dies ist jedoch aufgrund eines aufwändigen Pulssequenzverfahrens sehr umständlich und ist weiterhin überlagert von anderen Störsignalen in der Probe.

In US 4,250,257 A werden Verfahren und Vorrichtungen zur Analyse von Vollblut-Proben beschrieben. Dabei wird ein Gel verwendet, in welchem eine inerte Substanz aufgenommen ist. Diese inerte Substanz diffundiert aus dem Gel heraus in die Vollblut-Probe, während das Plasma der Vollblut-Probe in das Gel hineindiffundiert. Die Diffusion der inerten Substanz aus dem Gel heraus ist umgekehrt proportional zum Hämatokrit der Blutprobe. Weiterhin werden verschiedene Möglichkeiten offenbart, eine Hämatokritkorrektur zu bewirken: Zum einen kann mittels eines separaten Gels ein Hämatokrit und daraus ein Korrekturfaktor bestimmt werden, welcher anschließend für eine andere Blutprobe verwendet wird. Alternativ können eine Analyt-Nachweisreaktion und eine Hämatokritkorrektur auch mittels zweier unterschiedlicher Farbänderungen durchgeführt werden, wobei eine Farbänderung aus der Analyt-Nachweisreaktion resultiert und eine zweite Farbänderung aus der Diffusion eines Farbstoffs als inertem Medium in die Probe hinein. Der inerte Indikator kann also insbesondere ein Farbstoff sein, wobei beispielsweise Vitamin B-12 vorgeschlagen wird. Weiterhin wird eine Hämatokritkorrektur mittels Albumin als Assay beschrieben. Als Nachweissubstanz wird Bromcresol-Grün (BCG) vorgeschlagen.

In der US 7,548,773 wird die Möglichkeit einer Kalibration eines Messsystems beschrieben, das auf der Auflösung von Analyt in einem Referenzkanal beruht. Durch die Auflösung einer bekannten Menge Analyts durch Probe in dem Referenzkanal, kann parallel zur eigentlichen Analytbestimmung eine Differenzbestimmung zum Referenzkanal durchgeführt werden, um die Reaktionsgeschwindigkeit der Probe zu bestimmen. Hierdurch können Korrekturen an der Analytbestimmung vorgenommen werden. Nachteil dieser Methode ist jedoch die Notwendigkeit eines Lösungsprozesses eines zu bestimmenden Referenzmoleküls an einer Kapillarwand. Dieser kann auch von Eigenschaften des Systems beeinflusst werden, die nicht mit der Probe zusammen hängen. Darüber hinaus ist die Auflösung und Bewegung des analytidentischen Kalibrators von der Analytkonzentration abhängig. Eine Unterscheidung von Einflüssen der Analytkonzentration und anderer Faktoren auf die Bewegung des Kalibrators ist nicht möglich, was wiederum zu einer Verfälschung des bestimmten Analytgehaltes führen kann.

Aufgabe der Erfindung

Es ist daher eine Aufgabe der vorliegenden Erfindung, ein Verfahren, eine Vorrichtung und ein Testelement zum Nachweis mindestens eines Analyten in einer Probe bereitzustellen, welche die Nachteile bekannter Verfahren, Vorrichtungen und Testelemente zumindest teilweise vermeiden. Insbesondere sollen ein Verfahren, eine Vorrichtung sowie ein Testelement bereitgestellt werden, die eine Verfälschung des Messergebnisses durch Diffusionseffekte, auch bei unterschiedlichen Temperaturen der Probe oder des Testelements, zumindest teilweise vermeiden. Eine weitere Aufgabe ist es, ein Verfahren, eine Vorrichtung und ein Testelement zum Nachweis mindestens eines Analyten in einer Probe bereitzustellen, die einen vom Analyten unabhängigen Nachweis ermöglichen.

Beschreibung der Erfindung

Diese Aufgabe wird durch ein Verfahren, eine Vorrichtung sowie ein Testelement mit den Merkmalen der unabhängigen Ansprüche gelöst. Vorteilhafte Weiterbildungen der Erfindung, welche einzeln oder in Kombination realisiert werden können, sind in den abhängigen Ansprüchen dargestellt. Die im Folgenden beschriebene Vorrichtung kann insbesondere eingerichtet sein, um ein erfindungsgemäßes Testelement gemäß einer oder mehrerer der im Folgenden beschriebenen Ausführungsformen zu verwenden und/oder um ein Verfahren gemäß einer oder mehreren der im Folgenden beschriebenen Ausführungsformen durchzuführen. Weiterhin kann das Verfahren unter Verwendung einer erfindungsgemäßen Vorrichtung und/oder eines erfindungsgemäßen Testelements in einer oder mehreren der beschriebenen Ausführungsformen durchgeführt werden. Insofern kann für mögliche Ausgestaltungen der Vorrichtung und/oder des Testelements auf die Beschreibung des Verfahrens verwiesen werden und umgekehrt.

In einem ersten Aspekt der Erfindung wird ein Verfahren zur Bestimmung mindestens einer Konzentration mindestens eines Analyten in einer Probe vorgeschlagen. Bei der Probe kann es sich insbesondere um eine flüssige Probe handeln, insbesondere um eine Probe einer Körperflüssigkeit, wie beispielsweise Blut oder interstitielle Flüssigkeit. Das Verfahren kann insbesondere zum Nachweis mindestens eines Metaboliten in einer Probe einer Körperflüssigkeit verwendet werden.

In dem Verfahren wird mindestens ein Testelement mit mindestens einer Testchemie verwendet. Mindestens eine Reaktion der Testchemie mit dem Analyten wird detektiert. Weiterhin wird mindestens ein Marker verwendet, wobei mindestens eine Diffusion des Markers in der Probe oder mindestens einem Teil der Probe detektiert wird. Aus der Diffusion wird mindestens eine Korrekturinformation erzeugt, wobei aus der Reaktion der Testchemie mit dem Analyten unter Berücksichtigung der Korrekturinformation die Konzentration des Analyten bestimmt werden kann.

Die Testchemie befindet sich beispielsweise in einem Testfeld, das auf einem Testträger des Testelementes angebracht ist. Dabei ist unter dem Begriff Testchemie eine Substanz und/oder ein Substanzgemisch zu verstehen, das dazu eingerichtet ist, bei Anwesenheit des nachzuweisenden Analyten mindestens eine detektierbare Veränderung mindestens einer Eigenschaft durchzuführen. Insbesondere kann die Testchemie mindestens ein Reagens enthalten, das den Analyten in einer Nachweisreaktion umsetzt. Beispielsweise kann das Nachweisreagens mindestens ein enzymatisches Nachweisreagens umfassen. Als Beispiele derartiger analytspezifischer enzymatischer Nachweisreagenzien sind Oxireduktase-Enzyme (z.B. GlucDor/PQQ), Dehydrogenase-Enzyme, Oxidase-Enzyme oder ähnliche Enzyme oder Kombinationen der genannten und/oder anderer Enzyme, insbesondere Glucoseoxidase (GOD) oder Glucosedehydrogenase (beispielsweise FAD-, $NAD^+$- oder PQQ-abhängig) zu nennen. Bei der mindestens einen nachweisbaren Reaktion kann es sich insbesondere um eine optisch und/oder elektrochemisch nachweisbare Reaktion handeln. Auch andere Arten von Reaktionen sind jedoch grundsätzlich möglich. Insbesondere kann es sich um eine Reaktion handeln, bei welcher bei Anwesenheit des mindestens einen Analyten mindestens ein Nachweisstoff gebildet wird. Dabei können auch mehrere Nachweisstoffe gebildet und/oder verwendet werden, die einzeln, in Gruppen oder alle nachgewiesen werden können. Nachweisstoffe sind insbesondere Stoffe, die sich aufgrund der mindestens einen Nachweisreaktion bilden und/oder die an der mindestens einen Nachweisreaktion beteiligt sind und die direkt oder indirekt nachweisbar sind. Anhand des nachgewiesenen mindestens einen Nachweisstoffs kann beispielsweise der mindestens eine Analyt quantitativ und/oder qualitativ nachgewiesen werden. Für Beispiele für Ausformungen der Testchemie bzw. des Nachweisstoffes kann auf die WO 2010/052307 verwiesen werden.

Die Testchemie mit dem Nachweisreagens kann sich insbesondere in mindestens einer Testchemieschicht oder Nachweisschicht befinden. Die Begriffe der Testchemieschicht und der Nachweisschicht werden im Folgenden synonym verwendet. Die mindestens eine Testchemieschicht kann optional noch weitere Substanzen beinhalten, beispielsweise einen oder mehrere Füllstoffe, vorzugsweise umfassend eine oder mehrere Arten von Partikeln, beispielsweise anorganische Partikel. Die Partikel sind vorzugsweise nicht identisch mit dem Nachweisreagens oder zumindest nicht vollständig identisch mit dem Nachweisreagens. Das Nachweisreagens kann grundsätzlich auch eine Mischung mehrerer Nachweisreagenzien oder mehrere Stoffe umfassen, die zusammen das Nachweisreagens bilden können. Die Testchemieschicht kann beispielsweise analog zu der in EP 0 821 234 B1 beschriebenen, ersten Filmschicht des diagnostischen Testträgers ausgestaltet sein. Somit kann die Nachweisschicht bzw. die Testchemieschicht beispielsweise mindestens einen organischen Filmbildner umfassen. Beispielsweise kann dieser mindestens eine Filmbildner eine Polyvinylproprionat-Dispersion umfassen. Alternativ oder zusätzlich können jedoch auch anderer Filmbildner eingesetzt werden. Auch andere Aufbauten der Testchemieschicht sind jedoch grundsätzlich möglich.

Unter einer Detektion einer Reaktion der Testchemie mit dem Analyten ist allgemein ein Vorgang zu verstehen, bei welchem die Reaktion selbst und/oder eine oder mehrere an der Reaktion beteiligte Reaktanden und/oder eine oder mehrere Reaktionsprodukte qualitativ oder quantitativ erfasst werden, beispielsweise durch qualitative und/oder quantitative Erfassung mindestens einer Eigenschaftsänderung der Probe und/oder der Testchemie und/oder mindestens eines weiteren Elements oder Bereichs, welcher durch die Nachweisreaktion beeinflusst wird. Beispielsweise kann eine Detektion der Reaktion der Testchemie mit dem Analyten mittels elektrochemischer und/oder optischer Nachweisverfahren erfolgen. Derartige Nachweisverfahren, welche beispielsweise unter Verwendung mindestens eines Detektors durchgeführt werden können, sind dem Fachmann grundsätzlich bekannt, beispielsweise aus dem oben genannten Stand der Technik.

Bei dem Verfahren wird weiterhin, wie oben ausgeführt, mindestens ein Marker verwendet und mindestens eine Diffusion des Markers in der Probe oder in mindestens einem Teil der Probe detektiert. Unter einem Marker ist im Rahmen der vorliegenden Erfindung eine Substanz zu verstehen, die durch optische und/oder elektrochemische Nachweisverfahren zumindest lokal bestimmbar ist, beispielsweise indem mindestens eine Konzentration des Markers, beispielsweise eine lokale Konzentration, elektrochemisch und/oder optisch erfasst wird. Der mindestens eine Marker kann insbesondere eine nachweisbare chemische Substanz umfassen, welche beispielsweise optisch und/oder elektrochemisch nachgewiesen werden kann. Dieser mindestens eine Marker ist von dem mindestens einen nachzuweisenden Analyten verschieden, beispielsweise chemisch verschieden. Chemisch verschieden kann beispielsweise bedeuten, dass das einzelne Molekül des Markers durch mindestens ein Atom von dem Analytmolekül abweicht. So kann das Molekül des Markers mindestens ein Atom mehr oder weniger als der Analyt aufweisen. Alternativ kann chemisch verschieden jedoch auch bedeuten, dass die Anzahl und Abfolge der Atome in dem Markermolekül gleich zu der Abfolge der Atome im Analytmolekül sind, jedoch die räumliche Anordnung der Atome nicht an jeder Stelle identisch sind, wie dies beispielsweise bei Verbindungen mit asymmetrischen Kohlenstoffatomen vorkommt, die beispielsweise zu zwei Enantiomeren eines gleichen Moleküls führen. So könnten zwei Enantiomere verwendet werden, wovon das eine Enantiomer den Analyt darstellt und das andere Enantiomer den Marker. Unter einem Marker, welcher von den nachzuweisenden Analyten verschieden ist, wird dabei ein Marker im Sinne der obigen Definition verstanden, welcher zumindest teilweise nichtidentisch mit dem nachzuweisenden Analyten ist, beispielsweise indem der Marker mindestens eine chemische Substanz, beispielsweise mindestens eine chemische Verbindung, aufweist, welche nicht in dem nachzuweisenden Analyten enthalten ist. Beispielsweise kann der Marker mindestens eine optisch nachweisbare Substanz umfassen, beispielsweise mindestens einen Farbstoff, beispielsweise mindestens einen anorganischen oder organischen Farbstoff. Unter einem Farbstoff ist dabei allgemein eine Substanz, beispielsweise eine chemische Verbindung, zu verstehen, welche mindestens eine nachweisbare Wechselwirkung mit Licht im ultravioletten und/oder sichtbaren und/oder infraroten Spektralbereich durchführen kann, beispielsweise eine Reflexion unter Veränderung einer Wellenlänge und/oder unter Veränderung spektraler Eigenschaften des reflektierten Lichts, eine Streuung unter Veränderung einer Wellenlänge und/oder unter Veränderung spektraler Eigenschaften des gestreuten Lichts, eine Fluoreszenz, eine Phosphoreszenz oder eine Kombination der genannten Wechselwirkungen.

Unter lokal ist eine definierte Fläche oder ein definiertes Volumen des Testfeldes zu verstehen, das zum Nachweis des Markers herangezogen wird. Beispielsweise kann bei einer optischen Detektion des Markers diese Fläche bzw. das Volumen durch die Form des eingestrahlten Lichts definiert werden oder durch die Positionierung des Detektors. Bei einer elektrochemischen Detektion kann beispielsweise diese Fläche bzw. das Volumen durch die Form und Anordnung der Elektrode definiert werden. Insbesondere kann es sich bei dem Marker um einen Farbstoff handeln und/oder kann der Marker mindestens einen derartigen Farbstoff umfassen, dessen Diffusionsverhalten beispielsweise durch optische bzw. elektrochemische Nachweisverfahren ermittelt werden kann, beispielsweise in dem Testelement und/oder der Probe und/oder mindestens einem Teil der Probe. Der Marker ist bevorzugt verschieden von dem zu bestimmenden Analyten. Der Marker befindet sich vorzugsweise vor einer Benetzung des Testelementes bzw. des Markers mit Probe, und/oder vor einer analytspezifischen Nachweisreaktion auf oder/und in dem Testelement, bevorzugt in mindestens einem Testfeld des Testelementes. Unter einem Testfeld wird dabei ein Element verstanden, welches eine zusammenhängende Menge der Testchemie umfasst, beispielsweise mindestens eine Schicht der Testchemie sowie optional einer oder mehrerer weiterer Komponenten. Alternativ oder zusätzlich zu einer Ausgestaltung des Verfahrens, bei welcher der Marker zumindest vor einem Kontaktieren des Testelements und/oder der Testchemie in dem Testelement enthalten

ist, kann der Marker jedoch auch zumindest vor einem Kontaktieren des Testelements und/oder der Testchemie ganz oder teilweise außerhalb des Testelementes angeordnet sein. Der Marker ist vorzugsweise so ausgestaltet, dass bei einem Kontaktieren des Testelements mit der Probe, beispielsweise bei einer Benetzung des Testelementes und/oder des Markers mit Probe, mindestens eine Diffusion des Markers in der Probe oder mindestens einem Bestandteil der Probe stattfindet, welche detektiert werden kann. Die Diffusion kann beispielsweise in einem Überstand der Probe, welcher nicht von dem Testelement und/oder der Testchemie aufgenommen wurde, beispielsweise einem tropfenförmigen Überstand, und/oder in einem Teil der Probe stattfinden, welcher von dem Testelement oder einem Teil desselben aufgenommen wurde.

Insbesondere kann die Diffusion des Markers aus mindestens einem ersten Bereich in mindestens einen zweiten Bereich detektiert werden. Der erste und/oder zweite Bereich können jeweils Bereiche des Testelementes sein. Alternativ können der erste und/oder der zweite Bereich auch separat vom Testelement, beispielsweise in oder auf einem Korrekturelement, mit gleichem oder verschiedenem Aufbau wie das Testelement, angeordnet sein. Alternativ oder zusätzlich kann der zweite Bereich sich außerhalb des Testelements befinden, wie beispielsweise in der Probe und/oder einem Teil der Probe, der nicht in das Testelement aufgenommen wird, sondern beispielsweise als Überstand über dem Testelement verbleibt. Alternativ oder zusätzlich kann der erste und/oder zweite Bereich separat vom Testelement angeordnet sein, wobei einer der beiden Bereiche Bestandteil des Testelementes ist. Es ergibt sich somit eine Vielzahl von Möglichkeiten, die Diffusion nachzuweisen, beispielsweise indem lokal eine Konzentrationsänderung beobachtet wird.

Die Diffusion des Markers in der Probe oder mindestens einem Bestandteil desselben wird beispielsweise durch Konzentrationsunterschiede des Markers, beispielsweise zwischen dem ersten und zweiten Bereich hervorgerufen. So kann beispielsweise durch die Benetzung des Testelementes und/oder des Markers ein Konzentrationsunterschied zwischen mindestens einem ersten Bereich, in dem sich der Marker vor der Benetzung befindet, und mindestens einem zweiten Bereich, beispielsweise einem Überstand der Probe und/oder einem von dem ersten Bereich verschiedenen Bereich des Testelements und/oder eines Korrekturelements, entstehen, so dass aufgrund dieses Konzentrationsunterschiedes der Marker diffundiert.

Der Nachweis der Diffusion des Markers kann beispielsweise optisch und/oder elektrochemisch erfolgen. Beispielsweise können bei dem vorgeschlagenen Verfahren und/oder in der vorgeschlagenen Vorrichtung ein oder mehrere Markerdetektoren verwendet werden, welche eingerichtet sein können, um die Diffusion des Markers zu detektieren. Beispielsweise kann der mindestens eine Markerdetektor mindestens einen optischen Markerdetektor und/oder mindestens einen elektrochemischen Markerdetektor aufweisen. Ein elektrochemischer Markerdetektor kann beispielsweise eine oder mehrere elektrochemische Nachweiselektroden umfassen. Ein optischer Markerdetektor kann beispielsweise mindestens ein fotoempfindliches Detektorelement umfassen, beispielsweise mindestens eine Fotodiode. Der optische Markerdetektor kann so ausgestaltet sein, dass er Licht, das von einer Lichtquelle ausgesendet wird, detektieren kann. Dabei muss das Licht der Lichtquelle nicht direkt auf den Detektor gerichtet sein sondern kann durch verschiedene Elemente, wie beispielsweise Filter, Spiegel oder dem Testelement verändert bzw. reflektiert werden. Weiterhin kann der optische Markerdetektor selbst zusätzlich mindestens eine Lichtquelle umfassen Die Lichtquelle kann Licht in einem beliebigen Wellenlängenbereich ausstrahlen. Bevorzugt strahlt die Lichtquelle Licht in einem Wellenlängenbereich zwischen 200 und 1000 nm. Je nachdem in welchem Wellenlängenbereich der Analyt das Licht absorbiert, kann bevorzugt ein anderer Wellenlängenbereich für die Detektion des Markers verwendet werden. Beispielsweise kann zur Anregung des Markers Licht in einem Wellenlängenbereich des UV-Lichts, beispielsweise in einem Wellenlängenbereich zwischen 200 und 400 nm verwendet werden, wenn der Analyt Licht in einem Wellenlängenbereich von 500 bis 1000 nm absorbiert. Umgekehrt kann beispielsweise der Analyt in einem Wellenlängenbereich von 300 bis 400 nm detektiert werden, wenn der Marker Licht in einem Wellenlängenbereich von 400 bis 1000 nm absorbiert. Andere Wellenlängenbereich für die Anregung des Analyten bzw. Markers sind ebenfalls denkbar.

Die Lichtquelle kann beispielsweise mindestens eine Leuchtdiode umfassen, wobei die Lichtquelle beispielsweise zur Beleuchtung mindestens eines den Marker enthaltenden Testchemiefeldes und/oder mindestens eines den Marker enthaltenden Markerfeldes und/oder zur Beleuchtung der Probe eingerichtet sein kann.

Beispielsweise kann eine Konzentration des Markers in mindestens einem Beobachtungsvolumen, beispielsweise in dem ersten Bereich und/oder dem zweiten Bereich, erfasst werden, beispielsweise zu mindestens zwei verschiedenen Zeiten oder zu mehreren Zeitpunkten. Beispielsweise kann ein bestimmtes Volumen optisch und/oder elektrochemisch vermessen werden, in dem sich vor einer Benetzung mit Probe Marker befindet. Nach Benetzung dieses sowie anderer Volumina des Testfeldes wird sich aufgrund der Konzentrationsunterschiede an Marker die Konzentration des Markers in dem Volumen senken. Alternativ oder zusätzlich kann beispielsweise ein bestimmtes Volumen beobachtet werden, welches sich nach Benetzung mit Probe mit dem Marker anreichert. Die Diffusionsgeschwindigkeit des Markers hängt in der Regel von vielen verschiedenen Parametern ab, wie beispielsweise Temperatur, Druck, sowie der chemischen Umgebung des Markers. Unter einer chemischer Umgebung des Markers kann zum einen die Umgebung des Markers vor Benetzung mit Probe verstanden werden und/oder die Umgebung des Markers nach Benetzung mit Probe. Da die chemische Umgebung des Markers vorzugsweise vor Benetzung mit Probe bekannt sein sollte, wird die Diffusionsgeschwindigkeit des Markers nach Benetzung hauptsächlich durch die Bestandteile der Probe sowie der Temperatur beeinflusst, insbesondere wenn davon ausgegangen wird, dass bei annähernd Normaldruck gearbeitet wird. Vorteilhafterweise ist die Diffusion des Markers vor Benetzung mit Probe vernachlässigbar, da sich der Marker vorzugsweise in einer zumindest nahezu trockenen Umgebung befindet, in der vorzugsweise keine oder lediglich vernachlässigbare Diffusionsvorgänge stattfinden. So können beispielsweise mindestens ein erstes Detektorsignal zur Bestimmung einer Markerkonzentration vor Benetzung und mindestens ein zweites Detektorsignal zur Bestimmung einer Markerkonzentration nach Benetzung, beispielsweise über einen bestimmten Zeitraum und/oder zu einem bestimmten Zeitpunkt, als Grundlage dienen, um eine Diffusion des Markers, nach Benetzung des Markers mit Probe, zu detektieren, beispielsweise in Form einer Diffusionsgeschwindigkeit des Markers. Alternativ oder zusätzlich kann auch ein Zeitraum nur nach Benetzung des Markers beobachtet werden. Weiterhin alternativ oder zusätzlich kann ein Detektionssignal zu einem bestimmten Zeitpunkt nach Benetzung des Markers mit Probe erfolgen. Vorzugsweise ist die Diffusion des Markers nur von wenigen, zuvor bekannten Parametern abhängig. So sollte beispielsweise die Diffusionsgeschwindigkeit des, in einer Glucosebestimmung eingesetzten Markers, nicht von der Konzentration des zu bestimmenden Analyten, hier Glucose, abhängig sein.

Werden zur Detektion der Diffusion des Markers ein oder mehrere Detektorsignale verwendet, so können das Detektorsignal bzw. die Detektorsignale beispielsweise mit einer oder mehreren Referenzkurven, die charakteristisch für verschiedene Temperatur- und Konzentrationsverhältnisse der Probe sind, verglichen werden. Hieraus können beispielsweise direkt oder indirekt die Temperatur und/oder mindestens ein anderer Parameter der Probe bestimmt werden. Alternativ oder zusätzlich können zu verschiedenen Zeitpunkten nach Benetzung des Markers mit der Probe Konzentrationen des Markers detektiert werden und mit hinterlegten Referenzkurven verglichen werden. Alternativ oder zusätzlich können in bestimmten Zeitbereichen nach der Benetzung des Markers mit Probe Steigungen des Detektorsignals ermittelt werden, die ebenfalls mit Referenzkurven verglichen werden können. Auf diese Weise können beispielsweise sowohl die Temperatur-abhängige Beeinflussung der Diffusionsgeschwindigkeit des Markers, als auch die Beeinflussung der Diffusionsgeschwindigkeit des Markers durch die chemische Zusammensetzung der Probe, bestimmt werden.

Wie oben ausgeführt, wird bei dem vorgeschlagenen Verfahren aus der Diffusion des Markers mindestens eine Korrekturinformation erzeugt. Unter einer Korrekturinformation ist mindestens eine Information über mindestens eine Einflussgröße zu verstehen, wobei sich die Information aus der Diffusion des Markers ableiten lässt und wobei die Einflussgröße einen Einfluss auf die Konzentrationsbestimmung des Analyten hat oder haben kann. Beispielsweise kann es sich bei der Information um eine Information darüber handeln, wie mindestens ein Messwert, beispielsweise ein elektrochemischer und/oder optischer Messwert, in eine Analytkonzentration umzurechnen ist. Beispielsweise kann die Korrekturinformation eine Information darüber umfassen, welche Umrechnungsvorschrift bei einer Umrechnung des mindestens einen Messwerts in die Analytkonzentration anzuwenden ist und/oder wie eine Umrechnungsvorschrift auszugestalten und/oder zu verändern ist. Beispielsweise können mehrere Korrekturinformationen entsprechend detektierter Diffusion des Markers in einer Datenverarbeitungsvorrichtung und/oder in einem Datenspeicher hinterlegt sein. Alternativ oder zusätzlich kann mindestens eine Zuordnungsvorschrift hinterlegt sein, welche beispielsweise analytisch, empirisch oder semi-empirisch generiert werden kann, welche einer erfassten Diffusion jeweils eine Korrekturinformation für die Bestimmung der Analytkonzentration zuordnet.

Beispielsweise kann die Korrekturinformation durch einen Vergleich eines Detektorsignals einer Konzentrationsbestimmung des Markers mit hinterlegten Referenzkurven erzeugt werden, indem beispielsweise ein Verlauf des Detektorsignals zu einer bestimmten Referenzkurve zugeordnet werden kann, die wiederum charakteristisch für eine bestimmte Temperatur und/oder für einen bestimmten Hämatokrit ist. Es können weitere Korrekturinformationen durch die Bestimmung der Markerkonzentration bestimmt werden. So ist es beispielsweise möglich, eine Feuchte des Testelementes vor Benetzung mit Probe zu ermitteln, indem beispielsweise ein Detektorsignal für die Markerkonzentration vor Benetzung mit einem Referenzwert verglichen wird.

Bei dem Verfahren wird, wie oben ausgeführt, weiterhin aus der Reaktion der Testchemie mit dem Analyten unter Berücksichtigung der Korrekturinformation die Konzentration des Analyten bestimmt. Die Bestimmung der Konzentration des Analyten kann wie bereits beschrieben beispielsweise optisch und/oder elektrochemisch durchgeführt werden. Dabei wird beispielsweise die Reaktion der Testchemie mit dem Analyten durch den Nachweis eines Reagens, beispielsweise in Form eines Nachweisstoffes, das bei der Reaktion der Testchemie mit dem Analyten entsteht, durchgeführt. Hierbei kann beispielsweise mindestens ein Signal erzeugt werden, welches auch als Messwert bezeichnet wird. Das Signal kann beispielsweise während und/oder nach Reaktion der Testchemie mit dem Analyten ermittelt werden und kann durch die Korrekturinformation korrigiert werden, so dass beispielsweise eine Information über die tatsächliche Temperatur und optional den Hämatokrit berücksichtigt werden kann.

Die Korrekturinformation kann insbesondere ausgestaltet sein, um mindestens eine Störgröße der Probe zu korrigieren. Unter einer Störgröße ist allgemein mindestens eine Eigenschaft oder Eigenschaftskombination der Probe zu verstehen, welche unabhängig von der tatsächlichen Analytkonzentration ist, welche jedoch die Detektion der Reaktion der Testchemie mit dem Analyten und/oder die eigentliche Reaktion der Testchemie mit dem Analyten beeinflussen kann und dementsprechend eine Bestimmung der Analytkonzentration beeinflussen und/oder verfälschen kann. Insbesondere kann die mindestens eine Störgröße einen Anteil mindestens einer Störkomponente in der Probe beschreiben, also einen Anteil mindestens einer Substanz an der Probe, welche die Reaktion der Testchemie mit dem Analyten beeinflusst und/oder welche die Detektion der Reaktion der Testchemie mit dem Analyten beeinflusst. Beispielsweise kann diese Störkomponente ein Hämatokrit-Wert der verwendeten Probe, beispielsweise von Blut sein. Die Störgröße umfasst eine Temperatur der Probe und/oder kann eine Temperatur der Umgebung sein. In dem Verfahren ist die Korrekturinformation dementsprechend ausgestaltet, um einen Einfluss
der Temperatur der Probe und/oder des Testelements bei der Bestimmung der Konzentration des Analyten zu berücksichtigen. In
der Regel wird eine Reaktion des Analyten mit der Testchemie durch eine Erniedrigung der Temperatur verlangsamt. Umgekehrt wird in der Regel die Reaktion des Analyten bei Erniedrigung des Hämatokrits in der Probe erhöht.

Die Korrekturinformation umfasst also mindestens eine Information über mindestens eine Störgröße der Probe. Diese Störgröße umfasst, wie oben ausgeführt, eine Temperatur der Probe und/oder des Testelements sowie optional zusätzlich einen Hämatokrit der Probe. Auch andere Störgrößen sind grundsätzlich korrigierbar. Die Korrekturinformation wird unter Verwendung folgender Verfahrensschritte bestimmt, wobei die Verfahrensschritte vorzugsweise, jedoch nicht notwendigerweise, in der dargestellten Reihenfolge durchgeführt werden. Grundsätzlich ist auch eine andere Reihenfolge denkbar, oder einzelne oder mehrere Verfahrensschritte können zeitlich parallel, zeitlich überlappend oder einzelnen, gruppenweise oder insgesamt wiederholt durchgeführt werden.

In einem ersten Verfahrensschritt wird in mindestens einer Eichmessung ein allgemeiner Zusammenhang zwischen der mindestens einen Störgröße und der Diffusion des Markers erfasst. Dieser allgemeine Zusammenhang kann beispielsweise in Form einer oder mehrerer Eichkurven angegeben werden. Unter einem allgemeinen Zusammenhang soll dabei eine Vorschrift für eine Mehrzahl von verschiedenen Werten der Störgröße verstanden werden, welche beschreibt, wie diese Werte der Störgröße die Markerdiffusion beeinflussen. Die Vorschrift kann für einen kontinuierlichen Wertebereich der Störgröße ermittelt werden oder auch für einen diskontinuierlichen Wertebereich, beispielsweise eine Menge voneinander beabstandeter Werte der Störgröße. Dementsprechend kann der allgemeine Zusammenhang beispielsweise eine punktweise Zuordnung mehrerer Werte der Störgröße zu jeweils einer entsprechenden Beeinflussung der Markerdiffusion beinhalten. Alternativ oder zusätzlich kann die Vorschrift auch eine Gesetzmäßigkeit in Form einer analytischen Funktion beinhalten, welche auch als Eichkurve oder Eichfunktion bezeichnet werden kann und welche die Beeinflussung der Markerdiffusion durch die Störgröße analytisch beschreibt.

Die Eichmessung kann beispielsweise dadurch erfolgen, dass in einer Mehrzahl von Testproben oder Eichproben, in welchen die Störgröße bekannt ist, jeweils mindestens eine Markerdiffusion detektiert wird. Beispielsweise können Testproben hergestellt werden, welche einen Hämatokrit und/oder eine bekannte Temperatur aufweisen. Zu diesen Testproben kann jeweils mindestens ein Wert einer Markerdiffusion ermittelt werden. Wie unten näher ausgeführt, kann beispielsweise zu jeder Testprobe mindestens ein optischer Messwert, beispielsweise mindestens ein Remissionswert, eines Markerfeldes, welches mit der Testprobe in Kontakt steht, ermittelt werden. Auf diese Weise kann eine Menge von Wertepaaren bestimmt werden, welche jeweils die Störgröße und die zugehörige Markerdiffusion umfassen. Diese Wertepaare können selbst den allgemeinen Zusammenhang beschreiben, oder dieser allgemeine Zusammenhang kann aus den Wertepaaren ermittelt werden, beispielsweise mittels eines Fits. In vielen Fällen lässt sich, wie unten noch näher beschrieben wird, der allgemeine Zusammenhang durch eine Gerade beschreiben, deren Steigung und Achsenabschnitt aus den Wertepaaren durch einen entsprechenden Fit leicht bestimmt werden können. Diese Gerade kann dann als Eichkurve verwendet werden. Auch komplexere Eichkurven sind möglich, beispielsweise Exponentialfunktionen und/oder Polynome, welche den Zusammenhang zwischen den Wertepaaren möglichst gut beschreiben.

Der allgemeine Zusammenhang, insbesondere die Eichkurve oder Eichfunktion, kann insbesondere in mindestens einem Datenspeicher hinterlegt werden, beispielsweise in einem flüchtigen und/oder nicht flüchtigen Datenspeicher einer erfindungsgemäßen Vorrichtung, beispielsweise einer Auswertungseinheit der Vorrichtung. Beispielsweise kann die Erfindungsgemäßevorrichtung, welche unten noch näher beschrieben wird, mindestens eine Auswertungseinheit in Form einer Datenverarbeitungsvorrichtung umfassen. Diese Auswertungseinheit kann eingerichtet sein, um die Verfahrensschritte des erfindungsgemäßen Verfahrens vollständig oder teilweise durchzuführen. Die Eichmessung kann unabhängig von der Vorrichtung durchgeführt werden, kann jedoch auch ganz oder teilweise mit der erfindungsgemäßen Vorrichtung durchgeführt werden.

In einem weiteren Verfahrensschritt werden dann in der eigentlichen Probe, in welcher die Konzentration des Analyten bestimmt werden soll, sowohl die Reaktion der Testchemie mit dem Analyten als auch die Diffusion des Markers detektiert. Beispielsweise kann mindestens ein optischer Messwert, beispielsweise mindestens ein Remissionswert, ermittelt werden, welcher die Reaktion der Testchemie mit dem Analyten detektiert. Weiterhin kann mindestens ein optischer Messwert, beispielsweise mindestens ein weiterer Remissionswert, ermittelt werden, welcher die Diffusion des Markers detektiert. Die optischen Messwerte zur Detektion der Reaktion der Testchemie mit dem Analyten und zur Detektion der Markerdiffusion können insbesondere bei unterschiedlichen Wellenlängen erfasst werden.

In einem weiteren Verfahrensschritt wird dann die Korrekturinformation aus der detektierten Diffusion des Markers in der eigentlichen Probe und dem allgemeinen Zusammenhang zwischen der Störgröße und der Diffusion des

Markers bestimmt. Dies kann beispielsweise auf einfache Weise dadurch erfolgen, dass die detektierte Diffusion des Markers in der eigentlichen Probe, beispielsweise der optische Messwert des Markers und insbesondere die Remission des Markerfeldes, als Funktionswert des allgemeinen Zusammenhangs, beispielsweise als Funktionswert der Eichkurve oder Eichfunktion, eingesetzt wird, wobei die Korrekturinformation entsteht.

Die derart ermittelte Korrekturinformation kann dann zur Korrektur der Analytkonzentration und/oder zur Korrektur der Detektion der Reaktion der Testchemie mit dem Analyten verwendet werden. Beispielsweise kann zunächst in der Probe, in welcher die Analytkonzentration bestimmt werden soll, mindestens ein unkorrigierter optischer Messwert, beispielsweise eine Remission, erfasst werden, welche anschließend mit der Korrekturinformation korrigiert wird, um einen korrigierten optischen Messwert zu erhalten. Die Korrektur kann beispielsweise durch Addition oder Subtraktion der Korrekturinformation von dem unkorrigierten optischen Messwert und/oder durch Multiplikation der Korrekturinformation mit dem unkorrigierten optischen Messwert und/oder durch Bildung einer Linearkombination aus der Korrekturinformation und dem unkorrigierten optischen Messwert erfolgen. Beispiele werden unten noch näher ausgeführt.

In dem Verfahren kann allgemein die Konzentration einer oder mehrerer Analyte bestimmt werden. Bevorzugt wird der Analyt ausgewählt aus einer Gruppe, bestehend aus: Cholesterin, Lactat, Fructose, Glucose. Da Glucose in der Regel der wichtigste Analyt bei der Blutkontrolle für Diabetiker ist, werden im Folgenden das Verfahren, die Vorrichtung und das Testelement auf diesen Analyten hin beschrieben, was jedoch nicht einschränkend zu sehen ist, und übertragbar auch für andere Analyten gilt.

In dem Verfahren kann das Testelement auf verschiedenste Arten ausgestaltet sein. In einem bevorzugten Verfahren wird das Testelement ausgewählt aus einer Gruppe, bestehend aus: einem Teststreifen, einem Testband, einer Testnadel, insbesondere einem Microsampler. Im Folgenden werden, ohne Beschränkung weiterer, alternativ oder zusätzlich einsetzbarer Ausführungsformen, insbesondere flache, streifenförmige Testelemente, also Teststreifen beschrieben.

Das Testelement kann beispielsweise mindestens ein Testfeld umfassen. Wie oben ausgeführt, kann das Testfeld insbesondere einen zwei- oder dreidimensionalen Bereich des Testelements umfassen, welcher grundsätzlich für den durchführbaren Nachweis des Analyten einsetzbar ist. Insbesondere kann das Testfeld als Trockentestfeld ausgestaltet sein. Das Testfeld umfasst beispielsweise die Testchemie, die ein Nachweisreagens aufweist, welches eingerichtet ist, um bei Anwesenheit des Analyten eine nachweisbare Reaktion durchzuführen. Diesbezüglich kann beispielsweise auf die obige Beschreibung verwiesen werden, beispielsweise die im eingangs genannten Stand der Technik erwähnten Nachweisreagenzien. Zusätzlich zu dem mindestens einen Nachweisreagens, welches mindestens eine analytspezifische Reaktion durchführen kann, kann das Testfeld weitere Substanzen enthalten, beispielsweise Trägerstoffe, Hilfsstoffe, Pigmente, Füllstoffe, Pufferstoffe oder ähnliches. Zwischen weiteren Stoffen, die ebenfalls an der Reaktion zum Nachweis des Analyten beteiligt sein können, und dem eigentlichen Nachweisreagens wird im Folgenden dabei nicht unterschieden. Insbesondere kann das Nachweisreagens wie bereits erwähnt mindestens ein enzymatisches Nachweisreagens umfassen. In einem bevorzugten Verfahren weist das Testelement mindestens ein Testfeld mit mindestens einer Testchemieschicht auf, wobei die Testchemieschicht die Testchemie umfasst, wobei die Testchemie insbesondere mindestens ein Enzym enthält, insbesondere ausgewählt aus der Gruppe, bestehend aus: Glucosedehydrogenase, Glucoseoxidase.

Die Detektion der Reaktion des Analyten erfolgt elektrochemisch und/oder optisch, und/oder die Detektion der Diffusion des Markers erfolgt elektrochemisch und/oder optisch. Zu diesem Zweck können beispielsweise bei dem Verfahren und/oder in einer erfindungsgemäßen Vorrichtung ein oder mehrere Detektoren vorgesehen sein. Diese Arten der Detektion sind grundsätzlich im Stand der Technik bekannt. Bei der Detektion beispielsweise auf elektrochemischem Wege ist vorzugsweise zu gewährleisten, dass der mindestens eine Analytdetektor mindestens einen elektrochemischen Analytdetektor umfasst, wobei Elektroden des elektrochemischen Detektors mit dem zu analysierenden Reagens, beispielsweise Analyt und/oder Marker, in Kontakt treten können. Der mindestens eine elektrochemische Analytdetektor kann somit mindestens eine, vorzugsweise zwei oder mehr Elektroden zur elektrochemischen Detektion aufweisen. Bei einer alternativ oder zusätzlich realisierbaren optischen Detektion kann beispielsweise der mindestens eine Analytdetektor mindestens einen optischen Analytdetektor umfassen. Der mindestens eine optische Analytdetektor kann beispielsweise mindestens ein fotoempfindliches Detektorelement umfassen, beispielsweise mindestens eine Fotodiode. Dieses mindestens eine fotoempfindliche

Detektorelement des Analytdetektors kann beispielsweise ganz oder teilweise verschieden von einem optionalen fotoempfindlichen Detektorelement des Markerdetektors ausgebildet sein. Alternativ oder zusätzlich kann das fotoempfindliche Detektorelement des Analytdetektors auch ganz oder teilweise bauteilidentisch mit dem fotoempfindlichen Detektorelement des Markerdetektors ausgebildet sein, beispielsweise indem eine gemeinsame Fotodiode vorgesehen ist, welche sowohl Bestandteil des Analytdetektors als auch Bestandteil des Markerdetektors ist. Teilen sich der Markerdetektor und der Analytdetektor mindestens ein Bauelement, so kann beispielsweise ein zeitlich getrennter, beispielsweise zeitversetzter, Nachweis des Markers und/oder der Markerdiffusion und des Analyten erfolgen, beispielsweise indem ein gepulstes und/oder intermittierendes Messschema verwendet wird, in welchem zu bestimmten Zeiten mittels der Fotodiode die Markerdiffusion erfasst wird und zu anderen Zeiten mittels derselben Fotodiode der Analytnachweis

durchgeführt wird. Weiterhin kann der optische Analytdetektor wiederum mindestens eine Lichtquelle umfassen, beispielsweise mindestens eine Leuchtdiode, wobei die mindestens eine Lichtquelle beispielsweise zur Beleuchtung mindestens eines die Testchemie enthaltenden Testchemiefeldes eingerichtet sein kann. Die mindestens eine Lichtquelle des Analytdetektors kann beispielsweise wiederum ganz oder teilweise verschieden von der mindestens einen Lichtquelle des Markerdetektors ausgestaltet sein. Besonders bevorzugt umfasst der Analytdetektor mindestens eine Analytdetektor-Leuchtdiode, und der Markerdetektor umfasst mindestens eine Markerdetektor-Leuchtdiode, die von der Analytdetektor-Leuchtdiode verschieden ist. Alternativ oder zusätzlich kann die mindestens eine Lichtquelle des Analytdetektors jedoch auch wiederum ganz oder teilweise bauteilidentisch mit der mindestens einen Lichtquelle des Markerdetektors ausgestaltet sein.

Besonders bevorzugt wird beispielsweise ein Testelement verwendet, das zwei sich gegenüberliegende Oberflächen aufweist. Der Analytdetektor und/oder der Markerdetektor können dieses Testelement oder einen Teil desselben beispielsweise von einer Seite her, die auch als Detektionsseite bezeichnet werden kann, mit Licht bestrahlen, beispielsweise mittels mindestens einer Markerdetektor-Lichtquelle und/oder mittels mindestens einer Analytdetektor-Lichtquelle. Weiterhin kann das Verfahren derart durchgeführt werden und kann die Vorrichtung derart eingerichtet sein, dass von dem Testelement ausgehendes Licht, beispielsweise reflektiertes und/oder gestreutes Licht, detektiert wird, vorzugsweise ebenfalls von der Detektionsseite her, beispielsweise unter Verwendung des fotoempfindlichen Detektorelements des Markerdetektors und/oder des fotoempfindlichen Detektorelements des Analytdetektors. Bevorzugt wird das Testelement mit der Probe von der gegenüberliegenden Seite benetzt, welche beispielsweise auch als Probenaufgabeseite oder Applikationsseite bezeichnet werden kann und welche der Detektionsseite gegenüberliegen kann. So kann das Testelement, insbesondere der Teststreifen, beispielsweise mindestens eine Probenaufgabeseite und mindestens eine Detektionsseite umfassen. Alternativ oder zusätzlich kann eine Applikation der Probe jedoch auch an einer anderen Stelle erfolgen, beispielsweise an einer Applikationsstelle eines Kapillarelements des Testelements.

Bevorzugt weist das Testelement zwei räumlich getrennte Bereiche für die Detektion des Analyten und die Detektion des Markers auf. So können beispielsweise zwei aneinander grenzende Bereiche des Testelementes so zu einander angeordnet sein, dass sie beide mit der Probe in Kontakt gebracht werden können, jedoch untereinander kein Flüssigkeitsaustausch möglich ist, also keine Diffusion von einem Bereich zum anderen möglich ist. In einer alternativen Ausgestaltung können die Bereiche zur Detektion des Analyten bzw. des Markers zwar nebeneinander angeordnet sein, wobei jeweils die Diffusion des Analyten und/oder des Markers möglich sein soll. Besonders bevorzugt sind diese beiden Bereiche so zueinander angeordnet, dass sie, eingebracht in ein Messinstrument, von je einer Lichtquelle beleuchtet werden können. Der Bereich in dem sich der Marker befindet kann weiterhin als Kapillare ausgebildet sein, die bei Benetzung mit der Probe gefüllt wird. Zur Detektion des Markers kann beispielsweiseeine Diffusion des Markers in der Kapillare bestimmt werden.

In einem bevorzugten Verfahren weist das Testelement weiterhin mindestens eine Abtrennschicht auf. Diese Abtrennschicht kann beispielsweise eingerichtet sein, um mindestens einen Bestandteil der Probe, beispielsweise partikuläre und/oder zelluläre Bestandteile, zurückzuhalten, beispielsweise Hämoglobin und/oder rote Blutkörperchen, die sich in der Probe befinden. Alternativ oder zusätzlich kann die mindestens eine Abtrennschicht auch mindestens ein optisches Material umfassen, beispielsweise mindestens ein Pigment, beispielsweise um eine Reflexionsschicht in Form einer Pigmentschicht zu bilden. Beispielsweise kann das optische Material $TiO_2$, $ZrO_2$ oder $BaSO_4$ umfassen. Bevorzugt befindet sich die Abtrennschicht auf dem Testfeld. Besonders bevorzugt befindet sich die Abtrennschicht auf der Seite des Testelements auf der die Probe aufgebracht wird, also der Probenaufgabeseite. So kann beispielsweise die Probe auf der Seite des Testelements mit der Abtrennschicht aufgebracht werden, während die Detektion des Analyten beispielsweise optisch auf der gegenüberliegenden Seite erfolgen kann. Beim Eindringen der Probe in das Testelement können die Probe oder Teile der Probe eine oder mehrere Schichten des Testfeldes durchdringen, beispielsweise die Abtrennschicht, und dabei oder anschließend in Kontakt sowohl mit der Testchemie als auch optional mit dem Marker kommen. Weiterhin kann ein Teil der Probe in bzw. oberhalb der Abtrennschicht verbleiben, beispielsweise als Überstand oder Teil eines Überstands.

Der Marker kann mindestens vor Benetzung des Testelements mit Probe, insbesondere vor Beginn der Nachweisreaktion, in mindestens einem Bereich des Testelements enthalten sein. Dieser kann beispielsweise als erster Bereich bezeichnet werden. Ist der Marker nur in diesem mindestens einen ersten Bereich des Testelementes vorhanden, so kann dieser Bereich auch als Markerbereich bezeichnet werden. Dieser erste Bereich kann beispielsweise als Markerschicht ausgestaltet sein.

In einem bevorzugten Verfahren ist der Marker in mindestens einem Bereich des Testelements enthalten, wobei die Testchemie ebenfalls ganz oder teilweise in dem ersten Bereich enthalten ist. In diesem Fall können beispielsweise die Testchemieschicht und die Markerschicht identisch sein. Sobald die Probe mit dem Marker und der Testchemie in Kontakt gekommen ist, können beispielsweise sowohl Marker als auch Testchemie in dem Testelement und/oder in der Probe, bevorzugt in dem Testfeld, in einen oder mehrere Bereiche mit geringerer Konzentration diffundieren. Aufgrund von Konzentrationsunterschieden in unterschiedlichen Bereichen des Testfeldes, bzw. der Probe, an Marker bzw. Testchemie, können beispielsweise sowohl Marker als auch Testchemie in Bereiche der Probe mit geringerer Konzentration

dieser Bestandteile diffundieren. Es ist vorteilhaft, wenn das Diffusionsverhalten, insbesondere die Diffusionsgeschwindigkeit des Markers dem Diffusionsverhalten bzw. der Diffusionsgeschwindigkeit der Testchemie bzw. dem Analyten ähnelt. Auf diese Weise können beispielsweise sowohl der Marker als auch der Analyt in einem gleichen Bereich des Testfeldes detektiert werden.

Wenn sich der Marker und die Testchemie ganz oder teilweise in einem ersten Bereich des Testelementes befinden, kann die Detektion der Diffusion des Markers beispielsweise mit dem gleichen Detektor und/oder von der gleichen Seite des Testelementes erfolgen. Dies kann wie bereits erwähnt sowohl elektrochemisch als auch optisch durchgeführt werden. Es kann jedoch auch sowohl für den Marker als auch für die Testchemie jeweils mindestens ein eigener Detektor verwendet werden, beispielsweise mindestens ein Markerdetektor und mindestens ein Analytdetektor. Zur Detektion der Diffusion des Markers kann beispielsweise ein Bereich des Testelementes vermessen werden, der vor Benetzung des Testelementes mit Probe den Marker aufweist, oder es kann alternativ ein Bereich des Testelementes bzw. der Probe vermessen werden, der vor Benetzung des Markers mit Probe keinen Marker aufweist. Im ersten Fall wird folglich die Abnahme der Markerkonzentration beispielsweise nach Benetzung des Markers mit Probe vermessen, während im zweiten Fall beispielsweise die Erhöhung der Konzentration des Markers in dem vermessenen Bereich erfolgt.

Das Testelement kann insbesondere mindestens eine Markerschicht und/oder mindestens ein Markerfeld aufweisen, welche den mindestens einen Marker umfassen können. Allgemein ist im Rahmen der vorliegenden Erfindung unter einer Schicht oder einem Feld eine zusammenhängende Menge des jeweiligen Materials zu verstehen, welche eine laterale Ausdehnung und eine Dicke aufweist. Beispielsweise können die Schicht und/oder das Feld eine laterale Ausdehnung, insbesondere einen Durchmesser und/oder einen Äquivalentdurchmesser und/oder eine Seitenlänge, von mindestens 100 Mikrometern, vorzugsweise von mindestens 500 Mikrometern oder mindestens 1 Millimeter aufweisen. Weiterhin können die Schicht und/oder das Feld eine Dicke von weniger als 100 Mikrometern aufweisen, insbesondere eine Dicke von weniger als 50 $\mu$m. Eine Schicht kann insbesondere ein Bestandteil eines Mehrschicht-Aufbaus sein. Ein Feld kann insbesondere eine zugängliche Oberfläche aufweisen, beispielsweise eine Oberfläche, welche mit einer flüssigen Probe in Kontakt gebracht werden kann. Alternativ kann das Feld jedoch auch von einer oder mehreren weiteren Schichten und/oder Elementen bedeckt sein.

Das Testelement kann ein oder mehrere Testfelder aufweisen, welche die mindestens eine Testchemie umfassen können. Wie oben ausgeführt, kann der mindestens eine Marker ganz oder teilweise in dem mindestens einen Testfeld enthalten sein, beispielsweise ganz oder teilweise in die mindestens eine Testchemie eingemischt sein und/oder auf andere Weise in dem Testfeld enthalten sein. In einer alternativ oder zusätzlich realisierbaren Ausführung kann der mindestens eine Marker auch ganz oder teilweise separat von der Testchemie angeordnet sein, jedoch vorzugsweise auf demselben Testelement und demselben Testträger. Unter dem Ausdruck "separat" ist allgemein hierbei zu verstehen, dass in diesem bevorzugten Ausführungsbeispiel die Testchemie und der Marker nicht in einem gemeinsamen, zusammenhängenden Material enthalten sind, beispielsweise nicht in derselben Schicht und/oder nicht in demselben Schichtaufbau. Der Marker kann insbesondere zumindest vor einer Benetzung mit der Probe in mindestens einer Markerschicht angeordnet sein, die beispielsweise an die Testchemieschicht angrenzend oder beabstandet angeordnet ist, beispielsweise auf nebeneinander liegenden oder beabstandeten Flächen auf einem Testträger des Testelements. Diese Flächen können beispielsweise um mindestens 100 Mikrometer beabstandet sein, vorzugsweise um mindestens 200 Mikrometer oder sogar um mindestens 500 Mikrometer. Die mindestens eine Markerschicht kann alternativ oder zusätzlich durch eine oder mehrere Schichten von der Testchemieschicht getrennt angeordnet sein, beispielsweise in einem Schichtaufbau, welcher mindestens eine Testchemieschicht und mindestens eine von der Testchemieschicht verschiedene Markerschicht umfasst. Dabei können die Markerschicht und die Testchemieschicht unmittelbar aneinander angrenzen, oder es können ein oder mehrere weitere Schichten zwischen der Markerschicht und der Testchemieschicht angeordnet sein. Alternativ kann der Marker beispielsweise in mindestens einer Markerschicht oberhalb und/oder unterhalb des Testfeldes angeordnet sein. Die Markerschicht kann beispielsweise Bestandteil des Testelementes, insbesondere Bestandteil des Testfeldes sein, sie kann jedoch auch eine vom Testelement separate Schicht sein, die dennoch von der gleichen Probe benetzt wird. Alternativ kann die Markerschicht zwar auf dem Testelement angeordnet sein, jedoch nicht Bestandteil des Testfeldes sein. Durch die Separation des Markers von der Testchemie kann gewährleistet werden, dass der Marker die Diffusionseigenschaften bzw. Reaktionseigenschaften der Testchemie nicht beeinflusst. Wiederum alternativ oder zusätzlich können der Marker und/oder die Markerschicht auch ganz oder teilweise außerhalb des Testelements angeordnet sein, beispielsweise in einem separaten Korrekturelement und/oder in einer Vorrichtung zur Bestimmung mindestens einer Konzentration mindestens eines Analyten in einer Probe, beispielsweise einem Testgerät.

Die Detektion des Markers kann, wie bereits erwähnt, optional mit dem gleichen Detektor durchgeführt werden, wie die Detektion des Analyten. Dieser mindestens eine Detektor sollte vorzugsweise eine optische Detektion bei mehreren Wellenlängen durchführen können. Es kann alternativ auch ein kombinierter Detektor verwendet werden, der zwei Detektoreinheiten aufweist, die bei unterschiedlichen Wellenlängen detektieren können. Werden für den Analytnachweis und die Detektion des Markers unterschiedliche Detektoren verwendet, beispielsweise mindestens ein Analytdetektor

und mindestens ein Markerdetektor, beispielsweise mindestens ein optischer Analytdetektor und mindestens ein optischer Markerdetektor, so können diese Detektoren auf unterschiedliche Weise angeordnet sein. Beispielsweise können die Detektoren auf einander gegenüberliegenden Seiten des Testelements angeordnet sein, beispielsweise der Analytdetektor auf der Detektionsseite und der Markerdetektor auf der Probenaufgabeseite. Alternativ oder zusätzlich können die Detektoren jedoch auch ganz oder teilweise auf derselben Seite des Testelements angeordnet sein, beispielsweise auf der Detektionsseite. Weiterhin können Filter im Strahlengang zwischen Lichtquelle und Detektor eingesetzt werden. Filter haben im allgemeinen die Eigenschaft, eine oder mehrere Wellenlängen des Lichtes wegfiltern können, indem sie diese beispielsweise absorbieren oder reflektieren.

In einem bevorzugten Verfahren umfasst der Marker mindestens einen optisch nachweisbaren Farbstoff. Unter einem optisch nachweisbaren Farbstoff ist eine Substanz zu verstehen, deren Elektronen mit elektromagnetischen Wellen, vorzugsweise aus einem Wellenlängenbereich von 100 nm bis 1500 nm (im Folgenden als Licht bezeichnet), besonders bevorzugt aus einem Wellenlängenbereich von 100 nm bis 400 nm (auch als ultraviolettes Licht bezeichnet) und/oder von 300 bis 800 nm (im Folgenden als sichtbares Licht bezeichnet) und/oder von 800 nm bis 1500 nm (auch als infrarotes Licht bezeichnet), wechselwirken. Bevorzugte Wechselwirkungen sind ausgewählt aus der Gruppe: Absorption, Fluoreszenz und Phosphoreszenz. Beispielsweise kann der optisch nachweisbare Farbstoff derart ausgewählt sein, dass dieser die elektromagnetischen Wellen in zumindest einem Wellenlängenbereich oder bei mindestens einer Wellenlänge spektral beeinflusst, beispielsweise indem der Farbstoff bei einer oder mehreren Wellenlängen und/oder in mindestens einem Wellenlängenbereich Licht selektiv absorbiert. Diese spektrale Beeinflussung soll nachweisbar sein. Alternativ oder zusätzlich zu einer Beeinflussung durch Absorption kann sich die Wellenlänge des Lichtes beispielsweise so verändern, dass eine Verschiebung der Wellenlänge von eingestrahltem Licht zu emittiertem und optional detektiertem Licht erfolgt. Der Farbstoff kann somit beispielsweise einen Absorptionsfarbstoff und/oder einen Fluoreszenzfarbstoff und/oder einen Phosphoreszenzfarbstoff umfassen.

Eine optische Detektion kann beispielsweise dadurch erfolgen, dass ein Detektor mit mindestens einem optischen Sensor verwendet wird, welcher eingerichtet ist, um Licht zu empfangen und mindestens ein entsprechendes Signal zu erzeugen. Weiterhin kann beispielsweise mindestens eine Lichtquelle verwendet werden, beispielsweise um das Testelement und/oder einen Teil desselben und/oder die Probe und/oder einen Teil desselben und/oder ein optionales Korrekturelement und/oder einen Teil desselben zu beleuchten, wobei mittels des optischen Sensors beispielsweise reflektiertes und/oder gestreutes und/oder emittiertes Licht detektiert wird. Von Vorteil ist es bei einer optischen Detektion von Analyt und/oder Marker, wenn die Wellenlängen des eingestrahlten Lichtes und/oder des detektierten Lichtes deutlich unterschiedlich sind, um eine Unterscheidung der beiden Signale vornehmen zu können. Unter deutlich unterschiedlich ist ein Abweichen der zu detektierenden Wellenlängen zur Analytbestimmung gegenüber der zu detektierenden Wellenlänge zur Bestimmung des Markers von mindestens 20 nm, bevorzugt mindestens 30 nm, besonders bevorzugt von mindestens 50 nm zu verstehen.

In einer bevorzugten Ausgestaltung des Verfahrens wird der Analyt bei einer anderen Wellenlänge bestimmt als der Marker. Zu diesem Zweck kann der Markerdetektor beispielsweise mindestens eine Markerdetektor-Lichtquelle, und der Analytdetektor kann mindestens eine Analytdetektor-Lichtquelle aufweisen, wobei die Markerdetektor-Lichtquelle beispielsweise eingerichtet sein kann, um das Testelement mit Licht einer anderen Wellenlänge zu bestrahlen als die Analytdetektor-Lichtquelle. Beispielsweise kann das Licht der Markerdetektor-Lichtquelle eine kürzere Wellenlänge aufweisen als das Licht der Analytdetektor-Lichtquelle oder umgekehrt. Beispielsweise kann der Analyt mit Hilfe von Licht in einem Wellenlängenbereich zwischen 400 und 1000 nm, bevorzugt in einem Wellenlängenbereich zwischen 500 und 800 nm, besonders bevorzugt in einem Wellenlängenbereich zwischen 640 und 680 nm detektiert werden, beispielsweise mit Licht, ausgesendet von der Lichtquelle, bei 660 nm. Dabei kann der Marker beispielsweise mit Licht in einem Wellenlängenbereich von weniger als 400 nm nachgewiesen werden, beispielsweise mit Licht in einem Wellenlängenbereich von 300 nm bis (jedoch bevorzugt kleiner als) 400 nm, beispielsweise bei 360 nm. Auch ein umgekehrter Aufbau ist möglich. So kann alternativ beispielsweise der Marker mit Hilfe von Licht in einem Wellenlängenbereich zwischen 400 und 1000 nm, bevorzugt in einem Wellenlängenbereich zwischen 500 und 800 nm, besonders bevorzugt in einem Wellenlängenbereich zwischen 640 und 680 nm detektiert werden, beispielsweise mit Licht bei 660 nm. Dabei kann der Analyt beispielsweise mit Licht in einem Wellenlängenbereich von weniger als 400 nm nachgewiesen werden, beispielsweise mit Licht in einem Wellenlängenbereich von 300 nm bis (jedoch bevorzugt kleiner als) 400 nm, beispielsweise bei 360 nm. In einer bevorzugten Ausgestaltung des Verfahrens absorbiert der Marker Licht in einem Wellenlängenbereich von 400 bis 800 nm. Der Marker, beispielsweise in Form eines Farbstoffes, kann beispielsweise mit Hilfe von Licht mit einer Wellenlänge in einem Wellenlängenbereich zwischen 300 und 800 nm (jedoch bevorzugt kleiner als 400 nm) angeregt werden oder Licht in diesem Wellenlängenbereich absorbieren, bevorzugt in einem Wellenlängenbereich zwischen 300 und 500 nm, besonders bevorzugt in einem Wellenlängenbereich zwischen 340 und 380 nm angeregt werden. Auch eine umgekehrte Ausgestaltung ist möglich. Bevorzugt wird für die Analytbestimmung und die Markerbestimmung jeweils eine unterschiedliche Lichtquelle verwendet. So können beispielsweise zwei unterschiedliche Leuchtdioden verwendet werden. Die Leuchtdiode zur Anregung des Analyten kann beispielsweise ein Intensitätsmaximum um 660 nm aufweisen. Die Leuchtdiode zur Anregung des Markers kann beispielsweise ein Intensitätsmaximum

bei 360 nm aufweisen. Alternativ kann auch eine Lichtquelle verwendet werden, dessen Licht mit Hilfe von optischen Mitteln, wie Filter, Blenden oder Spiegel so aufbereitet wird, dass der Detektionsbereich zu unterschiedlichen Zeiten mit Licht unterschiedlicher Wellenlängen bestrahlt wird. Es ist bevorzugt, dass der Wellenlängenbereich des Lichtes zur Detektion des Markers mindestens 5 nm, bevorzugt mindestens 10 nm, besonders bevorzugt mindestens 50 nm, ganz besonders bevorzugt mindestens 100 nm von dem Wellenlängenbereich des Lichtes zur Detektion des Analyten entfernt liegt. Dies kann auf verschiedene Weise realisiert werden. Es ist weiterhin bevorzugt, dass der Wellenlängenbereich des Lichtes zur Detektion des Markers in einem Bereich von 5 bis 100 nm von dem Wellenlängenbereich des Lichtes zur Detektion des Analyten entfernt liegt. Zur Bestimmung des Abstands der Wellenlängenbereiche zur Detektion des Markers bzw. des Analyten wird bevorzugt der Abstand des Absorptionsmaximums des Markers bzw. des Analyten betrachtet. Es ist beispielsweise möglich zwei Lichtquellen mit unterschiedlichen Wellenlängenbereichen des ausgestrahlten Lichtes zu verwenden. Weiterhin kann auch nur eine Lichtquelle mit einem breiten Wellenlängenbereich des ausgestrahlten Lichtes verwendet werden, wobei Filter im Strahlengang eingesetzt werden können, die bewirken, dass die Bestimmung des Markers bei einer anderen Wellenlänge erfolgt als die Bestimmung des Analyten.

Bevorzugt umfasst der Marker mindestens einen optisch nachweisbaren Farbstoff. Hierbei kann es sich beispielsweise um einen organischen und/oder einen anorganischen Farbstoff handeln. Je nach Verwendungszweck kann es von Vorteil sein, dass der Farbstoff hydrophile oder hydrophobe Eigenschaften aufweist. Bei Verwendung von wässrigen Proben, wie beispielsweise Körperflüssigkeiten, wie Blut, Serum, Urin oder Sputum sollte der Farbstoff wenigstens zu einem Teil wasserlöslich sein. Insbesondere kann es sich um mindestens einen Farbstoff handeln, der hydrophil oder mindestens teilweise wasserlöslich ist. Weiterhin bevorzugt besitzt der Marker eine gute chemische Stabilität sowie eine gute Lichtstabilität und Verfügbarkeit.

In einem weiteren bevorzugten Verfahren ist der Marker ein Farbstoff, insbesondere ausgewählt aus der Gruppe bestehend aus: Cyaninfarbstoffen, Azofarbstoffen und Sulfonfarbstoffen, oder mindestens zwei davon. Diese Farbstoffe sind aufgrund ihrer Ladungsverteilung innerhalb des Farbstoffmoleküls dazu geeignet, Licht in einem bestimmten Wellenlängenbereich zu absorbieren. Durch die Absorption des Lichtes kann ihr Aufenthaltsort bestimmbar gemacht werden. Hierzu kann Licht einer bestimmten Wellenlänge in den nachzuweisenden Bereich eingestrahlt werden und durch eine Intensitätszu- bzw. -abnahme des reflektierten Lichtes in diesem Bereich, in Bezug auf einen vorhergehenden Zeitpunkt, das Vorhandensein des Markers bzw. das Abdiffundieren des Markers nachweisbar gemacht wird. Als Cyaninfarbstoffe kommen sämtliche dem Fachmann, für Nachweiszwecke bekannte Cyaninfarbstoffe in Betracht. Insbesondere Cyaninfarbstoffe ausgewählt aus der Gruppe Streptocyanin oder offenkettiges Cyanin, Hemicyanin und geschlossenkettiges Cyanin, wie Phthalocyanin, Formazan, Porphyrine und 1,1-Diethyl-4,4-Carbocyanin-Iodid sowie mindestens zwei davon. Als Azofarbstoffe kommen sämtliche dem Fachmann, für Nachweiszwecke bekannte Azofarbstoffe in Betracht. Insbesondere Azofarbstoffe ausgwählt aus der Gruppe aliphatischer und aromatischer Azoverbindungen, wie Anilingelb, Methylorange, Azobenzol, Hydroxynaphtholblau, 4-(Dimethylanilin)-azobenzol, Amaranth, Allurarot, Azorubin, Anthocyane, oder Pararot sowie mindestens zwei davon. Als Sulfonfarbstoffe kommen sämtliche dem Fachmann für Nachweiszwecke bekannte Sulfonfarbstoffe in Betracht. Insbesondere Sulfonfarbstoffe aus der Gruppe der aliphatischen und aromatischen Sulfonsäuren, wie Alkylbenzolsulfonsäuren und -solfonate, Brillantblau FCF, Azorubin und Naphthalinsulfonsäure sowie mindestens zwei davon.

In einem besonders bevorzugten Verfahren ist der Marker ausgewählt aus der Gruppe bestehend aus: Erioglaucin, Indigocarmin, Hydroxynaphtholblau, 1,1-Diethyl-4,4-Carbocyanin-Iodid und Amaranth, vorzugsweise Erioglaucin oder Hydroxynaphtholblau, oder mindestens zwei davon. Dabei absorbiert bevorzugt das Erioglaucin Licht der Wellenlänge 625 nm, das Indigocarmin Licht der Wellenlänge 608 nm, das Hydroxynaphtholblau Licht der Wellenlänge 650 nm, das 1,1-Diethyl-4,4-Carbocyanin-Iodid Licht der Wellenlänge 703 bzw. 648 nm sowie das Amaranth Licht der Wellenlänge 521 nm. Diese Farbstoffe weisen in der Regel ein Diffusionsverhalten auf, das nahezu unabhängig von der Anwesenheit bzw. der Konzentration des Analyten ist. Umgekehrt sollte der Marker das Verhalten des Analyten möglichst wenig beeinflussen. Es können jedoch auch andere Farbstoffe eingesetzt werden, die eine Abhängigkeit ihrer Diffusionsgeschwindigkeit in einer Probe von der Temperatur bzw. von partikulären oder zellulären Bestandteilen, insbesondere des Hämatokrits zeigen. Eine Aufstellung der Strukturformeln zu den genannten Farbstoffen und deren Absorptionsmaximum finden sich in der Tabelle 1 im Experimentellen Teil.

In einem bevorzugten Verfahren ist die Probe Blut oder ein Bestandteil hiervon.

Bevorzugt ist der Marker so eingerichtet, dass er möglichst wenig, vorzugsweise gar nicht, mit dem Analyten und/oder mit der Testchemie wechselwirkt, insbesondere reagiert, wobei eine Diffusionsgeschwindigkeit des Markers im Wesentlichen unabhängig von der Konzentration des Analyten ist. Es soll vorzugsweise erreicht werden, dass die Nachweisreaktion durch den Marker und die Diffusion des Markers jeweils ungestört ablaufen. Hierbei ist es bevorzugt, dass die Diffusionsgeschwindigkeit des Markers im Wesentlichen unabhängig von der Konzentration des Analyten ist. So sind insbesondere chemische Reaktionen oder andere Interaktionen zwischen Marker einerseits und Analyten oder Testchemie andererseits in der Regel unerwünscht. Vorzugsweise soll die Wechselwirkung zwischen Marker einerseits und Analyten und Testchemie andererseits so gering sein, dass die mit Analyten und Testchemie ohne Marker erhaltenen Signale um weniger als 3%, vorzugsweise um weniger als 2% und besonders bevorzugt weniger als 1% von Signalen

erhalten aus Messungen mit Analyten und Testchemie in Gegenwart des Markers abweichen.

Um eine Wechselwirkung zwischen dem Marker und dem Analyten möglichst gering zu halten, kann sich der Marker in einem Bereich befinden, der zwar mit der zu analysierenden Probe in Kontakt kommt, jedoch nicht mit der Testchemie. In einem bevorzugten Verfahren kann der Marker ganz oder teilweise separat von der Testchemie angeordnet sein. Der Marker kann beispielsweise zumindest vor Benetzung des Testelementes und/oder des Markers mit Probe ganz oder teilweise separat von der Testchemie angeordnet sein. Auf diese Weise kann zumindest unterbunden werden, dass der Marker von der Testchemie bzw. die Testchemie vom Marker, zumindest während des Benetzungsvorgangs, beeinflusst wird. Die Separation des Markers von der Testchemie kann beispielsweise dadurch erfolgen, dass ein separates Testfeld oder eine separate Testfeldschicht, mit Marker aber ohne Testchemie oder Nachweisreagens auf dem Testelement angeordnet ist. Alternativ oder zusätzlich kann ein eigenes Testelement mit Marker verwendet werden, dass zwar mit der gleichen Probe, möglichst zum gleichen Zeitpunkt und bei gleichen Temperaturbedingungen benetzt wird, aber keine Nachweisreagenzien enthält. Alternativ oder zusätzlich kann eine Beeinflussung der Diffusionsgeschwindigkeit des Markers durch den Analyten zuvor experimentell bestimmt werden und diese in dem Konzentrationsberechnungsverfahren berücksichtigt werden. In einer bevorzugten Ausführungsform des Verfahrens sind der Marker und die Testchemie in dem gleichen Testfeld angeordnet.

In einem bevorzugten Verfahren werden mindestens ein erster Marker und mindestens ein von dem ersten Marker verschiedener weiterer Marker eingesetzt, wobei die Diffusionsgeschwindigkeit des ersten Markers durch mindestens eine erste Eigenschaft der Probe und die Diffusionsgeschwindigkeit des mindestens einen weiteren Markers durch mindestens eine von der ersten Eigenschaft verschiedene weitere Eigenschaft der Probe beeinflusst werden. Durch die Verwendung von mehr als einem Marker in dem erfindungsgemäßen Verfahren können beispielsweise mögliche Beeinflussungen des Markers durch unterschiedliche Eigenschaften der Probe bestimmt werden. Es kann beispielsweise ein erster Marker, insbesondere ausgewählt aus der Gruppe, bestehend aus Erioglaucin, Indigocarmin, Hydroxynaphtholblau, 1,1-Diethyl-4,4-Carbocyanin-Iodid und Amaranth, dazu verwendet werden, um einen Einfluss einer ersten Eigenschaft der Probe auf die Diffusionsgeschwindigkeit des Markers zu ermitteln. Beispielsweise kann diese erste Eigenschaft der Probe einer Temperatur des benetzten Testfeldes und/oder der Probe, oder ein Hämatokrit der Probe sein, um beispielsweise mindestens eine erste Korrekturinformation zu generieren. Der mindestens eine weitere Marker kann beispielsweise ebenfalls ausgewählt sein aus der Gruppe, Erioglaucin, Indigocarmin, Hydroxynaphtholblau, 1,1-Diethyl-4,4-Carbocyanin-Iodid und Amaranth und dazu verwendet werden mindestens eine weitere Eigenschaft der Probe, wie beispielsweise Temperatur oder Hämatokrit der Probe zu bestimmen und beispielsweise mindestens eine zweite Korrekturinformation zu generieren. Bevorzugt wird eine Kombination aus einem ersten Marker, vorzugsweise ein Sulfonfarbstoff wie Erioglaucin, zur Bestimmung des Einflusses der Temperatur auf die Diffusionsgeschwindigkeit eingesetzt und ein weiterer Marker, vorzugsweise ein Azofarbstoff wie Hydroxynaphtholblau, um eine Bestimmung eines weiteren Einflusses, wie beispielsweise den Hämatokrit der Probe, auf die Diffusionsgeschwindigkeit, vornehmen zu können. Zudem entspricht es einer Ausgestaltung des erfindungsgemäßen Verfahrens, dass ein einziger Marker, vorzugsweise ein Sulfonfarbstoff wie Erioglaucin, für die Bestimmung des Einflusses einer ersten und mindestens einer weiteren Eigenschaft, vorzugsweise Temperatur und Hämatokrit, der Probe auf die Diffusionsgeschwindigkeit dieses Markers ausreicht.

Durch die Verwendung von mehr als einem Marker kann eine erhöhte Genauigkeit des Testverfahrens ermöglicht werden. Werden beispielsweise zwei verschiedene Marker benutzt, wobei der eine Marker eine Abhängigkeit der Diffusionsgeschwindigkeit von der Temperatur aufweist, jedoch nur eine geringe Abhängigkeit der Diffusionsgeschwindigkeit aufgrund des Hämatokrit, und ein zweiter Marker, der eine große Hämatokritabhängigkeit der Diffusionsgeschwindigkeit aufweist, aber nur eine geringe Temperaturabhängigkeit der Diffusionsgeschwindigkeit zeigt, so können genauere Korrekturinformationen zur Bestimmung der Konzentration des Analyten erworben werden. Beispielsweise können unterschiedliche Marker mit unterschiedlichen spektralen Eigenschaften verwendet werden, beispielsweise unterschiedlich absorbierende Farbstoffe.

In einem bevorzugten Verfahren wird, wie oben ausgeführt, die Reaktion der Testchemie mit dem Analyten mittels mindestens eines ersten Detektors detektiert, welcher auch als Analytdetektor bezeichnet werden kann. Bezüglich möglicher Ausgestaltungen des Analytdetektors kann auf die obige Beschreibung verwiesen werden. Insbesondere kann der Analytdetektor mindestens ein erstes fotoempfindliches Detektorelement umfassen, beispielsweise mindestens eine erste Fotodiode. Weiterhin kann der Analytdetektor mindestens eine Analytdetektor-Lichtquelle umfassen, beispielsweise mindestens eine erste Leuchtdiode. Die Diffusion des Markers kann mittels mindestens eines zweiten Detektors detektiert werden, welcher auch als Markerdetektor oder Diffusionsdetektor bezeichnet wird. Bezüglich möglicher Ausgestaltungen des Markerdetektors kann auf die obige Beschreibung verwiesen werden. Insbesondere kann der Markerdetektor mindestens ein zweites fotoempfindliches Detektorelement umfassen, beispielsweise mindestens eine zweite Fotodiode. Weiterhin kann der Markerdetektor mindestens eine Markerdetektor-Lichtquelle umfassen, beispielsweise mindestens eine zweite Leuchtdiode. Wie bereits diskutiert, können der erste Detektor und der zweite Detektor getrennt ausgebildet sein oder können auch zumindest teilweise identisch sein. Insbesondere können die Analytdetektor-Lichtquelle und die Markerdetektor-Lichtquelle verschieden ausgebildet werden, wohingegen das erste

fotoempfindliche Detektorelement und das zweite fotoempfindliche Detektorelement bauteilidentisch ausgestaltet werden können. Beispielsweise können auf der Detektionsseite des Testelements die Analytdetektor-Lichtquelle und die Markerdetektor-Lichtquelle zur Beleuchtung des Testelements oder von Teilen desselben vorgesehen sein, sowie ein gemeinsames fotoempfindliches Detektorelement, beispielsweise eine gemeinsame Fotodiode, welches beispielsweise abwechselnd reflektiertes Licht der Markerdetektor-Lichtquelle und der Analytdetektor-Lichtquelle empfängt. Auch andere Ausgestaltungen sind denkbar, beispielsweise Ausgestaltungen, bei welchem unterschiedliche fotoempfindliche Detektorelemente eingesetzt werden.

Wie bereits erwähnt, kann die Detektion des Analyten und/oder des Markers beispielsweise jeweils unabhängig voneinander elektrochemisch und/oder optisch erfolgen. Wenn beispielsweise der Analyt elektrochemisch und der Marker optisch, oder der Analyt optisch und der Marker elektrochemisch analysiert werden sollen, ist in der Regel sowohl mindestens ein elektrochemischer als auch mindestens ein optischer Detektor notwendig. Alternativ können sowohl der Analyt als auch der Marker optisch und/oder elektrochemisch detektiert werden. Hierzu können beispielsweise zwei verschiedene Detektoren verwendet werden, was insbesondere dann vorteilhaft ist, wenn die Wellenlängenbereiche zur Ermittlung der Analytkonzentration von den Wellenlängenbereichen zur Ermittlung der Markerkonzentration sehr unterschiedlich sind. Es ist jedoch beispielsweise auch möglich, einen Detektor zu verwenden, der die Möglichkeit aufweist, bei mehr als einer Wellenlänge Licht zu detektieren. Befinden sich Analyt und Marker nicht in einer gemeinsamen Testchemieschicht, so kann es vorteilhaft sein, die Detektion des Analyten und des Markers von unterschiedlichen Seiten des Testelementes vorzunehmen. Hierzu würde vorteilhafterweise die Detektion des Analyten auf der der Probenaufgabenseite abgewandten Seite erfolgen, während die Detektion des Markers beispielsweise außerhalb des Testelementes stattfinden kann, wie beispielsweise dem Probenüberstand, oder alternativ in einem Bereich des Testfeldes, das näher an der Probenaufgabenseite liegt. Alternativ können auch für den Fall, dass der Marker sich nicht auf oder in dem gleichen Testfeld wie die Nachweisreagenzien befindet, ein oder zwei Detektoren verwendet werden. So kann beispielsweise ein Detektor die Fläche von zwei Testfeldern oder zwei Testelementen abdecken. Dabei können die Bereiche mit Marker mit Licht einer anderen Wellenlänge bestrahlt werden als die Bereiche ohne Marker.

Die Konzentration des Markers und/oder des Nachweisreagenses kann entweder in direktem oder indirektem Wege nachgewiesen werden. Bei einem direkten Nachweis wird ein Verfahren verwendet, welches unmittelbar die Anwesenheit der nachzuweisenden Substanz, in diesem Fall des Markers bzw. des Nachweisreagenses für den Analyten, qualitativ und/oder quantitativ detektiert. Bei einem indirekten Nachweis hingegen wird über mindestens einen gedanklichen, theoretischen oder experimentellen Zwischenschritt qualitativ und/oder quantitativ auf die Anwesenheit des mindestens einen Markers bzw. des einen Nachweisreagenses geschlossen. Beispielsweise kann dies über die Anwesenheit und/oder Bildung und/oder Abnahme von einem oder mehreren weiteren Stoffen geschehen, wobei beispielsweise aus einer Kenntnis über mindestens einen Reaktionsmechanismus wiederum qualitativ und/oder quantitativ auf den Marker bzw. Analyten geschlossen werden kann, so dass dieser indirekt nachgewiesen werden kann. Ein bekanntes Beispiel eines derartigen Nachweisreagenses für den Nachweis von Blutglucose ist Nicotinamid-Adenin-Dinukleotid (NADH), also die reduzierte Form von Nicotinamid-Adenin-Dinukleotid ($NAD^+$), welches beispielsweise direkt photometrisch nachgewiesen werden kann. Insgesamt kann für die Ausgestaltung möglicher Marker bzw. Nachweisreagenzien und Testfelder jedoch weitgehend auf den Stand der Technik verwiesen werden.

In einer bevorzugten Ausgestaltung wird die Reaktion der Testchemie mit dem Analyten mittels mindestens eines ersten Detektors detektiert wird, wobei die Diffusion des Markers mittels mindestens eines weiteren Detektors, insbesondere mittels mindestens eines zweiten Detektors, detektiert wird.

In einem weiteren Aspekt der vorliegenden Erfindung wird ein Testelement zur Verwendung in einem Verfahren gemäß einer oder mehrerer der oben beschriebenen Ausgestaltungen vorgeschlagen, wobei das Testelement mindestens eine Testchemie aufweist, wobei die Testchemie eingerichtet ist, um mindestens eine detektierbare Reaktion mit dem Analyten durchzuführen, wobei das Testelement weiterhin mindestens einen Marker in mindestens einem ersten Bereich des Testelements aufweist, wobei der Marker eingerichtet ist, um mindestens teilweise aus dem ersten Bereich des Testelements in mindestens einen zweiten Bereich zu diffundieren.

Das Testelement weist eine Testchemie auf, wie sie bereits für das Verfahren beschrieben und definiert wurde. Die Testchemie ist dabei eingerichtet, um mindestens eine detektierbare Reaktion mit dem Analyten durchzuführen, wobei das Testelement weiterhin mindestens einen Marker in mindestens einem Bereich des Testelements aufweist. Wie ebenfalls für das Verfahren bereits beschrieben, weist das Testelement bevorzugt ein Testfeld auf, das die Testchemie aufnimmt. In der Testchemie befinden sich ein oder mehrere Nachweisreagenzien zum Nachweis des Analyten in der Probe. In einem Bereich des Testfeldes oder in einem weiteren Bereich des Testelementes befindet sich zusätzlich mindestens ein Marker, wobei der Marker eingerichtet ist, um zumindest teilweise aus dem mindestens ersten Bereich des Testelements in mindestens einen zweiten Bereich zu diffundieren. Dieser zweite Bereich kann, wie bereits für das Verfahren beschrieben, ein Bereich innerhalb des Testfeldes oder außerhalb des Testfeldes sein, bzw. innerhalb des Testelementes bzw. außerhalb des Testelementes sein. Ein bevorzugter Bereich hierfür ist der Überstand der Probe über dem Testelement. So kann beispielsweise der Marker aus einem ersten Bereich des Testelementes in einen zweiten Bereich diffundieren, der beispielsweise ein Überstand der Probe ist. Der Marker kann sich beispielsweise zusammen

mit der Testchemie in einer Testchemieschicht befinden. Der Marker kann insbesondere bei Benetzung des Testelementes mit Probe in die Probe hinein diffundieren, in mindestens einen zweiten Bereich, der entweder innerhalb oder außerhalb des Testelementes liegen kann. Bezüglich möglicher Ausgestaltungen des Markers kann auf die obige Beschreibung verwiesen werden. Insbesondere kann der Marker eine Substanz sein oder umfassen, die zusätzlich zu der Testchemie in das Testelement eingebracht wird. Bevorzugt reagiert und/oder interagiert der Marker nicht mit der Testchemie und/oder Bestandteilen der Testchemie. Hierdurch kann gewährleistet werden, dass das Diffusionsverhalten des Markers im Wesentlichen unabhängig von dem Verhalten der Testchemie und/oder des Analyten ist. Hierdurch wird eine unabhängige Möglichkeit zur Bestimmung von Eigenschaften der Probe gewährleistet, um eine Korrekturinformation zu erhalten, die wie bereits erwähnt eine Genauigkeit der Bestimmung der Konzentration des Analyten ermöglichen kann.

Das Testelement kann insbesondere eine Form aufweisen, wie sie im Stand der Technik grundsätzlich bekannt sein kann, wie beispielsweise die Form eines Teststreifens, eines Testbands, einer Testnadel oder eines Microsamplers, also eines Elements, welches mindestens eine Nadel oder Lanzette und mindestens ein Kapillarelement aufweist. Auch andere Formen des Testelements sind jedoch grundsätzlich möglich.

In einem weiteren Aspekt der Erfindung wird eine Vorrichtung zur Bestimmung mindestens einer Konzentration mindestens eines Analyten in einer Probe, insbesondere unter Verwendung eines Verfahrens nach einer oder mehreren der zuvor beschriebenen Ausführungsformen des Verfahrens zur Bestimmung mindestens einer Konzentration mindestens eines Analyten in einer Probe, vorgeschlagen. Die Vorrichtung umfasst mindestens ein Testelement mit mindestens einer Testchemie, wobei die Vorrichtung eingerichtet ist, um mindestens eine Reaktion der Testchemie mit dem Analyten zu detektieren. Die Vorrichtung umfasst mindestens einen Marker. Die Vorrichtung ist eingerichtet, um mindestens eine Diffusion des Markers in der Probe oder mindestens einem Bestandteil der Probe zu detektieren. Die Vorrichtung ist weiterhin eingerichtet, um aus der Diffusion des Markers mindestens eine Korrekturinformation zu erzeugen. Die Vorrichtung ist weiterhin eingerichtet, um aus der Reaktion der Testchemie mit dem Analyten unter Berücksichtigung der Korrekturinformation die Konzentration des Analyten zu bestimmen.

Das Testelement kann insbesondere ein Testelement wie zuvor beschrieben sein. Zur Detektion der Reaktion der Testchemie auf dem Testelement kann beispielsweise mindestens ein Detektor, hier auch Analytdetektor genannt, in der Vorrichtung vorhanden sein. Dies kann beispielsweise mindestens ein elektrochemischer Analytdetektor und/oder ein optischer Analytdetektor, wie beispielsweise hinlänglich im Stand der Technik beschrieben, sein. Dabei sind beispielsweise Detektoren verwendbar, die Licht bei verschiedenen Wellenlängen detektieren können und/oder zeitaufgelöst Detektionen vornehmen können. Beispielsweise können hierfür Siliciumdioden, wie sie beispielsweise im Stand der Technik unter der Bezeichnung *BPW34* beispielsweise von Osram bekannt sind, verwendet werden. Beispielsweise kann Licht auf die Testchemie und/oder auf den Marker gerichtet werden. Dieses kann entweder monochromatisch sein oder mehr als eine Wellenlänge beinhalten, beispielsweise wenn eine Weißlichtquelle verwendet wird. Bevorzugt absorbieren der Nachweisstoff und der Marker bei unterschiedlichen Wellenlängen das eingestrahlte Licht. Es kann beispielsweise für den Nachweisstoff Licht der Wellenlänge 360 nm eingestrahlt werden und für den Marker eine davon abweichende Wellenlänge, wie sie beispielsweise für die Farbstoffe oben angegeben wurde.

Die Vorrichtung umfasst, wie oben ausgeführt, mindestens einen Marker, welcher vollständig oder teilweise von dem Analyten verschieden ist, beispielsweise strukturchemisch von dem Analyten verschieden ist. Dieser Marker kann sich insbesondere in und/oder auf dem Testelement befinden, kann aber auch ganz oder teilweise separat von dem Testelement in der Vorrichtung enthalten sein. Die Vorrichtung ist eingerichtet, um mindestens eine Diffusion des Markers in der Probe oder mindestens einen Bestandteil der Probe zu detektieren. Hierzu kann die Vorrichtung beispielsweise mindestens einen Markerdetektor umfassen, welcher auch als Diffusionsdetektor bezeichnet werden kann. Bezüglich möglicher Ausgestaltungen des Markerdetektors oder Diffusionsdetektors kann auf die obige Beschreibung verwiesen werden. Der Diffusionsdetektor kann beispielsweise die Diffusion des Markers optisch und/oder elektrochemisch erfassen. Beispielsweise kann der Diffusionsdetektor die Konzentration des Markers in mindestens einem Bereich, beispielsweise in den ersten Bereich und/oder dem zweiten Bereich, zu mindestens zwei Zeitpunkten, beispielsweise als Funktion der Zeit, erfassen. Hierzu kann durch den Detektor beispielsweise eine Reflexion eines eingestrahlten Lichtes über einen bestimmten Zeitraum erfasst werden. Dieser Zeitraum kann beispielsweise von dem Moment der Benetzung des Testelementes bis zu einem bestimmten Zeitpunkt, an dem die Diffusion annähernd abgeschlossen ist, durchgeführt werden. Alternativ können zu vordefinierten Zeitpunkten vor, während und/oder nach der Aufgabe von Probe auf das Testelement Detektorsignale erfasst werden. Handelt es sich bei dem Marker um einen Farbstoff, insbesondere einen Absorptionsfarbstoff, wie er für das Verfahren oben beschrieben wurde, so kann aus den einzelnen Messpunkten bei bekannter Verhaltensweise des Markers in unterschiedlich gearteten Proben, die beispielsweise in Temperatur und ihrer Zusammensetzung variieren, auf die Temperatur bzw. die Zusammensetzung bestimmter Bestandteile der Probe rückgeschlossen werden und/oder auf andere Weise ein Einfluss dieser Eigenschaften der Probe auf die Bestimmung der Analytkonzentration erfasst werden. Alternativ oder zusätzlich kann in bestimmten Bereichen einer Detektionskurve des Detektors, vor, während und/oder nach Benetzung des Testelementes und/oder Markers mit Probe eine Steigung der Detektionskurve ermittelt werden. Es sind weitere statistische Auswertungen der Messsignale möglich, wie sie hinlänglich für kinetische Beobachtungen von Diffusionsvorgängen bekannt sind. Der Analytdetektor

und der Diffusionsdetektor können ein und derselbe Detektor sein und/oder in einem Detektorgehäuse untergebracht sein. Es können aber auch zwei von einander separate Detektoren sein.

Die Vorrichtung ist weiterhin, wie oben ausgeführt, eingerichtet, um aus der Diffusion des Markers mindestens eine Korrekturinformation zu erzeugen, wobei die Vorrichtung weiterhin eingerichtet ist, um aus der Reaktion der Testchemie mit dem Analyten unter Berücksichtigung der Korrekturinformation die Konzentration des Analyten zu bestimmen. In einer bevorzugten Ausgestaltung wird die Reaktion der Testchemie mit dem Analyten mittels mindestens eines ersten Detektors detektiert wird, wobei die Diffusion des Markers mittels mindestens eines weiteren Detektors detektiert wird. Der erste und/oder der weitere Detektor können beispielsweise der zuvor erwähnte mindestens eine Analytdetektor und/oder der mindestens eine Diffusionsdetektor sein. Die Vorrichtung kann zur weiteren Auswertung dieser Detektorsignale beispielsweise mindestens eine Auswertungsvorrichtung, beispielsweise mit mindestens einer Datenverarbeitungsvorrichtung, umfassen. Diese Datenverarbeitungsvorrichtung kann beispielsweise zum einen aus dem Detektorsignal zur Ermittlung der Markerdiffusion eine oder mehrere Korrekturinformationen erzeugen und/oder aus den Daten des Detektors zur Detektion der Reaktion der Testchemie mit dem Analyten die Konzentration des Analyten bestimmen. Je nach Wahl und/oder Anordnung des Markers innerhalb der Vorrichtung, beispielsweise des Testelementes, kann zu der Vermessung der Diffusion des Markers und/oder der Reaktion der Testchemie mit dem Analyten ein und derselbe Detektor verwendet werden, oder es können zwei oder mehr verschiedene Detektoren verwendet werden. Diese Detektoren können sich auf der gleichen Seite des Testelementes oder auf gegenüberliegenden Seiten des Testelementes befinden. In der Datenverarbeitungsvorrichtung können beispielsweise eine Referenzkurve bzw. ein oder mehrere Referenzwerte für die Reaktion der Testchemie mit dem Analyten hinterlegt sein sowie auch Referenzwerte bzw. Referenzkurven für die Diffusion des Markers in der Probe bei verschiedenen Temperaturen bzw. unterschiedlichem Hämatokrit. Um eine genauere Konzentrationsbestimmung des Analyten vorzunehmen, kann die Datenverarbeitungsvorrichtung dazu eingerichtet sein, die berechneten Analytkonzentrationen durch die Korrekturinformation zu korrigieren. Dies kann beispielsweise auf verschiedene, im Stand der Technik grundsätzlich hinlänglich bekannte statistische Methoden erfolgen.

Zusammenfassend sind im Rahmen der vorliegenden Erfindung folgende Ausführungsformen besonders bevorzugt:

Ausführungsform 1: Verfahren zur Bestimmung mindestens einer Konzentration mindestens eines Analyten in einer Probe, insbesondere mindestens eines Metaboliten in einer Probe einer Körperflüssigkeit, wobei mindestens ein Testelement mit mindestens einer Testchemie verwendet wird, wobei mindestens eine Reaktion der Testchemie mit dem Analyten detektiert wird, wobei weiterhin mindestens ein Marker verwendet wird, wobei der Marker von dem Analyten verschieden ist, wobei der Marker mindestens eine chemische Verbindung aufweist, wobei mindestens eine Diffusion des Markers in der Probe oder mindestens einem Teil der Probe detektiert wird, wobei die Detektion der Reaktion des Analyten elektrochemisch und/oder optisch erfolgt, und/oder dass die Detektion der Diffusion des Markers elektrochemisch und/oder optisch erfolgt, wobei aus der Diffusion mindestens eine Korrekturinformation erzeugt wird, wobei aus der Reaktion der Testchemie mit dem Analyten unter Berücksichtigung der Korrekturinformation die Konzentration des Analyten bestimmt wird, wobei die Korrekturinformation ausgestaltet ist, um einen Einfluss der Temperatur der Probe und/oder des Testelements bei der Bestimmung der Konzentration des Analyten zu berücksichtigen, wobei die Korrekturinformation mindestens eine Information über mindestens eine Störgröße der Probe umfasst, wobei die Störgröße eine Temperatur der Probe und/oder des Testelements umfasst, wobei die Korrekturinformation unter Verwendung folgender Verfahrensschritte bestimmt wird:

- in mindestens einer Eichmessung wird ein allgemeiner Zusammenhang zwischen der Störgröße und der Diffusion des Markers erfasst;
- in der Probe, in welcher die Konzentration des Analyten bestimmt werden soll, wird sowohl die Reaktion der Testchemie mit dem Analyten als auch die Diffusion des Markers detektiert;
- die Korrekturinformation wird aus der detektierten Diffusion des Markers in der Probe und dem allgemeinen Zusammenhang zwischen der Störgröße und der Diffusion des Markers bestimmt.

Ausführungsform 2: Verfahren nach einer der vorhergehenden Ausführungsformen, dadurch gekennzeichnet, dass das Testelement mindestens ein Testfeld mit mindestens einer Testchemieschicht umfasst, wobei die Testchemieschicht die Testchemie umfasst, wobei die Testchemie insbesondere mindestens ein Enzym beinhaltet, insbesondere ausgewählt aus der Gruppe, bestehend aus: Glucosedehydrogenase, Glucoseoxidase.

Ausführungsform 3: Verfahren nach einer der vorhergehenden Ausführungsformen, dadurch gekennzeichnet, dass der Marker in mindestens einem ersten Bereich des Testelements enthalten ist, wobei die Testchemie ebenfalls ganz oder teilweise in dem ersten Bereich enthalten ist.

Ausführungsform 4: Verfahren nach einer der vorhergehenden Ausführungsformen, dadurch gekennzeichnet, dass der Marker ganz oder teilweise separat von der Testchemie angeordnet ist.

Ausführungsformen 5: Verfahren nach einer der vorhergehenden Ausführungsformen, dadurch gekennzeichnet, dass der Marker nicht mit dem Analyten und/oder mit der Testchemie reagiert, wobei eine Diffusionsgeschwindigkeit des Markers im Wesentlichen unabhängig von der Konzentration des Analyten ist.

Ausführungsform 6: Verfahren nach einer der vorhergehenden Ausführungsformen, dadurch gekennzeichnet, dass der Marker mindestens einen optisch nachweisbaren Farbstoff umfasst, insbesondere mindestens einen Farbstoff der hydrophil oder wasserlöslich ist.

Ausführungsform 7: Verfahren nach einer der vorhergehenden Ausführungsformen, dadurch gekennzeichnet, dass der Marker in einem Wellenlängenbereich von 300 bis 800 nm Licht absorbiert.

Ausführungsform 8: Verfahren nach einer der vorhergehenden Ausführungsformen, dadurch gekennzeichnet, dass der Marker ein Farbstoff ist, insbesondere ausgewählt aus der Gruppe bestehend aus: Cyaninfarbstoffen, Azofarbstoffen und Sulfonfarbstoffen oder mindestens zwei davon.

Ausführungsform 9: Verfahren nach einer der vorhergehenden Ausführungsformen, dadurch gekennzeichnet, dass der Marker ausgewählt ist aus der Gruppe bestehend aus: Erioglaucin, Indigocarmin, Hydroxynaphtholblau, 1,1-Diethyl-4,4-Carbocyanin-Iodid und Amaranth, vorzugsweise Erioglaucin oder Hydroxynaphtholblau, oder mindestens zwei davon.

Ausführungsform 10: Verfahren nach einer der vorhergehenden Ausführungsformen, dadurch gekennzeichnet, dass mindestens ein erster Marker und mindestens ein von dem ersten Marker verschiedener weiterer Marker eingesetzt werden, wobei die Diffusionsgeschwindigkeit des ersten Markers durch mindestens eine erste Eigenschaft der Probe und die Diffusionsgeschwindigkeit des mindestens einen weiteren Markers durch mindestens eine von der ersten Eigenschaft verschiedene weitere Eigenschaft der Probe beeinflusst werden, wobei die Eigenschaft der Probe eine Temperatur der Probe umfasst.

Ausführungsform 11: Verfahren nach einer der vorhergehenden Ausführungsformen, dadurch gekennzeichnet, dass die Reaktion der Testchemie mit dem Analyten mittels mindestens eines ersten Detektors detektiert wird, wobei die Diffusion des Markers mittels mindestens eines weiteren Detektors detektiert wird.

Ausführungsform 12: Vorrichtung zur Bestimmung mindestens einer Konzentration mindestens eines Analyten in einer Probe, insbesondere unter Verwendung eines Verfahrens nach einer der vorhergehenden, ein Verfahren betreffenden Ausführungsformen, wobei die Vorrichtung mindestens ein Testelement mit mindestens einer Testchemie umfasst, wobei die Vorrichtung eingerichtet ist, um mindestens eine Reaktion der Testchemie mit dem Analyten zu detektieren, wobei die Vorrichtung mindestens einen Marker umfasst, wobei der Marker von dem Analyten verschieden ist, wobei der Marker mindestens eine chemische Verbindung aufweist, wobei die Vorrichtung eingerichtet ist, um mindestens eine Diffusion des Markers in der Probe oder mindestens einem Bestandteil der Probe zu detektieren, wobei die Detektion der Reaktion des Analyten elektrochemisch und/oder optisch erfolgt und/oder dass die Detektion der Diffusion des Markers elektrochemisch und/oder optisch erfolgt, wobei die Vorrichtung eingerichtet ist, um aus der Diffusion des Markers mindestens eine Korrekturinformation zu erzeugen, wobei die Vorrichtung weiterhin eingerichtet ist, um aus der Reaktion der Testchemie mit dem Analyten unter Berücksichtigung der Korrekturinformation die Konzentration des Analyten zu bestimmen, wobei die Korrekturinformation ausgestaltet ist, um einen Einfluss der Temperatur der Probe und/oder des Testelements bei der Bestimmung der Konzentration des Analyten zu berücksichtigen, wobei die Korrekturinformation mindestens eine Information über mindestens eine Störgröße der Probe umfasst, wobei die Störgröße eine Temperatur der Probe und/oder des Testelements umfasst, wobei die Vorrichtung eingerichtet ist, um die Korrekturinformation unter Verwendung folgender Verfahrensschritte zu bestimmen:

- in mindestens einer Eichmessung wird ein allgemeiner Zusammenhang zwischen der Störgröße und der Diffusion des Markers erfasst;
- in der Probe, in welcher die Konzentration des Analyten bestimmt werden soll, wird sowohl die Reaktion der Testchemie mit dem Analyten als auch die Diffusion des Markers detektiert;
- die Korrekturinformation wird aus der detektierten Diffusion des Markers in der Probe und dem allgemeinen Zusammenhang zwischen der Störgröße und der Diffusion des Markers bestimmt.

Kurze Beschreibung der Figuren

Weitere Einzelheiten und Merkmale der Erfindung ergeben sich aus der nachfolgenden Beschreibung von bevorzugten Ausführungsbeispielen, insbesondere in Verbindung mit den Unteransprüchen. Hierbei können die jeweiligen Merkmale für sich alleine oder zu mehreren in Kombination miteinander verwirklicht sein. Die Erfindung ist nicht auf die Ausführungsbeispiele beschränkt. Die Ausführungsbeispiele sind in den Figuren schematisch dargestellt. Gleiche Bezugsziffern in den einzelnen Figuren bezeichnen dabei gleiche oder funktionsgleiche beziehungsweise hinsichtlich ihrer Funktionen einander entsprechende Elemente.

Im Einzelnen zeigen:

Figur 1a eine schematische Darstellung eines Testelementes mit einem Testfeld;

Figur 1b eine schematische Darstellung eines Testfeldes mit Marker vor Benetzung des Testelementes;

Figur 1c eine schematische Darstellung eines Testfeldes mit einem Marker nach Benetzung des Testfeldes;

Figur 1d: eine schematische Darstellung einer Vorrichtung mit einem Testelement;

Figur 1e: eine schematische Darstellung einer Vorrichtung mit einem alternativen Testelement;

Figur 1f: eine Abwandlung des Ausführungsbeispiels gemäß Figur 1e;

Figur 2 Kurvenverläufe nach einer Benetzung eines mit 0,05 % Erioglaucin versehenen Testfeldes bei verschiedenen Temperaturen mit Proben unterschiedlichen Glucosegehalts;

Figur 3 Kurvenverläufe eines mit 0,1 % Erioglaucin versehenen Testfeldes nach Benetzung mit Blut bei verschiedenen Temperaturen;

Figur 4 Kurvenverläufe eines mit Hydroxynaphtholblau dotierten Testfeldes bei verschiedenen Temperaturen;

Figur 5 Kurvenverläufe eines mit 0,05 % Erioglaucin dotierten Testfeldes nach Benetzung mit Blut bzw. Wasser bei verschiedenen Temperaturen;

Figur 6 Kurvenverläufe eines mit 0,05 % Erioglaucin dotierten Testfeldes mit Blut mit verschiedenem Hämatokrit;

Figur 7 Kurvenverläufe von Messungen an verschiedenen Testfeldern, die mit 0,05 % Erioglaucin mit verschiedenen Hämatokritzusätzen bei verschiedenen Glucosekonzentrationen aufgenommen wurden;

Figur 8 Kurvenverläufe von mit Erioglaucin dotierten Testfeldern mit unterschiedlichem Hämatokrit bei verschiedenen gemittelten Glucosekonzentrationen;

Figur 9 Kurvenverläufe von Testfeldern, die mit 0,05 % und 0,1 % Erioglaucin versetzt wurden;

Figur 10 eine schematische Darstellung des Einflusses von Temperatur und Hämatokrit auf das Analytsignal nach 4 sec;

Figur 11 eine schematische Darstellung des Einflusses von Temperatur und Hämatokrit auf das Remissionsverhalten eines Markers;

Figur 12 eine Korrelation zwischen einem Systemfehler bei einer Glucosemessung und einer Remissionsmessung an einem Markerfeld bei unterschiedlichen Hämatokritwerten;

Figur 13 eine Darstellung des Systemfehlers eines herkömmlichen Testelementsystems bei verschiedenen Hämatokrit ohne Verwendung der Korrekturinformation des Markers; und

Figur 14 eine Darstellung des Systemfehlers des erfindungsgemäßen Testelementsystems bei verschiedenen Hämatokrit unter Verwendung der Korrekturinformation des Markers.

Ausführungsbeispiele

In Figur 1a ist ein Testelement 50 zum Nachweis mindestens eines Analyten 117 in einer Probe 110 gezeigt, auf dem ein Testfeld 100 auf einem Testträger 60 angeordnet ist. Dieses Testelement 50 ist dazu eingerichtet in einer Vorrichtung 190 aus Figur 1d eingesetzt zu werden. Das Testfeld 100 beinhaltet eine Testchemie 102. In dieser Testchemie 102 befinden sich, wie bereits beschrieben, eine oder mehrere Nachweisreagenzien, bevorzugterweise ein Enzym, das den Analyten 117 umsetzt und so auf eine Analytkonzentration schließen lässt. Zusätzlich zu der Testchemie 102 kann sich auch mindestens ein Marker 104, 104' in oder auf dem Testfeld 100 befinden. Der Marker 104, 104' kann sich jedoch auch auf einem separaten Testelement 50 befinden oder an einer anderen Stelle auf dem Testträger 60 des gezeigten Testelementes 50. Der Marker 104, 104' kann zusammen mit der Testchemie 102 angeordnet sein und auf diese Weise beispielsweise mindestens eine Testchemieschicht 118 bilden. Die Testchemieschicht 118 wird im Folgenden auch als Reaktivschicht bezeichnet. Alternativ kann der Marker 104, 104' auch beispielsweise in einer separaten Schicht angeordnet sein und eine separate Markerschicht 120 bilden, wie in Figur 1b und 1c gezeigt. Die Testchemieschicht 118, in der sich die Testchemie 102 befindet wird aufgrund ihrer Reaktivität auch als Reaktivschicht 118 bezeichnet. Hieran angrenzend befindet sich eine Pigmentschicht 122. Die Pigmentschicht 122 kann auch beabstandet zu der Testchemieschicht 118 sein. In der Pigmentschicht 122 ist beispielsweise Pigment eingebracht, wie beispielsweise $TiO_2$ oder $ZrO_2$. Die Pigmentschicht 122 dient bei einer optischen Detektion dazu, wie in Fig. 1d gezeigt, das eingestrahlte Licht 150b, das sowohl durch den Testträger 60 als auch die Reaktivschicht 118 dringt, zu reflektieren. Hierdurch wird gewährleistet, dass das Licht 150b der Lichtquelle 140 zwar mit den Nachweisreagenzien in der Reaktiv- oder Testchemieschicht 118 interagieren kann, aber nicht durch andere Bestandteile des Blutes 110 beeinflusst wird. Diese störenden Bestandteile, wie beispielsweise rote Blutkörperchen, können durch die Abtrennschicht 106 von der Reaktivschicht 118 ferngehalten werden.

In den Figuren 1b und 1c ist jeweils ein Testfeld 100 auf einem Testträger 60 eines Testelementes 50 gezeigt, wie es in der Vorrichtung 190 aus Figur 1d vermessen werden kann. Das Testfeld 100 aus Figur 1b zeigt den Zustand des Testfeldes 100 vor Benetzung 108 mit einer Probe 110. Das Testfeld 100 in Figur 1c zeigt den Zustand nach Benetzung 108 des Testfeldes 100 mit Probe 110. Dabei kann die Probe 110 vorzugsweise aus Blut bestehen. Der Marker 104, 104' befindet sich bevorzugterweise in einem ersten Bereich 114 des Testfeldes 100, in dem sich bevorzugterweise ebenfalls die Testchemie 102 befinden kann. Alternativ kann die Testchemie 102 auch in einer separaten Schicht 118 bzw. in einem zweiten Bereich 116 des Testfeldes 100 untergebracht sein. Ist der Marker 104, 104' in einer separaten Schicht 120 untergebracht, so wird von einer Markerschicht 120 gesprochen, von der getrennt die Testchemieschicht oder Reaktivschicht 118 unterschieden wird. Sind Marker 104, 104' und Testchemie 102 in ein und derselben Schicht untergebracht, so wird nur von einer Testchemieschicht 118, 120 gesprochen. In den Figuren 1a, 1b und 1c (hier nicht gezeigt), können jedoch über, zwischen oder unter den beiden Schichten 118 und 120 eine oder mehrere weitere Schichten vorhanden sein. Des Weiteren können weitere Zusatzstoffe wie Pigmente, Füllstoffe, Hilfsstoffe und andere Substanzen in sowohl der Testchemieschicht 118 als auch der Markerschicht 120 vorhanden sein, wodurch eine Pigmentschicht 122 entsteht. Alternativ können die zusätzlichen Substanzen in nur einer oder in mehreren der Schichten 118, 120 vorhanden sein. Wie aus den Figuren 1b und 1c ersichtlich, verteilt sich der Marker 104, 104' nach Benetzung 108 mit Probe 110 aus dem ersten Bereich 114 des Testfeldes 100, in diesem Fall der Testchemieschicht 118, in mindestens einen zweiten Bereich 116. Dieser zweite Bereich 116 kann sich direkt benachbart zum ersten Bereich 114 befinden oder von diesem ersten Bereich 114 beabstandet sein. Bevorzugterweise ist der Marker 104, 104' so ausgestaltet, dass er durch alle Schichten des Testfeldes 100 hindurch diffundieren kann. Es kann der zweite Bereich 116 auch der Überstand 112 des von dem Testfeld 100 nicht aufgenommenen Blutes 110 sein, das sich an der Probenaufgabeseite 107 angesammelt hat. Alternativ oder zusätzlich kann der zweite Bereich 116, in den der Marker 104, 104' nach Kontakt mit der Probe 110 diffundiert und detektiert werden kann, außerhalb des Testelementes 50 liegen. Es können sich weitere Schichten zwischen dem ersten 114 und zweiten Bereich 116 befinden, wie beispielsweise die Abtrennschicht 106. Der zweite Bereich 116 kann alternativ oder zusätzlich auch Bestandteil des Testelementes 50 und/oder Testfeldes 100 sein.

Zur Detektion des diffundierenden Markers 104, 104' kann einer oder beide der Bereiche 114 und/oder 116 herangezogen werden. Wird der erste Bereich 114 hierzu herangezogen, so wird auf optischem oder elektrochemischem Wege ein "Ausbluten" von Marker 104, 104' aus dem ersten Bereich 114 detektiert. Unter einem Ausbluten ist hier eine Diffusion des Markers 104, 104' aus dem ersten Bereich 114 zu verstehen. Je nach verwendetem Marker 104, 104' kann dies zu einer Zunahme oder Abnahme des Messsignals führen. Alternativ kann in einem Teil oder dem ganzen zweiten Bereich 116 das Hineindiffundieren des Markers 104, 104' detektiert werden. Je nachdem, ob es sich um einen Absorptionsmarker handelt, dessen Reflexion 160a von eingestrahltem Licht 150a detektiert wird, oder beispielsweise um einen Fluoreszenzfarbstoff, dessen Umwandlung des eingestrahlten Lichtes zu einer anderen Wellenlänge hin detektiert wird, nimmt das Messsignal während des Diffusionsvorgangs zu bzw. ab. Umgekehrt wird das Messsignal ab- bzw. zunehmen, wenn die Detektion des Absorptionsfarbstoffes bzw. Fluoreszenzfarbstoffes in dem zweiten Bereich 116 stattfindet. Alternativ können beide Bereiche 114 und 116 zur Detektion der Diffusion des Markers 104, 104' verwendet werden.

Alternativ zu der optischen Detektion kann, wie bereits erwähnt, die Detektion der Diffusion des Markers 104, 104' auch elektrochemisch erfolgen. Hierzu würde beispielsweise eine Elektrode, die in dem ersten Bereich 114 untergebracht ist, das Ausbluten des ersten Bereiches 114 an Marker 104, 104' nach Benetzung 108 detektieren.

In Figur 1d wird eine Vorrichtung 190 gezeigt, die zwei Lichtquellen 130 und 140 in einem Gehäuse 188 auf je einer Seite eines Testelementes 50 aufweist, das in das Gehäuse eingeschoben ist. In dieser Vorrichtung 190 kann von der ersten Lichtquelle 130 Licht 150a auf die Oberseite eines in der Vorrichtung 190 zu vermessenden Testelementes 50 gestrahlt werden. Das ausgehende Licht 150a der ersten Lichtquelle 130 trifft auf die Oberfläche des Testfeldes 100 des Testelementes 50 und wird dort reflektiert. Der an dem Testfeld 100 reflektierte Lichtstrahl 160a wird von einem ersten Detektor 170 aufgefangen. Auf dem Weg von der ersten Lichtquelle 130 bis zum ersten Detektor 170 bewegt sich das Licht 150a zum Teil durch die Probe 110 und/oder den Überstand der Probe 112, der gleichzeitig einen zweiten Bereich 116 der Vorrichtung 190 darstellen kann. Dabei kann das Licht 150a, 160a auf Marker 104, 104' treffen, der beispielsweise nach Benetzung 108 mit Probe 110 aus dem ersten Bereich 114 in den zweiten Bereich 116 diffundiert ist und nun einen Teil des eingestrahlten Lichtes 150a und/oder reflektierten Lichtes 160a absorbiert.

Alternativ oder zusätzlich kann das Ausbluten an Marker 104, 104' nach Benetzung 108 des Testfeldes 100 mit einem zweiten Detektor 180 detektiert werden. Dies wird bevorzugt vorgenommen, wenn der Marker 104, 104' zusammen mit der Testchemie 102 in der Testchemie- oder Reaktivschicht 118 eingebracht ist. Hierzu wird auf der Unterseite des Testelements 50, gegenüber der Probenaufgabeseite 107, Licht 150b einer zweiten Lichtquelle 140 durch den Testträger 60 und die Testchemie 102 in der Testchemie- oder Reaktivschicht 118 gestrahlt und an der Pigmentschicht 122 reflektiert. Das an der Pigmentschicht 122 reflektierte Licht 160b der zweiten Lichtquelle 140 wird von dem zweiten Detektor 180 aufgefangen. Auf diese Weise kann sowohl das Licht für die Nachweisreaktion detektiert werden, das Rückschlüsse auf die umgesetzte Menge des Analyten 117 gibt, als auch das vom Marker 104, 104' beeinflusste Licht oder die beeinflusste Strahlung, das die Diffusion des Markers 104, 104' widerspiegelt. Wenn folglich Lichtquelle 140 und Detektor 180 ausgestaltet sind, um sowohl Licht bei der entsprechenden Wellenlänge zur Detektion des Nachweisreagenzes der Testchemie 102 als auch zur Detektion des Markers 104, 104' auszustrahlen bzw. zu detektieren, so kann eine Lichtquelle 140 und ein Detektor 180 ausreichen, um sowohl die Nachweisreaktion des Analyten 117, als auch die Markerdiffusion nach Benetzung 108 des Testfeldes 100 zu beobachten.

In den oder anschließend an die Detektoren 170 und/oder 180 können sich zusätzlich Auswerteeinheiten 175 und/oder 185 befinden, die das Signal beispielsweise mit dort hinterlegten Referenzkurven oder Referenzwerten vergleichen.

Als Detektoren 170 und 180 können beispielsweise Fotodioden, CCD-Sensoren (Charge coupled device) oder CMOS-Sensoren (Complementary Metal Oxide Semiconductor) eingesetzt werden. Als Lichtquellen 130 und/oder 140 können beispielsweise Leuchtdioden, Quecksilberlampen oder sonstige Lichtquellen mit den gewünschten Wellenlängen eingesetzt werden. Wie bereits erwähnt, kann sich in oder an den Detektoren 170 und/oder 180 anschließend eine Auswerteeinheit 175 und/oder 185 befinden. Diese Auswerteeinheit 175 und/oder 185 kann beispielsweise ein Mikroprozessor oder eine andere geeignete Recheneinheit sein. Es ist jedoch möglich, dass sich in dem Detektor lediglich ein Speicher befindet, der Daten an eine externe Auswerteeinheit (hier nicht gezeigt) übermittelt oder von ihr ausgelesen werden kann. Das Testelement 50 kann nach der Messung aus dem Gehäuse 188 wieder herausgenommen werden, um ein weiteres Testelement 50 für eine weitere Messung einschieben zu können. Das Gehäuse 188 dient vor allem als Schutz für die elektronischen Teile innerhalb des Gehäuses 188, wie Detektoren 170 und/oder 180 mit Auswerteeinheit 175 und/oder 185 sowie Lichtquellen 130 und/oder 140. Weiterhin dient das Gehäuse 188 aber auch als Schutz für den Benutzer, um einer Fehlbedienung vorzubeugen. Das Gehäuse 188 weist bevorzugterweise eine Öffnung 189 auf, um das Testelement 50 mit dem Testfeld 100 in das Gehäuse 188 einbringen zu können.

Alternativ zu der Anordnung des Markers 104, 104' in einem begrenzten Bereich des Testfeldes 100, wie es in der Figur 1b gezeigt ist, kann sich der Marker 104, 104' im gesamten Testfeld 100 des Testelements 50 befinden, so dass das Ausbluten des Testfeldes 100 an Marker 104, 104' detektiert werden kann, wobei der Überstand 112 nach Benetzung 108 des Testfeldes 100 als zweiter Bereich 116 dienen würde.

Eine alternative Anordnung von Testchemie 102 und Marker 104, 104' wird in den Figuren Figur 1e und 1f gezeigt. Hier ist ein Testelement 50 gezeigt, das einen Testträger 60 aufweist, auf dem ein Testfeld 100 angeordnet ist, in dem nebeneinander eine Markerschicht 120 und eine Reaktivschicht 118 angeordnet sind. In der Markerschicht 120 befindet sich der Marker 104, 104', der optional durch eine Barriere 101 von der Reaktivschicht 118 getrennt sein kann. Bevorzugt weist das Testelement 50 keine Barriere 101 zwischen Markerschicht 120 und Reaktivschicht 118 auf. Die optionale Barriere 101 kann bewirken, dass vor und/oder nach Benetzung des Testfeldes 100 mit Probe 110 möglichst kein Stoffaustausch zwischen Markerschicht 120 und Reaktivschicht 118 stattfindet. Nach Benetzung des Testfeldes 100 ist es üblicherweise akzeptabel, dass ein Stoffaustausch durch Diffusion jedoch stattfinden kann. Diese Anordnung der Markerschicht 120 neben der Reaktivschicht 118 bewirkt, dass diese beiden Schichten unabhängig voneinander durch jeweils eine Lichtquelle 130 und 140 angestrahlt werden können. So wird die Markerschicht 120 von dem eingestrahlten Licht 150a der ersten Lichtquelle 130 angestrahlt und die Reaktivschicht 118 von dem eingestrahlten Licht

150b der zweiten Lichtquelle 140. Die Markerschicht 120 und die Reaktivschicht 118 können gleiches oder unterschiedliches Volumen und/oder gleiche oder unterschiedliche Schichtdicke aufweisen. In Figur 1e ist eine Ausgestaltung gezeigt, bei welcher die Reaktivschicht 118 und die Markerschicht 120 eine unterschiedliche Schichtdicke aufweisen, und in Figur 1f eine Ausgestaltung, bei welcher die Reaktivschicht 118 und die Markerschicht 120 eine im Wesentlichen gleiche Schichtdicke aufweisen. Bevorzugt weisen die Markerschicht 120 und die Reaktivschicht 118 eine im Wesentlichen gleiche Schichtdicke und/oder ein im Wesentlichen gleiches Volumen auf. Unter im Wesentlichen gleich kann dabei beispielsweise eine Identität verstanden werden oder auch eine Abweichung der Schichtdicken bzw. der Volumina voneinander um nicht mehr als 20 %, vorzugsweise um nicht mehr als 10 % und besonders bevorzugt um nicht mehr als 5 %. Der Marker 104, 104' kann nach Benetzung des Testfeldes 100 mit der Probe 110 aus dem ersten Bereich 114, beispielsweise mindestens eines Teils der Markerschicht 120, in den zweiten Bereich 116 diffundieren. Der zweite Bereich 116 kann den Überstand 112 der Probe 110 umfassen und/oder eine weitere Schicht 116 oberhalb und/oder seitlich (hier nicht gezeigt) der Markerschicht 120. Wenn die Barriere 101 so ausgestaltet ist, dass sie für den Marker 104, 104' durchlässig ist, so kann auch die Reaktivschicht 118 mindestens als Teil des zweiten Bereichs 116 dienen, in den der Marker 104, 104' nach Benetzung diffundieren kann. Umgekehrt kann sich ein Teil des zu detektierenden Analyts sowohl in dem Überstand 112 als auch in dem zweiten Bereich 116 befinden.

Bevorzugt sind der Bereich in den der Marker 104, 104' diffundieren kann und der Bereich in den der Analyt 117 diffundieren kann gleich groß. Die Größenverhältnisse der Schichten 116 und 118 sind in Figur 1e nicht notwendigerweise für alle denkbaren Ausführungsformen mit nebeneinander angeordneter Markerschicht 120 und Reaktivschicht 118 maßstabsgetreu wiedergegeben. So können der zweite Bereich 116 zusammen mit dem ersten Bereich 114 bzw. der Markerschicht 120 die gleiche Schichtdicke und/oder das gleiche Volumen aufweisen wie die Reaktivschicht 118. Auch eine andere Ausgestaltung ist jedoch denkbar. In einer alternativen Ausführungsform kann die Markerschicht 120 für sich gesehen die gleiche Schichtdicke und/oder das gleiche Volumen aufweisen wie die Reaktivschicht 118 (siehe Figur 1f). Auch diesbezüglich ist jedoch eine andere Ausgestaltung denkbar. Zur Detektion des reflektierten Lichtes in Form des reflektierten Lichtes der ersten Lichtquelle 160a und der zweiten Lichtquelle 160b dient in diesem Fall ein Detektor 170. Zusätzlich könnte auch ein weiterer Detektor verwendet werden, so dass die reflektierten Lichtstrahlen 150b und 160b getrennt von einander aufgefangen werden können.

In den Figuren 2 bis 9 sind Kurvenverläufe oder Kinetiken für das Herausfließen verschiedener Farbstoffe aus einer Marker- bzw. Testchemieschicht 118, 120 in einem herkömmlichen Testfeld 100, in den Überstand der Probe 112 gezeigt. Dabei befindet sich der Marker 104, 104' vor Benetzung 108 des Testfeldes 100 in der Testchemieschicht 118. Die Zusammensetzungen der verwendeten Schichten 118 sind in Tabelle 1 und 2 angegeben. Die Kurven in den Figuren 2-9 wurden in Remission detektiert. Unter einer Detektion in Remission oder einer Detektion eines Remissionswerts wird dabei allgemein eine optische Messung bezeichnet, welche auf einer Reflexion von Wellen, insbesondere von Licht, basiert, insbesondere einer Reflexion diffuser, ungerichteter Wellen. Die Remission kann auf verschiedene Weise detektiert werden, wobei beispielsweise mindestens ein Signal eines Detektors erfasst wird, welcher reflektiertes Licht detektiert. Die Remission kann, wie in den Figuren 2-9 auf der vertikalen Achse angegeben, beispielsweise einfach als Signal I in willkürlichen Einheiten (symbolisiert durch "[-]") angegeben werden. Alternativ könnte die Remission auch in anderen Einheiten angegeben werden, beispielsweise in Form eines oberflächenbezogenen Maßes für die Remission, also eines Remissionsgrads. Auch ein Verhältnis von remittierter zu eingestrahlter Energie in Prozent könnte angegeben werden, beispielsweise ein so genannter Albedo-Wert.

Die Schichten wurden bei 660 nm mit einem Messgerät, hier beispielsweise einem so genannten Gen5Red-Meßgerät, mit einer LED, die Licht bei einer Wellenlänge von 660 nm ausstrahlt, angeregt und in Remission mit Hilfe eines Siliciumdetektors BPW34 detektiert. Zur Vermessung des Verhaltens der verschiedenen Farbstoffe wurden Testelemente 50 mit Testfeldern 100 auf einem Testträger 60, wie unter Figur 1a. 1b, 1c und 1d beschrieben, verwendet. In verschiedenen Experimenten wurde auf die Reaktivschicht 118 eine weitere Testchemieschicht 118, 120 aufgetragen, die neben den Nachweisreagenzien noch einen unterschiedlichen Prozentsatz an Farbstoff enthielt (0,05 % oder 0,1 %). Diese wird im Folgenden Markerschicht 120 genannt. Es werden 2 unterschiedliche Schichten, deren Zusammensetzung in Tabelle 2 und 3 angegeben sind, durch Rakeln bei einer Geschwindigkeit von 5m/min auf eine Pokalonfolie, die als Testträger 60 dient, aufgebracht. Diese Pokalonfolie des Typs N332EM von Lonza ist einseitig matt, coronabehandelt und 140 $\mu$m dick. In den Rezepturen der Testchemieschicht 118 und der Markerschicht 120 wurde Glucosedehydrogenase zusammen mit dem Coenzym cNAD (carba-NAD) eingesetzt. Diese wurde photometrisch bei 360 nm von der, der Probenaufgabeseite gegenüberliegenden Seite, mit Hilfe eines BPW34 Detektorsvermessen. Die Diffusion des Markers 104, 104' wurde mit Hilfe einer Leuchtdiode und einem Detektor auf der Testfeldunterseite, also gegenüber der Probenaufgabeseite vermessen.

Es wurden Farbstoffe ausgewählt, deren Absorptionsmaximum deutlich unterschiedlich zu der Wellenlänge ist, bei der die Glucosereaktion detektiert wird. Außerdem weist das Nachweisreagens dort auch keine nennenswerte Absorption auf. Weitere Kriterien für den ausgewählten Farbstoff sind Wasserlöslichkeit und eine gute chemische und Lichtstabilität sowie Verfügbarkeit.

In der folgenden Tabelle 1 sind die untersuchten Farbstoffe mit ihren Strukturformeln und Absorptionsmaxima

in unterschiedlichen Lösungsmitteln angegeben.

*Tabelle 1: Name, Strukturformel, Absorptionsmaximum in den in Klammern angegebenen Medien*

| Farbstoff | Struktur | Absorption |
|---|---|---|
| Erioglaucin | 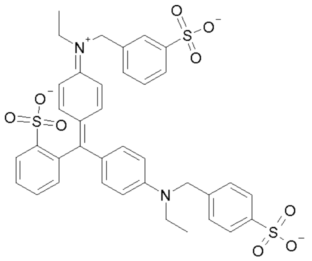 | 625 nm (H2O) |
| Indigocarmine | 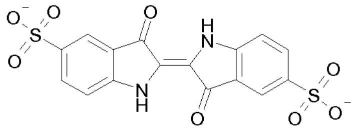 | 608 nm (H2O) |
| Hydroxynaphthol blue | 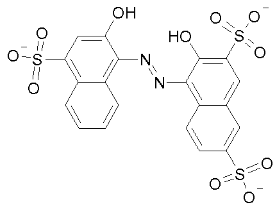 | 650 nm (Me-OH/H2O) |
| 1,1-Diethyl-4,4-Carbocyanin-Iodid (1260) | 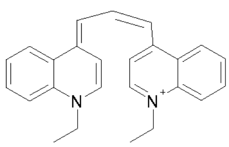 | 703 (648) nm (Me-OH) |
| Amaranth | 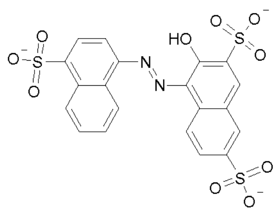 | 521 nm (H2O) |

Diese Farbstoffe wurden den Testfeldern 100, der in Figur 2-9 untersuchten Testelementen 50 in verschiedenen Verhältnissen als Trockensubstanz zugemischt. Der Farbstoffanteil wird bei den einzelnen Figurenbeschreibungen mit angegeben.

Der Aufbau der Testchemieschicht 118 und Markerschicht 120 für die nachfolgenden Experimente wird in den beiden folgenden Tabellen 2 und 3 angegeben.

*Tabelle 2: Rezeptur für die 1. Testchemieschicht, wobei: RF: Reihenfolge; Subst: Substanz; MV: Masterverhältnis; Konz: Konzentration; A-Rez: Arbeitsrezeptur; Rez: Rezeptur; FS: Feststoff; spTa: spezifischer Trockenauftrag*

| RF | Subst. (Hersteller) | Konz | A-Rez | Rez | Ist | MV | FS | spTa |
|---|---|---|---|---|---|---|---|---|
| | | | $g/m^2$ | g/100g | g | | g | $g/m^2$ |
| **1** | **Verdicker** | **7%** | **19.961** | **14,84** | **103,93** | | | |
| | H2O | | 15,606 | 11,61 | | 806,9 | 0,00 | |
| | Gantrez S97 (ISP Corp. New Jersey) | | 1,397 | 1,04 | | 70,0 | 1,397 | 0,37 |
| | NaOH 16%ig | pH=6,8 | 2,958 | 2,20 | | 123,1 | 0,473 | 0,13 |

(fortgesetzt)

| RF | Subst. (Hersteller) | Konz | A-Rez | Rez | Ist | MV | FS | spTa |
|---|---|---|---|---|---|---|---|---|
| | | | g/$m^2$ | g/100g | g | | g | g/$m^2$ |
| | | | | | | | | |
| | **Suspension** | | **33,598** | **24,99** | **175,10** | | | |
| **5** | Wasser bid. | | 30,60,15 2 | 22,76 | | 182,20 | | 0,00 |
| **6** | Sipernat FK 320DS (Evonik) | | 3,00 | 2,23 | | 17,80 | 2,998 | 0,80 |
| | | | | | | | | |
| | **Netzmittel/ Sonstiges** | | **12,88** | **9,58** | **67,12** | | | |
| **2** | Mega 8 | | 0,300 | 0,22 | 1,56 | | 0,300 | 0,08 |
| **3** | Geropon | | 0,040 | 0,03 | 0,210 | | 0,040 | 0,01 |
| **7** | Propiofan70VAR (BASF) | | 8,044 | 5,98 | 41,90 | | 4,022 | 1,07 |
| **4** | PVP 25000 (Fluka) | | 0,999 | 0,74 | 5,20 | | 0,999 | 0,27 |
| **8** | K-$PO_4$-Puffer pH 6.8 (Merck) | 1M | 3,50 | 2,60 | 18,25 | | 0,483 | 0,13 |
| | | | | | | | | |
| **9** | **Enzym-Lösung** | | **68,03** | **50,59** | **354,50** | | | |
| | K-$PO_4$-Puffer pH 6.8 (Merck) | 0,1M | 31,0 | 23,05 | | 177,50 | 0,43 | 0,12 |
| | cNAD-Na (Roche) | | 5,98 | 4,45 | | 34,2 | 5,98 | 1,60 |
| | GlucDHmut2 #4 (kU/g)/ (kU/$m^2$) (Roche) | | 3,0/ 915,00 | 2,23/ 680,46 | | 17,2 | 3,00 | 0,80/ 244,14 |
| | Wasser bid . | | 28,0 | 20,86 | | 160,6 | | 0,24 |
| | pH vor Einstellen | 6,8 | | | | | | |
| | pH nach Einstellen | 6,77 | | | | | | |
| | | | | | | | | |
| | NaOH 16%ig zum Nachstellen | | | | | | 0,000 | |
| | Summe | | **134,47** | **100,00** | **700,65** | | **41,09** | **18,96** |

Die Substanzen wurden in der aufgeführten Reihenfolge auf die Pokalonfolie eines Testfeldes eines Testträgers aufgebracht. Anschließend wurden die Schichten ca. 15 Min. bei 50°C getrocknet.

*Tabelle 3: Rezeptur für die 2. Testchemieschicht, wobei: RF: Reihenfolge; Subst: Substanz; AS: Aufschlämmung; Konz: Konzentration; A-Rez: Arbeitsrezeptur; Rez: Rezeptur; TL: Teillösungen; FS: Feststoff; spTa: spezifischer Trockenauftrag*

| RF | Subst. | Konz | A-Rez | Rez | ist | TL | FS | spTa |
|---|---|---|---|---|---|---|---|---|
| | | | g/$m^2$ | g/100g | g | | G | g/$m^2$ |
| | **AS** | | **69,503** | **71,66** | **537,37** | | | |
| **2** | K-PO4 pH=6.8 | **1M** | 8,800 | 9,07 | 68,04 | | 1,25 | 0,58 |
| **3** | $ZrO_2$ (FCM) | | 35,551 | 36,65 | 274,88 | | 35,55 | 16,40 |

(fortgesetzt)

| RF | Subst. | Konz | A-Rez | Rez | ist | TL | FS | spTa |
|---|---|---|---|---|---|---|---|---|
| | | | g/m2 | g/100g | g | | G | g/m2 |
| | | | | | | | | |
| **1** | Wasser bid. | | 25,152 | 25,93 | 194,45 | | | |
| | **Verdicker** | **7%** | **23,329** | **24,05** | **180,35** | | | |
| **4** | H2O | | 18,824 | 19,41 | | 806,9 | 0,00 | 0,00 |
| | Gantrez S97 | | 1,633 | 1,68 | | 70,0 | 1,63 | 0,75 |
| | NaOH 16%ig | pH=6,8 | 2,872 | 2,96 | | 123,1 | 0,46 | 0,21 |
| | | | | | | | | |
| | **Netzmittel/ Sonstiges** | | **4,165** | **4,29** | **32,17** | | | |
| | | | | | | | | |
| **5** | Mega 8 | | 0,235 | 0,24 | 1,82 | | 0,24 | 0,11 |
| **6** | Propiofan | | 3,930 | 4,05 | 30,35 | | 1,97 | 0,91 |
| **7** | **NaOH 16%ig** | | | | | | 0,00 | 0,00 |
| | **pH vor Einstellen:** | 6,7 | | | | | | |
| | Summe | | **96,997** | **100,00** | **749,89** | | **41,09** | **18,96** |

Die Substanzen wurden in der aufgeführten Reihenfolge auf die 1. Schicht auf das Testfeld des obigen Testträgers aufgebracht. Das Aufbringen fand über ein Rakelblatt statt, das durch Rakeln über Tisch die genannten Chemikalien gleichmäßig auf den Testträger aufbracht. Der jeweilige Farbstoff wurde als letzter Schritt auf den Testträger aufgebracht. Die Beschichtung fand bei Raumtemperatur und einer rel. Luftfeuchte von ca. 43 % statt. Anschließend wurde die Schicht ca. 20 Min. bei 50 °C getrocknet.

Die verwendeten Pufferlösungen wurden wie folgt hergestellt:

*Tabelle 4: Herstellvorschrift für Pufferlösungen*

| **Gantrezlösung 7% pH=6.9** | **g** | |
|---|---|---|
| Wasser bid | 806,9 | |
| Gantrez S97 533448 | 70,0 | **7,0 %** |
| NaOH 16% | 123,1 | |
| | 1000,00 | |
| **pH (6.8+/- 0.1):** | | **6,92** |
| | | |
| **Kieselsäure-Suspension (Wasser bid.)** | | |
| Kieselsäure Sipernat FK320DS 533235 | 17,8 | **8,9 %** |
| Wasser bid. | 182,2 | |
| | 200 | |
| | | |
| **K2HPO4-Lösung 1M (bis zur Eichmarke auf 500mL auffüllen)** | | 174,18 g/mol |
| K2HPO4 (03593622001 / # 51833200) | 87,10 | **15,4 %** |
| bidest. Wasser | 478,60 | |

(fortgesetzt)

| | | |
|---|---|---|
| **Kieselsäure-Suspension (Wasser bid.)** | | |
| | 565,70 | |
| | | |
| **KH2PO4-Lösung 1M (bis zur Eichmarke auf 500mL auffüllen)** | | 136,09 g/mol |
| KH2PO4 (03593665001 / # 51833100) | 68,05 | **12,5 %** |
| bidest. Wasser | 476,76 | |
| | 544,81 | |
| | | |
| **K-Phosphat - Puffer pH=6.8 1M** | | **13,8 %** |
| K2HPO4-Lösung (15.4%ig) vorlegen | 400,00 | |
| KH2PO4-Lösung (12,5%ig) | 494,80 | |
| Summe des Puffers bei pH 7.0 | 894,80 | |
| **pH (6.8±0.1):** | | **6,8** |
| | | |
| **K-Phosphat-Puffer 0.1M pH=6,8 (bis zur Eichmarke auf 1L auffüllen)** | | |
| K-Phosphat-Puffer 1M pH=6,8 | 100 | |
| Wasser bid. | 900 | **1,40 %** |
| | 1000 | |
| **pH (6,8± 0.1):** | | **6,8** |

Auf die oben ausgeführte Art und Weise wurden Testelemente mit den Farbstoffen aus Tabelle 5 hergestellt und in Versuchen getestet.

*Tabelle 5: Zusammensetzung der Testfelder bei den verschiedenen Experimenten, wie unter Figuren 2-9 beschrieben.*

| | **1.Schicht (66μm) End-pH-Wert beträgt 6,75** | **2.Schicht (58μm)** |
|---|---|---|
| **V1** | Standard | Standard |
| **V2** | Standard + 0,05Gew.% Amaranth (17,9mg/$m^2$) | Standard |
| **V3** | Standard + 0,1Gew.% Amaranth (35,8mg/$m^2$) | Standard |
| **V4** | Standard + 0,05Gew.% Erioglaucin (17,9mg/$m^2$) | Standard |
| **V5** | Standard + 0,1Gew.% Erioglaucin (35,8mg/$m^2$) | Standard |
| **V6** | Standard + 0,05Gew.% Hydroxynaphtylblau (17,9mg/$m^2$) | Standard |
| **V7** | Standard + 0,1Gew.% Hydroxynaphtylblau (35,8mg/$m^2$) | Standard |
| **V8** | Standard + 0,05Gew.% Indigokarmin (17,9mg/$m^2$) | Standard |
| **V9** | Standard + 0,1Gew.% Indigokarmin (35,8mg/$m^2$) | Standard |
| **V10** | Standard + 0,05Gew.% 1,1-Diethyl-4,4-Carbocyanin-Iodid (1260) (17,9mg/$m^2$) | Standard |
| **V11** | Standard + 0,1Gew.% 1,1-Diethyl-4,4-Carbocyanin-Iodid (1260) (35,8mg/$m^2$) | Standard |

Der Farbstoff wurde der 1. Schicht vor dem Auftrag auf das Testfeld als Trockensubstanz zugemischt und mit einem Flügelrührer (ca. 200 U/min) homogenisiert, anschließend gesiebt und zentrifugiert.

Alle Farbstoffe zeigen ein temperaturabhängiges Diffundieren, beispielsweise "Herausfließen", des Farbstoffes

in das darüber liegende Blut, was sich in einer Erhöhung des Remissionssignals widerspiegelt, da sich die Konzentration des absorbierenden Farbstoffes in der Markerschicht 120 erniedrigt. Die jeweilige Größe der Abhängigkeit hängt von der Struktur des einzelnen Farbstoffes ab.

Als Beispiel wird hier die Kinetik des Farbstoffes Erioglaucin gezeigt, der die stärkste Temperaturabhängigkeit zeigt. Die Kinetik ist nicht abhängig von der Glucosekonzentration, wie es zu erwarten wäre.

In Figur 2 sind verschiedene Kurvenverläufe der Diffusionskinetik des Farbstoffes Erioglaucin innerhalb des Testfeldes vor und nach Benetzung des Testfeldes mit Probe (hier Blut) anhand von drei verschiedenen Kurvenscharen 204, 206 und 208 gezeigt, wobei die Intensität des reflektierten Lichtes vermessen wurde. Es wurde Licht der Wellenlänge 625 nm auf das Testfeld gestrahlt. Das Testfeld beinhaltet 0,05 % Erioglaucin als Marker in dem ersten Bereich 114 des in Figur 1b gezeigten Testfeldes 100. Das Licht mit der Wellenlänge 625 nm wird von der Unterseite des Testfeldes 100 direkt auf die Markerschicht 120 geleitet und der Detektor nimmt das reflektierte Licht von dieser Testfeldseite auf. Verschiedene Testfelder wurden einer Temperaturabhängigkeitsmessung unterzogen, indem Blut mit 2 Glucosekonzentrationen auf die Schicht aufgebracht wurde und bei 660 nm vermessen wurde. In dem Kurvenverlauf können drei verschiedene Kurvenscharen unterschieden werden, wobei die erste Kurvenschar 204 die Reflexion des Testfeldes bei 5 °C und zwei verschiedenen Glucosekonzentrationen, nämlich 0 mg/dl 204a und 550 mg/dl 204b in der Probe aufweist. Die zweite Kurvenschar 206 wurde bei 25 °C entweder mit 0 mg/dl Glucosezusatz 206a oder 550 mg/dl Glucosezusatz 206b vermessen, während die dritte Kurvenschar 208 bei 45 °C vermessen wurde und ein Teil der Kurven mit einer Probe ohne Zusatz von Glucose 208a erzeugt wurde, während ein zweiter Teil mit einem Zusatz von 550 mg/dl Glucose 208b ermittelt wurde. Wie man in Figur 2 erkennen kann, ist die Steigung der Kinetik stark abhängig von der Temperatur: bei 5°C ist das Herausfließen des Farbstoffes deutlich langsamer als bei 25°C und die Geschwindigkeit wiederum langsamer als bei 45°C. Über die Konzentration des Farbstoffes in der Markerschicht lässt sich die Höhe und die Schnelligkeit des Endsignals beeinflussen. Die Menge an Analyt, in diesem Fall Glucose hat keinen Einfluss auf die Diffusion des Markers und umgekehrt, wie anhand der Kurvenverläufe zu erkennen ist.

Ähnliche Ergebnisse wurden mit einer Konzentration von 0,1 % Erioglaucin in der Markerschicht 120 im Testfeld 100 erreicht, wie dies in Figur 3 gezeigt ist. Auch hier sind wiederum drei Kurvenscharen 204, 206 und 208 zu unterscheiden, die für die Temperaturen 5 °C für die erste Kurvenschar 204, 25 °C für die zweite Kurvenschar 206 und 45 °C für die dritte Kurvenschar 208 ermittelt wurden.

In beiden Figuren 2 und 3 können die unterschiedlichen Glucosekonzentrationen bei den jeweiligen Temperaturen nicht voneinander unterschieden werden, was an den Untergruppen 204a, 206a und 208a für 0 mg/dl Glucose bzw. 204b, 206b und 208b mit jeweils 550 mg/dl Glucose gezeigt ist. Aus diesen Kurvenverläufen kann abgeleitet werden, dass die Diffusion des Farbstoffes Erioglaucin zwar temperaturabhängig ist, jedoch nicht von der Analytkonzentration abhängig ist. Aus diesem Grund eignet sich dieser Farbstoff, um anhand seiner Diffusionsgeschwindigkeit auf die Temperatur der Probe Rückschlüsse zu nehmen. Dies konnte nicht nur für den Farbstoff Erioglaucin gezeigt werden, sondern ist in Figur 4 ebenfalls für den Farbstoff Hydroxynaphtholblau gezeigt. Auch hier wurden bei verschiedenen Temperaturen, nämlich 5 °C für die Kurvenschar 204 sowie 25 °C für die Kurvenschar 206 und 45 °C für die dritte Kurvenschar 208 bei unterschiedlichen Glucosezusätzen die Abhängigkeit der Diffusionsgeschwindigkeit des Markers gemessen. Auch hier wurde grundsätzlich gefunden, dass die Diffusionsgeschwindigkeit des Markers zwar temperaturabhängig ist, jedoch nicht von der Konzentration des Analyten, in diesem Fall Glucose, abhängig ist.

In der Figur 5 konnte zusätzlich gezeigt werden, dass die Diffusionsgeschwindigkeit des Markers Erioglaucin nicht nur von der Temperatur, sondern auch von dem Hämatokrit der Probe abhängig ist. Hierzu wurden bei verschiedenen Temperaturen, nämlich 5 °C für die Kurvenschar 210, 25 °C für die Kurvenschar 212 und 45 °C für die Kurvenschar 214 Reflexionskurven aufgenommen. Dabei hängt es von dem Hämatokrit der Probe ab, welchen Verlauf die Kurve nimmt. So können Kurven bei 5 °C in ihrer Kinetik unterschieden werden, je nachdem, ob sie Blut als Probe aufweisen wie es für die Kurvenschar 210b gezeigt ist oder ob, wie für die Kurvenschar 210a, die Probe aus Wasser besteht. Auch die Kurvenschar 212 kann in ihrem Verlauf unterschieden werden, einmal für Blutproben 212b bei 25 °C und Kurven 212a für Wasserproben bei 25 °C. Die weitere Kurvenschar 214 bei 45 °C kann nochmals unterschieden werden in die Proben Wasser mit der Kurve 214a sowie für Blut mit den Kurven 214b. Wie deutlich zu erkennen ist, diffundiert der Marker schneller aus dem Testfeld 100 heraus bei erhöhter Temperatur. Weiterhin kann eine unterschiedliche Diffusionsgeschwindigkeit bei den jeweiligen Temperaturen für verschiedenes Hämatokrit unterschieden werden. So zeigt sich, dass bei der gleichen Temperatur von beispielsweise 5 °C eine deutlich höhere Diffusion bei geringerem Hämatokrit zu finden ist. Anhand dieser Kurvenverläufe kann folglich auf den Einfluss von Temperatur und Hämatokrit zusammen geschlossen werden. Ist die Temperatur bekannt, so kann aufgrund der Markerdiffusion auf den Hämatokrit geschlossen werden und umgekehrt.

Um die Beeinflussung der Messungen durch Hämatokrit weiter zu untersuchen, wurde die Testchemieschicht 118 mit dem Marker 104, 104' in Form von Erioglaucin untersucht. Hierbei wurde das Herausfließen des Farbstoffes aus der Schicht untersucht. Die Figur 6 zeigt den Einfluss des Hämatokrits auf die Farbstoffkinetik. In der Figur 6 wird der Kurvenverlauf von drei verschiedenen Proben mit unterschiedlichem Hämatokrit gezeigt, wobei das Testfeld 100 mit 0,05 % Erioglaucin beschichtet wurde. So weisen die Kurven der Kurvenschar 204 einen Hämatokrit von 65 % auf,

während die Kurven der Kurvenschar 206 einen Hämatokrit von 45 % aufweisen und die Kurven der Kurvenschar 208 einen Hämatokrit von 25 %. Die aufgebrachte Probe enthält dabei keine Glucose. Hierbei handelt es sich um Blutproben, die durch Rollen bei Raumtemperatur auf 0 mg/dl Glucose gebracht wurden, wobei durch das Rollen eine Probenbewegung erzeugt wurde und wobei ein praktisch vollständiger Abbau einer vorhandenen Glucose über Nacht durch die in der Probe enthaltenen Zellen erfolgte.

In der Figur 7 sind Kurvenscharen 204, 206 und 208 mit jeweils 65 %, 45 % und 25 % Hämatokrit in Blut aufgenommen worden, wobei sich in den Kurvenscharen Proben mit unterschiedlichen Glucosekonzentrationen befinden. Für jede Kurvenschar 204, 206 und 208 wurden die Konzentrationen 0, 90, 150, 350 und 550 mg/dl Glucose aufgezeichnet. Anhand dieser Kurvenscharen ist ebenfalls zu erkennen, dass die Diffusion des Markers annähernd unabhängig von der Konzentration an Analyt 117 der Probe ist, jedoch eine Abhängigkeit der Kinetik von dem Hämatokrit besteht. Folglich kann aus dem Kurvenverlauf der Hämatokrit der Probe bestimmt werden. Alternativ kann auch zu einem bestimmten Zeitpunkt, wie hier eingezeichnet nach 5 Sekunden, der Reflexionsunterschied zum Zeitpunkt 0, der dem Probeaufgabezeitpunkt entspricht, ermittelt werden und daraus der Hämatokrit der Probe ermittelt werden.

Figur 8 zeigt die Mittelwerte für die jeweiligen Konzentrationen 0, 90, 150, 350 und 550 mg/dl Glucose aus den Kurvenscharen 204, 206 und 208 aus der Figur 7. Dies verdeutlicht die Unabhängigkeit der Kurvenverläufe von der Glucosekonzentration der Probe.

In Figur 9 sind ebenfalls drei Kurvenscharen 204, 206 und 208 zu erkennen, wobei innerhalb der Kurvenschar 204 eine Kurve 216 für ein Testfeld mit 0,05 % Erioglaucin als Marker dotiert ist, während die Kurve 222 von einem Testfeld mit 0,1 % Erioglaucin stammt. Beide Kurven wurden mit einer wässrigen Lösung und einem Hämatokrit von 65 % aufgenommen. Die Kurvenschar 206 wurde mit einem Hämatokrit von 45 % aufgenommen und die Kurvenschar 208 mit einem Hämatokrit von 25 %. Auch bei der Kurvenschar 206 ist eine Kurve 224 mit 0,05 % Erioglaucin von einer Kurve 226 mit 0,1 % Erioglaucin zu unterscheiden, während in der Kurvenschar 208 eine Kurve 218 mit 0,05 % Erioglaucin und einer Kurve 220 mit 0,1 % Erioglaucin unterschieden werden kann. In der Regel werden die Messungen nach 4 bis 5 Sekunden gestoppt, um eine möglichst kurze Messzeit zu realisieren. Aus den Kurvenverläufen 204, 206 und 208, die jeweils zwei verschiedene Markerkonzentrationen beinhalten ist bei der Linie, die bei 5 Sekunden nach Probenaufgabe gezogen wurde zu erkennen, dass eine Unterscheidung zwischen den beiden Konzentrationen von 0,1 und 0,05 % an Erioglaucin kaum zu unterscheiden. Das bedeutet, dass selbst bei einer Abnahme an Farbstoff in einem Testelement über die Lebensdauer keine Einschränkung der Korrekturmöglichkeit zu befürchten ist.

Insgesamt ist zu beobachten, dass alle in den Figuren 2 bis 9 untersuchten Farbstoffe ein temperaturabhängiges Herausfließen des Farbstoffes in den Überstand aus Blut nach Benetzung des Testfeldes zeigen. Dies zeigt sich in einer Erhöhung des Remissionssignals, da sich die Konzentration des absorbierenden Farbstoffes in der Testschicht erniedrigt. Die jeweilige Größe bzw. das jeweilige Ausmaß der Abhängigkeit von der Temperatur hängt von der Struktur des einzelnen Farbstoffes ab.

Figur 10 zeigt schematisch das Verhalten eines Analytsignals einer Probe, in diesem Fall Glucose in Blut, bei verschiedenen Temperaturen und verschiedenen Hämatokritwerten. Das Analytsignal ist in Figur 10 auf der vertikalen Achse aufgetragen und mit c bezeichnet und angegeben in Milligramm pro Deziliter. Unter einem Analytsignal ist hierbei allgemein ein beliebiger Messwert oder eine beliebige Messgröße oder aus einer Messung abgeleitete Größe zu verstehen, welche die Analytkonzentration wiedergibt oder welche mit der Analytkonzentration korreliert oder welche einen Rückschluss auf die Analytkonzentration ermöglicht. Das Analytsignal kann alternativ zu der Angabe in mg/dl auch in anderen Einheiten angegeben werden, beispielsweise in Einheiten eines Detektorsignals in Remissionswerten oder ähnlichen Einheiten. Das Analytsignal ist in Figur 10 jeweils erfasst zu einem Zeitpunkt von 4 Sekunden nach einer Benetzung des Testfeldes mit der Probe, was in Figur 10 mit "@ 4s" symbolisiert ist. Es wird daraufhingewiesen, wie oben bereits ausgeführt, dass es sich bei den in Figur 10 dargestellten Werten des Analytsignals nicht um Messwerte sondern um schematische, fiktive Werte handelt, die beispielhaft einen typischen Verlauf des Analytsignals wiedergeben.

In Figur 10 sind Abhängigkeiten des Analytsignals von zwei verschiedenen Einflüssen unterschiedlicher Eigenschaften der Probe schematisch dargestellt, nämlich der Temperatur T, angegeben in °C, und eines Hämatokritwerts Hct, angegeben in Prozent. Diese Eigenschaften sind auf der horizontalen Achse dargestellt. In Figur 10 sind dabei fiktive Analytwerte, die beispielsweise im Rahmen einer Messreihe mit Variation der Temperatur T erfasst werden könnten, als ausgefüllte Kreise dargestellt und mit den Bezugsziffern 234 bezeichnet. Analytwerte, die im Rahmen einer Messreihe mit Variation des Hämatokrits erfasst werden könnten, sind als nicht-ausgefüllte Kreise dargestellt und mit den Bezugsziffern 236 bezeichnet.

Die in Figur 10 dargestellten Werte könnten beispielsweise mit einem Messaufbau erfasst werden, bei welchem von einer Applikationsseite her die Probe auf ein die Testchemie und optional den Marker enthaltendes Testfeld aufgebracht wird. Von einer der Applikationsseite gegenüberliegenden Detektionsseite des Testfeldes wird das Testfeld mit einer Analytdetektor-Lichtquelle bestrahlt, beispielsweise mittels einer ersten Leuchtdiode, und mittels eines fotoempfindlichen Detektors, beispielsweise einer Fotodiode, wird an dem Testfeld gestreutes Licht erfasst, um einen Remissionswert zu erhalten. Dieser Remissionswert der Analyt-Messung kann dann mittels einer bekannten, festen Vorschrift in die Analytkonzentration c (unkorrigiert), angegeben in Milligramm pro Deziliter oder in anderen Konzentrationseinheiten, umgerechnet werden. Die Analytdetektor-Lichtquelle kann beispielsweise in ihren spektralen Eigenschaften derart angepasst sein, dass diese eine Wellenlänge aufweist, die von einem Farbstoff der Testchemie absorbiert wird. Beispielsweise kann die Analytdetektor-Lichtquelle bei einer Wellenlänge von 360 nm emittieren.

Der Verlauf der Punkte für eine Variation der Temperatur 234 zeigt eine Erniedrigung der Messwerte für den Analyten Glucose bei sinkender Temperatur. Dies bedeutet, dass der Benutzer eines herkömmlichen Testelementes ohne Temperaturkorrektur einen höheren Analytmesswert angezeigt bekommt, wenn die Temperatur höher als bei der Eichung ist und einen niedrigeren Wert, wenn die Temperatur bei der Messung niedriger ist als die bei der Eichung des Systems. Dieses Verhalten ist mit hoher Wahrscheinlichkeit dadurch bedingt, dass durch eine Erniedrigung der Temperatur allgemein insbesondere ein Diffusionsverhalten gehemmt wird, so dass beispielsweise der nachzuweisende Analyt langsamer zur Testchemie vordringt als bei einer höheren Temperatur. Weiterhin wird möglicherweise auch die eigentliche Nachweisreaktion zum Nachweis des Analyten verlangsamt.

Ein umgekehrtes Verhalten zeigt das Messsignal in Bezug auf Hämatokrit in der Probe. In Figur 10 ist anhand der Messpunkte für Hämatokrit 236 zu erkennen, dass das Messsignal für Glucose abnimmt, wenn der Hämatokrit zunimmt. Das Messsignal für Glucose nimmt in dem Temperaturbereich von 5 °C bis 45 °C in dem gleichen Maße ab, wie bei einem Anstieg des Hämatokrit von 25 % auf 65 %. Die Ursache dieses Verhaltens ist mit hoher Wahrscheinlichkeit ebenfalls in einer Hemmung von Diffusionsprozessen durch den erhöhten Hämatokrit zu suchen.

Die beiden genannten Effekte sind in der Regel nicht direkt zu trennen, was für eine Korrektur jedoch unerheblich ist. So kann beispielsweise allgemein eine Korrektur auf eine Kombination eines bestimmten Hämatokrits mit einer bestimmten Temperatur erfolgen, ohne dass Hämatokrit und Temperatur bekannt sind. Alternativ könnte jedoch auch beispielsweise eine dieser Störgrößen, wie Temperatur und Hämatokrit, unabhängig bestimmt werden, also unabhängig von der Markerdiffusion. Beispielsweise könnte bei dem erfindungsgemäßen Verfahren und/oder in der erfindungsgemäßen Vorrichtung mindestens ein Temperaturfühler verwendet werden, mittels dessen beispielsweise eine Umgebungstemperatur und/oder eine Temperatur des Testelements und/oder eine Temperatur der Probe (zwischen diesen Temperaturen wird allgemein im Rahmen der vorliegenden Erfindung in der Regel nicht unterschieden) bestimmt werden kann. Bei Kenntnis der Temperatur kann dann beispielsweise eine Korrektur mittels der ermittelten Markerdiffusion auf einen bestimmten Hämatokrit erfolgen.

Die Korrektur der Analytkonzentration kann beispielsweise derart durchgeführt werden, wie unten noch näher ausgeführt wird, dass als korrigierte Werte der Analytkonzentration eine Analytkonzentration bei beispielsweise 21 °C oder 25 °C (Raumtemperatur) und einem Hämatokrit von 40 % ermittelt wird, so dass beispielsweise korrigierte Analytkonzentrationen, welche unter verschiedenen Bedingungen (Temperatur, Hämatokrit) erfasst wurden, mit einander verglichen werden können.

In Figur 11 ist hingegen der Einfluss der Temperatur und/oder des Hämatokrits auf ein Markersignal gezeigt. Wiederum sind fiktive Werte aufgetragen, welche symbolisch einen typischen Verlauf eines Markersignals zeigen. Die Werte des Markersignals könnten mit einem Messaufbau analog zu dem oben zu Figur 10 beschriebenen Messaufbau erfasst werden oder mit demselben Messaufbau. Beispielsweise könnte wiederum von einer Applikationsseite her die Probe auf ein den Marker und optional auch die Testchemie enthaltendes Feld, beispielsweise ein Markerfeld und/oder ein Testfeld, aufgebracht werden. Von einer der Applikationsseite gegenüberliegenden Detektionsseite des Testfeldes kann das Testfeld mit einer Markerdetektor-Lichtquelle bestrahlt werden, beispielsweise mittels einer zweiten Leuchtdiode, und mittels eines fotoempfindlichen Detektors, beispielsweise einer Fotodiode. Es wird an dem Markerfeld und/oder Testfeld gestreutes Licht erfasst, um einen Remissionswert R zu erhalten. Die Markerdetektor-Lichtquelle kann beispielsweise in ihren spektralen Eigenschaften derart angepasst sein, dass diese eine Wellenlänge aufweist, die von einem Marker absorbiert wird. Beispielsweise kann die Markerdetektor-Lichtquelle bei einer Wellenlänge von 660 nm emittieren.

In Figur 11 ist das Remissionsverhalten R (angegeben auf der vertikalen Achse ohne Einheiten) eines Markers, beispielsweise Erioglaucin, gezeigt, welches das gleiche Verhalten bei Temperaturveränderung wie bei Hämatokritänderung in einem bestimmten Bereich dieser Parameter aufweist. Dabei kann die Remission R beispielsweise an einem Markerfeld als Funktion der Zeit nach Benetzung des Markerfeldes bzw. Testfeldes mit der Probe erfasst. Nach 4 Sekunden kann die Messung gestoppt und der Remissionswert R bei dieser Zeit verzeichnet werden.

Wie die Figur 11 zeigt, steigt beispielsweise die Remission des Markers bei Erniedrigung der Temperatur von 45 °C auf 5 °C genauso stark an, wie die Erhöhung des Hämatokrit von 25 % auf 65 %. Wiederum sind somit zwei Effekte zu verzeichnen, die auf die beiden Störgrößen, Temperatur und Hämatokrit, zurückzuführen sind. Bei steigender Temperatur und sinkendem Hämatokrit wird die Diffusion des Markers in die Probe hinein begünstigt, so dass die von der Detektionsseite her betrachtete Färbung des Markersfeldes und/oder des den Marker enthaltenden Testfeldes abnimmt, wodurch die Remission zunimmt. Umgekehrt wird bei sinkender Temperatur und steigendem Hämatokrit die Diffusion des Markers in die Probe hinein gehemmt, so dass die Färbung des Markerfeldes und/oder des den Marker enthaltenden Testfeldes erhalten bleibt und die Remission somit niedrig bleibt. Beispielsweise kann die Remission wiederum zu einem Zeitpunkt von 4 s nach Aufbringen der Probe oder zu einem anderen vorgegebenen Zeitpunkt nach Aufbringen der Probe erfasst werden. Wiederum sind die beiden Effekte typischerweise nur schwer zu trennen. Da die

Remission bei der Marker-Messung jedoch von beiden Störgrößen abhängig ist, kann die Remission bei der Markermessung, welche zumindest unabhängig ist von der Analytkonzentration, eine Gesamtkorrektur auf beide Störgrößen gemeinsam ermöglichen.

In den Figuren 12 bis 14 ist die Möglichkeit einer Korrektur einer Glucosemessung durch eine Remissionsmessung eines Markerfeldes exemplarisch erläutert. Für diese Messungen wurden 10 bis 50 Proben mit unterschiedlichen Glucosekonzentrationen zwischen 30 mg/dl und 550 mg/dl optisch vermessen, wobei Remissionsmessungen durchgeführt wurden und anschließend auf herkömmliche Weise, d.h. in dem Fachmann bekannter Weise ohne Berücksichtigung des Hämatokrits und der Temperatur, die Remissionswerte in entsprechende Glucosekonzentrationen umgewandelt wurden. Dabei wurden jeweils 10-15 Messungen an Proben mit einem vorgegebenen Hämatokritwert durchgeführt, wobei Messungen an Proben mit drei verschiedenen Hämatokritwerten durchgeführt wurden, nämlich jeweils 10-15 Messungen an Proben mit 25% (in den Figuren 12 bis 14 mit den Bezugsziffern 228 bezeichnet), 10-15 Messungen an Proben mit 45% (in den Figuren 12 bis 14 mit den Bezugsziffern 230 bezeichnet) und 10-15 Messungen an Proben mit 65 % (in den Figuren 12 bis 14 mit den Bezugsziffern 232 bezeichnet).

Aufgetragen auf der vertikalen Achse in den Figuren 12 bis 14 ist jeweils der so genannte "Systemfehler", welcher hier mit "E" bezeichnet ist. Der Systemfehler ist in Glucosemessgeräten eine dem Fachmann bekannte Größe. Der Systemfehler wird bei Glucosekonzentrationen bis zu 100 mg/dl in absoluten Einheiten angegeben, und bei Glucosekonzentrationen oberhalb von 100 mg/dl in Prozent. Der Systemfehler berechnet sich jeweils aus dem gemessenen Wert und dem tatsächlichen Wert der Glucosekonzentration, welcher, wie oben ausgeführt, durch die Diffusion der Glucose in der Probe beeinflusst wird. Der tatsächliche Wert der Glucosekonzentration kann beispielsweise mittels einer bekannten Labormethode und/oder durch eine bekannte Einwaage an Glucose in eine Probe bei einer Herstellung der Probe bestimmt werden. Zur Berechnung des Systemfehlers E wird die Abweichung des Messwertes mittels optischer Remissionsmessung an einem Testfeld vom tatsächlichen Wert bestimmt. Bei gemessenen Konzentrationen von bis zu 100 mg/dl wird die absolute Abweichung als Systemfehler angegeben. Bei gemessenen Konzentrationen oberhalb von 100 mg/dl wird die Abweichung zur tatsächlichen Konzentration ins Verhältnis gesetzt.

In Figur 12 ist auf der horizontalen Achse die Remission R eines Markerfeldes angegeben, welche durch die Diffusion des Markers in der Probe beeinflusst wird. Als Marker wurde hier wieder Erioglaucin verwendet, dessen Remission 4 s nach Aufbringen der Probe auf das Markerfeld bzw. Testfeld erfasst wurde, analog zu den oben beschriebenen Messungen in Figur 11. Die Remission ist hier angegeben in Prozent, bezogen auf eine absolute Remission. Auch andere Darstellungen von Ergebnissen einer Markermessung könnten alternativ auf dieser horizontalen Achse aufgetragen werden.

Die Ergebnisse in Figur 12 zeigen, dass die Punktescharen eine Korrelation zwischen dem Systemfehler E der Glucosemessung und dem Remissionsverhalten des Markers aufweisen, welche zur Korrektur genutzt werden kann. Die Korrelation kann beispielsweise durch eine Gerade beschrieben werden, insbesondere eine Eichgerade, mit der Geradengleichung $E = m \cdot R + b$, wobei die Parameter m (Steigung) und b (Achsenabschnitt) beispielsweise aus einer Anpassung an experimentelle Werte bestimmt werden können, nach gängigen Verfahren. Dies kann beispielsweise mittels eines so genannten Fits erfolgen. Mittels der auf diese Weise bestimmten Parameter können nachfolgend Analytmessungen korrigiert werden, beispielsweise indem bei einer Analytmessung zunächst unkorrigierte Werte einer Remission und/oder einer Analytkonzentration bestimmt werden, welche anschließend korrigiert werden, beispielsweise mittels eines Korrekturfaktors entsprechend der bekannten Parameter der Korrelation, so dass beispielsweise eine Korrektur der Analytmessung auf eine korrigierte Remission und/oder eine korrigierte Analytkonzentration bei 25 °C und 40 % Hämatokrit erfolgen kann.

Die Korrelation in Figur 12 zeigt also, dass die Beeinflussung der Glucosemessung durch den Hämatokrit mit der Beeinflussung der Markerremission durch den Hämatokrit korreliert. Exemplarisch wurde zur Korrektur dieser Beeinflussung eine lineare Korrelation angenommen, wobei grundsätzlich auch komplexere Korrelationen möglich sind. Um diese Korrelation zu quantifizieren, wurde eine Fitgerade 238 an die Messwerte in Figur 12 angepasst. Die Fitparameter dieser Fitgeraden 238 können nun genutzt werden, um nach einer Erfassung der Remission des Markerfeldes entsprechende Messwerte der Glucosekonzentration zu korrigieren. Dies ist in den Figuren 13 und 14 gezeigt.

In Figur 13 sind Systemfehler E für verschiedene Hämatokritwerte in Blut ohne Benutzung der oben genannten Korrekturinformation als Funktion des Hämatokrits gezeigt. Es ist deutlich zu erkennen, dass aufgrund der unkorrigierten Messpunkte die Systemfehler von ca. -22 % bei einem Hämatokrit von 65 % bis hin zu knapp 20 % bei einem Hämatokrit von 25 % streuen.

Werden diese Messwerte hingegen mit Hilfe der Korrekturinformation aus der Fitgeraden 238 korrigiert, so kann die Streuung der Systemfehler drastisch reduziert werden. Dies ist in Figur 14 gezeigt. In Figur 14 wurden dieselben Messwerte zugrundegelegt, welche auch der Messung in Figur 13 zu Grunde lagen. Die Messwerte wurden jedoch zunächst mit der Korrekturinformation aus der Fitgeraden 238 korrigiert und anschließend der Systemfehler E ermittelt.

Der Systemfehler kann beispielsweise mit Hilfe einer Geradengleichung beschrieben werden:

$$E = m * R + b \qquad (1),$$

wobei E der Systemfehler, m die Steigung der Geraden, R der Remissionswert und b der y-Achsenabschnitt der Geraden ist.

Mit Hilfe des auf diese Weise ermittelten Fehlers kann ein später ermittelter unkorrigierter Glucosemesswert in einen korrigierten Glucosemesswert umgerechnet werden. Es ergibt sich:

$$gc_{korr} = gc_{unkorr} - E = gc_{unkorr} - m * R - b \qquad (2)$$

wobei $gc_{korr}$ = korrigierter Glucosemesswert; $gc_{unkorr}$ = unkorrigierter Glucosemesswert; E = Systemfehler, m = Steigung der Geraden, R = Remissionswert; b = y-Achsenabschnitt ist.

Die korrigierten Ergebnisse in Figur 14 zeigen, dass mittels der Markermessung und der aufgrund beispielsweise der Messung in Figur 12 bekannten Korrelation eine effiziente Bereinigung des Einflusses der Störgrößen Temperatur und Hämatokrit erfolgen kann. So liegen nach der Korrektur in Figur 14 alle Punktescharen 228, 230 und 232, mit jeweils 25 %, 45 % und 65 % Hämatokrit annähernd in einem Band von ca. +/- 10 % bzw. +/- 10 mg/dl Systemfehler. Das bedeutet eine Halbierung der Streuung des Systemfehlers gegenüber den nicht korrigierten Werten aus Figur 13, was die Sicherheit der Messung für den Benutzer deutlich erhöht.

Beispiel einer Gewinnung einer Korrekturinformation zur Korrektur einer Analytmessung und Beispiel einer Korrektur:

Im Folgenden wird ein Beispiel einer Korrektur einer Glukosemessung anhand einer aus einer Markerdiffusion erzeugten Korrekturinformation exemplarisch beschrieben. Allgemein gilt dabei, dass die Remission R sowohl bei einer Messung des Markers als auch bei einer Messung eines Analytsignals eine Funktion F der Temperatur T und des Hämatokrits Hct ist, wobei in beiden Fällen dieselbe Abhängigkeit auftritt:

$$R[\%] = F(T, Hct) \qquad (3)$$

Da die Einflüsse der einzelnen Störgrößen der Temperatur und des Hämatokrits in der Regel eine gemeinsame Störgröße T x Hct bilden, da in vielen Fällen diese Einflüsse der Temperatur und des Hämatokrits nicht getrennt sondern gemeinsam korrigiert werden sollen und da die Einflüsse der Temperatur und des Hämatokrits auf die gemessene Remission R in der Regel gegenläufig sind (siehe beispielsweise Figur 11), kann angesetzt werden:

$$R[\%] = F(T \times Hct) \qquad (4)$$

Die Funktion F stellt eine Eichkurve dar, welche sich aus einer Markermessung analog zu den oben anhand der Figuren 11 bis 13 beschriebenen Messungen einer Markerdiffusion ermitteln lässt. Beispielsweise kann eine Messreihe analog zu den Messungen in Figur 11 aufgenommen werden und daraus diese Eichkurve bestimmt werden. Wie sich beispielsweise aus der Darstellung in Figur 11 ergibt, lässt sich die Eichkurve F in vielen Fällen als Gerade beschreiben, mit der Gleichung:

$$F(R_{Marker}) = a + m \cdot R_{Marker} \qquad (5)$$

Dabei stellt a einen Achsenabschnitt oder Offset dar, wohingegen m eine Steigung der Eichkurve beschreibt. $R_{Marker}$ stellt die gemessene Remission des Markers bei einer bestimmten Konzentration des Analyten, einer bestimmtem Temperatur und eines bestimmtem Hämatokrit dar. Aus den Messpunkten der Messreihe beispielsweise in Figur 11 können durch einen geeigneten Fit die Parameter a und m bestimmt werden.

Mit dieser bekannten Eichkurve lässt sich dann bei einer Analytmessung aus einer einzigen Messung oder mehreren Messungen einer Markerremission $R_{Marker}$, das heißt einer durch den Marker und dessen Diffusion in der Probe bedingten bzw. beeinflussten Remission, eine Korrekturinformation erzeugen. Mittels dieser Korrekturinformation lässt sich dann eine gemessene Analytremission $R_{Analyt}$, d.h. eine durch die Nachweisreaktion bedingte bzw. beeinflusste Remission, korrigieren und in eine korrigierte oder tatsächliche Analytremission $R_{Analyt}^*$ umwandeln:

$$R_{Analyt}^{*} = R_{Analyt} - F(R_{Marker}) \quad (6)$$

$$R_{Analyt}^{*} = R_{Analyt} - (a + m \cdot R_{Marker}) \quad (7)$$

Diese korrigierte Analytremission lässt sich dann, beispielsweise mittels einer weiteren aus Vergleichsmessungen gewonnenen Eichkurve, in eine Analytkonzentration umwandeln, insbesondere umrechnen, beispielsweise in eine Glucosekonzentration.

Bezugszeichenliste

| | |
|---|---|
| 50 | Testelement |
| 60 | Testträger |
| 100 | Testfeld |
| 101 | Barriere |
| 102 | Testchemie |
| 104 | Marker |
| 104' | Marker |
| 106 | Abtrennschicht |
| 107 | Probenaufgabeseite |
| 108 | Benetzung |
| 110 | Probe/Blut |
| 112 | Überstand |
| 114 | erster Bereich |
| 116 | zweiter Bereich |
| 117 | Analyt |
| 118 | Testchemieschicht/Reaktivschicht |
| 120 | Markerschicht |
| 122 | Pigmentschicht |
| 130 | erste Lichtquelle |
| 140 | zweite Lichtquelle |
| 150a | Licht der ersten Lichtquelle |
| 150b | Licht der zweiten Lichtquelle |
| 160a | reflektiertes Licht von erster Lichtquelle |
| 160b | reflektiertes Licht von zweiter Lichtquelle |
| 170 | erster Detektor |
| 175 | erste Auswerteeinheit |
| 180 | zweiter Detektor |
| 185 | zweite Auswerteeinheit |
| 188 | Gehäuse |
| 189 | Öffnung |
| 190 | Vorrichtung |
| 200 | Intensitätsskala |
| 202 | Zeitskala |
| 204 | 1. Kurvenschar |
| 204a | 0 mg/dl Gluc |
| 204b | 550 mg/dl Gluc |
| 206 | 2. Kurvenschar |
| 206a | 0 mg/dl Gluc |
| 206b | 550 mg/dl Gluc |
| 208 | 3. Kurvenschar |
| 208a | 0 mg/dl Gluc |
| 208b | 550 mg/dl Gluc |
| 210 | 4. Kurvenschar |
| 210a | $H_2O$ |

| | |
|---|---|
| 210b | Blut |
| 212 | 5. Kurvenschar |
| 212a | $H_2O$ |
| 212b | Blut |
| 214 | 6. Kurvenschar |
| 214a | $H_2O$ |
| 214b | Blut |
| 216 | 0,05 % Erioglaucin |
| 218 | 0,1 % Erioglaucin |
| 220 | 0,05 % Erioglaucin |
| 222 | 0,1 % Erioglaucin |
| 224 | 0,05 % Erioglaucin |
| 226 | 0,1 % Erioglaucin |
| 228 | Punkteschar mit 25 % Hämatokrit |
| 230 | Punkteschar mit 45 % Hämatokrit |
| 232 | Punkteschar mit 65 % Hämatokrit |
| 234 | Messpunkt für Temperatur |
| 236 | Messpunkt für Hämatokrit |
| 238 | Fitgerade |

**Patentansprüche**

1. Verfahren zur Bestimmung mindestens einer Konzentration mindestens eines Analyten (117) in einer Probe (110), insbesondere mindestens eines Metaboliten in einer Probe (110) einer Körperflüssigkeit, wobei mindestens ein Testelement (50) mit mindestens einer Testchemie (102) verwendet wird, wobei mindestens eine Reaktion der Testchemie (102) mit dem Analyten (117) detektiert wird, wobei weiterhin mindestens ein Marker (104, 104') verwendet wird, wobei der Marker (104, 104') von dem Analyten (117) verschieden ist, wobei der Marker (104, 104') mindestens eine chemische Verbindung aufweist, wobei mindestens eine Diffusion des Markers (104, 104') in der Probe (110) oder mindestens einem Teil der Probe (110) detektiert wird, wobei die Detektion der Reaktion des Analyten (117) elektrochemisch und/oder optisch erfolgt, und/oder dass die Detektion der Diffusion des Markers (104, 104') elektrochemisch und/oder optisch erfolgt, wobei aus der Diffusion mindestens eine Korrekturinformation erzeugt wird, wobei aus der Reaktion der Testchemie (102) mit dem Analyten (117) unter Berücksichtigung der Korrekturinformation die Konzentration des Analyten (117) bestimmt wird, wobei die Korrekturinformation ausgestaltet ist, um einen Einfluss der Temperatur der Probe (110) und/oder des Testelements (50) bei der Bestimmung der Konzentration des Analyten (117) zu berücksichtigen, wobei die Korrekturinformation mindestens eine Information über mindestens eine Störgröße der Probe (110) umfasst, wobei die Störgröße eine Temperatur der Probe (110) und/oder des Testelements (50) umfasst, wobei die Korrekturinformation unter Verwendung folgender Verfahrensschritte bestimmt wird:

   - in mindestens einer Eichmessung wird ein allgemeiner Zusammenhang zwischen der Störgröße und der Diffusion des Markers (104, 104') erfasst;
   - in der Probe (110), in welcher die Konzentration des Analyten (117) bestimmt werden soll, wird sowohl die Reaktion der Testchemie (102) mit dem Analyten (117) als auch die Diffusion des Markers (104, 104') detektiert;
   - die Korrekturinformation wird aus der detektierten Diffusion des Markers (104, 104') in der Probe (110) und dem allgemeinen Zusammenhang zwischen der Störgröße und der Diffusion des Markers (104, 104') bestimmt.

2. Verfahren nach dem vorhergehenden Anspruch, **dadurch gekennzeichnet, dass** das Testelement (50) mindestens ein Testfeld (100) mit mindestens einer Testchemieschicht (118) umfasst, wobei die Testchemieschicht (118) die Testchemie (102) umfasst, wobei die Testchemie (102) insbesondere mindestens ein Enzym beinhaltet, insbesondere ausgewählt aus der Gruppe, bestehend aus: Glucosedehydrogenase, Glucoseoxidase.

3. Verfahren nach einem der vorhergehenden Ansprüche, **dadurch gekennzeichnet, dass** der Marker (104, 104') in mindestens einem ersten Bereich (114) des Testelements (50) enthalten ist, wobei die Testchemie (102) ebenfalls ganz oder teilweise in dem ersten Bereich (114) enthalten ist.

4. Verfahren nach einem der vorhergehenden Ansprüche, **dadurch gekennzeichnet, dass** der Marker (104, 104') ganz oder teilweise separat von der Testchemie (102) angeordnet ist.

5. Verfahren nach einem der vorhergehenden Ansprüche, **dadurch gekennzeichnet, dass** der Marker (104, 104') nicht mit dem Analyten (117) und/oder mit der Testchemie (102) reagiert, wobei eine Diffusionsgeschwindigkeit des Markers (104, 104') im Wesentlichen unabhängig von der Konzentration des Analyten (117) ist.

6. Verfahren nach einem der vorhergehenden Ansprüche, **dadurch gekennzeichnet, dass** der Marker (104, 104') mindestens einen optisch nachweisbaren Farbstoff umfasst, wobei der Marker mindestens einen Farbstoff, der hydrophil oder wasserlöslich ist, umfasst.

7. Verfahren nach einem der vorhergehenden Ansprüche, **dadurch gekennzeichnet, dass** der Marker (104, 104') in einem Wellenlängenbereich von 300 bis 800 nm Licht absorbiert.

8. Verfahren nach einem der vorhergehenden Ansprüche, **dadurch gekennzeichnet, dass** der Marker (104, 104') ein Farbstoff ist, wobei der Farbstoff ausgewählt ist aus der Gruppe bestehend aus: Cyaninfarbstoffen, Azofarbstoffen und Sulfonfarbstoffen oder mindestens zwei davon.

9. Verfahren nach einem der vorhergehenden Ansprüche, **dadurch gekennzeichnet, dass** der Marker (104, 104') ausgewählt ist aus der Gruppe bestehend aus: Erioglaucin, Indigocarmin, Hydroxynaphtholblau, 1,1-Diethyl-4,4-Carbocyanin-Iodid und Amaranth, vorzugsweise Erioglaucin oder Hydroxynaphtholblau, oder mindestens zwei davon.

10. Verfahren nach einem der vorhergehenden Ansprüche, **dadurch gekennzeichnet, dass** mindestens ein erster Marker (104, 104') und mindestens ein von dem ersten Marker (104, 104') verschiedener weiterer Marker (104, 104') eingesetzt werden, wobei die Diffusionsgeschwindigkeit des ersten Markers (104, 104') durch mindestens eine erste Eigenschaft der Probe (110) und die Diffusionsgeschwindigkeit des mindestens einen weiteren Markers (104, 104') durch mindestens eine von der ersten Eigenschaft verschiedene weitere Eigenschaft der Probe (110) beeinflusst werden, wobei die Eigenschaft der Probe (110) eine Temperatur der Probe (110) umfasst.

11. Verfahren nach einem der vorhergehenden Ansprüche, **dadurch gekennzeichnet, dass** die Reaktion der Testchemie (102) mit dem Analyten (117) mittels mindestens eines ersten Detektors (170) detektiert wird, wobei die Diffusion des Markers (104, 104') mittels mindestens eines weiteren Detektors (180) detektiert wird.

12. Vorrichtung (190) zur Bestimmung mindestens einer Konzentration mindestens eines Analyten (117) in einer Probe (110), insbesondere unter Verwendung eines Verfahrens nach einem der vorhergehenden Verfahrensansprüche, wobei die Vorrichtung (190) mindestens ein Testelement (50) mit mindestens einer Testchemie (102) umfasst, wobei die Vorrichtung (190) eingerichtet ist, um mindestens eine Reaktion der Testchemie (102) mit dem Analyten (117) zu detektieren, wobei die Vorrichtung (190) mindestens einen Marker (104, 104') umfasst, wobei der Marker (104, 104') von dem Analyten (117) verschieden ist, wobei der Marker (104, 104') mindestens eine chemische Verbindung aufweist, wobei die Vorrichtung (190) eingerichtet ist, um mindestens eine Diffusion des Markers (104, 104') in der Probe (110) oder mindestens einem Bestandteil der Probe (110) zu detektieren, wobei die Detektion der Reaktion des Analyten (117) elektrochemisch und/oder optisch erfolgt, und/oder dass die Detektion der Diffusion des Markers (104, 104') elektrochemisch und/oder optisch erfolgt, wobei die Vorrichtung (190) eingerichtet ist, um aus der Diffusion des Markers (104, 104') mindestens eine Korrekturinformation zu erzeugen, wobei die Vorrichtung (190) weiterhin eingerichtet ist, um aus der Reaktion der Testchemie (102) mit dem Analyten (117) unter Berücksichtigung der Korrekturinformation die Konzentration des Analyten (117) zu bestimmen, wobei die Korrekturinformation ausgestaltet ist, um einen Einfluss der Temperatur der Probe (110) und/oder des Testelements (50) bei der Bestimmung der Konzentration des Analyten (117) zu berücksichtigen, wobei die Korrekturinformation mindestens eine Information über mindestens eine Störgröße der Probe (110) umfasst, wobei die Störgröße eine Temperatur der Probe (110) und/oder des Testelements (50) umfasst, wobei die Vorrichtung (190) eingerichtet ist, um die Korrekturinformation unter Verwendung folgender Verfahrensschritte zu bestimmen:

- in mindestens einer Eichmessung wird ein allgemeiner Zusammenhang zwischen der Störgröße und der Diffusion des Markers (104, 104') erfasst;
- in der Probe (110), in welcher die Konzentration des Analyten (117) bestimmt werden soll, wird sowohl die Reaktion der Testchemie (102) mit dem Analyten (117) als auch die Diffusion des Markers (104, 104') detektiert;
- die Korrekturinformation wird aus der detektierten Diffusion des Markers (104, 104') in der Probe (110) und dem allgemeinen Zusammenhang zwischen der Störgröße und der Diffusion des Markers (104, 104') bestimmt.

## Claims

1. Method for determining at least one concentration of at least one analyte (117) in a sample (110), more particularly at least one metabolite in a sample (110) of a body fluid, wherein at least one test element (50) having at least one test chemistry (102) is used, wherein at least one reaction of the test chemistry (102) with the analyte (117) is detected, wherein additionally at least one label (104, 104') is used, wherein the label (104, 104') is different from the analyte (117), wherein the label (104, 104') has at least one chemical compound, wherein at least one diffusion of the label (104, 104') in the sample (110) or at least part of the sample (110) is detected, wherein the detection of the reaction of the analyte (117) is achieved electrochemically and/or optically, and/or in that the detection of the diffusion of the label (104, 104') is achieved electrochemically and/or optically, wherein at least one piece of correction information is generated from the diffusion, wherein the concentration of the analyte (117) is determined from the reaction of the test chemistry (102) with the analyte (117) while taking into account the piece of correction information, wherein the piece of correction information is designed to take into account an influence of the temperature of the sample (110) and/or of the test element (50) during the determination of the concentration of the analyte (117), wherein the piece of correction information comprises at least one piece of information about at least one interfering variable of the sample (110), wherein the interfering variable comprises a temperature of the sample (110) and/or of the test element (50), wherein the piece of correction information is determined by using the following method steps:

   - in at least one calibration measurement, a general relationship between the interfering variable and the diffusion of the label (104, 104') is acquired;
   - in the sample (110) in which the concentration of the analyte (117) is to be determined, both the reaction of the test chemistry (102) with the analyte (117) and the diffusion of the label (104, 104') is detected;
   - the piece of correction information is determined from the detected diffusion of the label (104, 104') in the sample (110) and from the general relationship between the interfering variable and the diffusion of the label (104, 104').

2. Method according to the preceding claim, **characterized in that** the test element (50) comprises at least one test field (100) having at least one test chemistry layer (118), wherein the test chemistry layer (118) comprises the test chemistry (102), wherein the test chemistry (102) includes in particular at least one enzyme, selected in particular from the group consisting of: glucose dehydrogenase, glucose oxidase.

3. Method according to any of the preceding claims, **characterized in that** the label (104, 104') is contained in at least one first region (114) of the test element (50), wherein the test chemistry (102) is likewise completely or partly contained in the first region (114).

4. Method according to any of the preceding claims, **characterized in that** the label (104, 104') is arranged completely or partly separately from the test chemistry (102).

5. Method according to any of the preceding claims, **characterized in that** the label (104, 104') does not react with the analyte (117) and/or with the test chemistry (102), wherein a diffusion rate of the label (104, 104') is substantially independent of the concentration of the analyte (117).

6. Method according to any of the preceding claims, **characterized in that** the label (104, 104') comprises at least one optically detectable dye, wherein the label comprises at least one dye which is hydrophilic or water-soluble.

7. Method according to any of the preceding claims, **characterized in that** the label (104, 104') absorbs light within a wavelength range of 300 to 800 nm.

8. Method according to any of the preceding claims, **characterized in that** the label (104, 104') is a dye, wherein the dye is selected from the group consisting of: cyanine dyes, azo dyes and sulfone dyes or at least two thereof.

9. Method according to any of the preceding claims, **characterized in that** the label (104, 104') is selected from the group consisting of: erioglaucine, indigo carmine, hydroxynaphthol blue, 1,1-diethyl-4,4-carbocyanine iodide and amaranth, preferably erioglaucine or hydroxynaphthol blue, or at least two thereof.

10. Method according to any of the preceding claims, **characterized in that** at least one first label (104, 104') and at least one further label (104, 104') different from the first label (104, 104') are used, wherein the diffusion rate of the first label (104, 104') and the diffusion rate of the at least one further label (104, 104') are influenced by at least one

first property of the sample (110) and by at least one further property of the sample (110) different from the first property, respectively, wherein the property of the sample (110) comprises a temperature of the sample (110).

11. Method according to any of the preceding claims, **characterized in that** the reaction of the test chemistry (102) with the analyte (117) is detected by means of at least one first detector (170), wherein the diffusion of the label (104, 104') is detected by means of at least one further detector (180) .

12. Device (190) for determining at least one concentration of at least one analyte (117) in a sample (110), using in particular a method according to any of the preceding method claims, wherein the device (190) comprises at least one test element (50) having at least one test chemistry (102), wherein the device (190) is configured to detect at least one reaction of the test chemistry (102) with the analyte (117), wherein the device (190) comprises at least one label (104, 104'), wherein the label (104, 104') is different from the analyte (117), wherein the label (104, 104') has at least one chemical compound, wherein the device (190) is configured to detect at least one diffusion of the label (104, 104') in the sample (110) or at least one constituent of the sample (110), wherein the detection of the reaction of the analyte (117) is achieved electrochemically and/or optically, and/or in that the detection of the diffusion of the label (104, 104') is achieved electrochemically and/or optically, wherein the device (190) is configured to generate at least one piece of correction information from the diffusion of the label (104, 104'), wherein the device (190) is additionally configured to determine the concentration of the analyte (117) from the reaction of the test chemistry (102) with the analyte (117) while taking into account the piece of correction information wherein the piece of correction information is designed to take into account an influence of the temperature of the sample (110) and/or of the test element (50) during the determination of the concentration of the analyte (117), wherein the piece of correction information comprises at least one piece of information about at least one interfering variable of the sample (110), wherein the interfering variable comprises a temperature of the sample (110) and/or of the test element (50), wherein the device (190) is configured to determine the piece of correction information using the following method steps:

- in at least one calibration measurement, a general relationship between the interfering variable and the diffusion of the label (104, 104') is acquired;
- in the sample (110) in which the concentration of the analyte (117) is to be determined, both the reaction of the test chemistry (102) with the analyte (117) and the diffusion of the label (104, 104') is detected;
- the piece of correction information is determined from the detected diffusion of the label (104, 104') in the sample (110) and from the general relationship between the interfering variable and the diffusion of the label (104, 104').

## Revendications

1. Procédé de détermination d'au moins une concentration d'au moins un analyte (117) dans un échantillon (110), notamment d'au moins un métabolite dans un échantillon (110) d'un liquide corporel, au moins un élément d'essai (50) présentant au moins une chimie d'essai (102) étant utilisé, au moins une réaction de la chimie d'essai (102) avec l'analyte (117) étant détectée, au moins un marqueur (104, 104') étant en outre utilisé, le marqueur (104, 104') étant différent de l'analyte (117), le marqueur (104, 104') comprenant au moins un composé chimique, au moins une diffusion du marqueur (104, 104') dans l'échantillon (110) ou au moins une partie de l'échantillon (110) étant détectée, la détection de la réaction de l'analyte (117) ayant lieu de manière électrochimique et/ou optique, et/ou en ce que la détection de la diffusion du marqueur (104, 104') a lieu de manière électrochimique et/ou optique, au moins une information de correction étant générée à partir de la diffusion, la concentration de l'analyte (117) étant déterminée à partir de la réaction de la chimie d'essai (102) avec l'analyte (117) en prenant en compte l'information de correction, l'information de correction étant conçue pour prendre en compte un effet de la température de l'échantillon (110) et/ou de l'élément d'essai (50) lors de la détermination de la concentration de l'analyte (117), l'information de correction comprenant au moins une information relative à au moins une grandeur de perturbation de l'échantillon (110), la grandeur de perturbation comprenant une température de l'échantillon (110) et/ou de l'élément d'essai (50), l'information de correction étant déterminée en utilisant les étapes de procédé suivantes :

- lors d'au moins une mesure d'étalonnage, une relation générale entre la grandeur de perturbation et la diffusion du marqueur (104, 104') est mesurée ;
- dans l'échantillon (110) dans lequel la concentration de l'analyte (117) doit être déterminée, aussi bien la réaction de la chimie d'essai (102) avec l'analyte (117) que la diffusion du marqueur (104, 104') sont détectées ;
- l'information de correction est déterminée à partir de la diffusion détectée du marqueur (104, 104') dans

l'échantillon (110) et de la relation générale entre la grandeur de perturbation et la diffusion du marqueur (104, 104').

2. Procédé selon la revendication précédente, **caractérisé en ce que** l'élément d'essai (50) comprend au moins un champ d'essai (100) comprenant au moins une couche de chimie d'essai (118), la couche de chimie d'essai (118) comprenant la chimie d'essai (102), la chimie d'essai (102) contenant notamment au moins une enzyme, notamment choisie dans le groupe constitué par : les glucose-déshydrogénases, les glucose-oxydases.

3. Procédé selon l'une quelconque des revendications précédentes, **caractérisé en ce que** le marqueur (104, 104') est contenu dans au moins une première zone (114) de l'élément d'essai (50), la chimie d'essai (102) étant également contenue en totalité ou en partie dans la première zone (114).

4. Procédé selon l'une quelconque des revendications précédentes, **caractérisé en ce que** le marqueur (104, 104') est agencé en totalité ou en partie séparément de la chimie d'essai (102).

5. Procédé selon l'une quelconque des revendications précédentes, **caractérisé en ce que** le marqueur (104, 104') ne réagit pas avec l'analyte (117) et/ou avec la chimie d'essai (102), une vitesse de diffusion du marqueur (104, 104') étant essentiellement indépendante de la concentration de l'analyte (117).

6. Procédé selon l'une quelconque des revendications précédentes, **caractérisé en ce que** le marqueur (104, 104') comprend au moins un colorant détectable optiquement, le marqueur comprenant au moins un colorant qui est hydrophile ou soluble dans l'eau.

7. Procédé selon l'une quelconque des revendications précédentes, **caractérisé en ce que** le marqueur (104, 104') absorbe la lumière dans une plage de longueur d'onde allant de 300 à 800 nm.

8. Procédé selon l'une quelconque des revendications précédentes, **caractérisé en ce que** le marqueur (104, 104') est un colorant, le colorant étant choisi dans le groupe constitué par : les colorants cyanine, les colorants azoïques et les colorants sulfones ou au moins deux d'entre eux.

9. Procédé selon l'une quelconque des revendications précédentes, **caractérisé en ce que** le marqueur (104, 104') est choisi dans le groupe constitué par : l'érioglaucine, le carmin d'indigo, le bleu d'hydroxynaphtol, l'iodure de 1,1-diéthyl-4,4-carbocyanine et l'amaranthe, de préférence l'érioglaucine ou le bleu d'hydroxynaphtol, ou au moins deux d'entre eux.

10. Procédé selon l'une quelconque des revendications précédentes, **caractérisé en ce qu'**au moins un premier marqueur (104, 104') et au moins un autre marqueur (104, 104') différent du premier marqueur (104, 104') sont utilisés, la vitesse de diffusion du premier marqueur (104, 104') étant influencée par au moins une première propriété de l'échantillon (110), et la vitesse de diffusion dudit au moins un autre marqueur (104, 104') étant influencée par au moins une autre propriété de l'échantillon (110) différente de la première propriété, la propriété de l'échantillon (110) comprenant une température de l'échantillon (110).

11. Procédé selon l'une quelconque des revendications précédentes, **caractérisé en ce que** la réaction de la chimie d'essai (102) avec l'analyte (117) est détectée au moyen d'au moins un premier détecteur (170), la diffusion du marqueur (104, 104') étant détectée au moyen d'au moins un autre détecteur (180).

12. Dispositif (190) pour la détermination d'au moins une concentration d'au moins un analyte (117) dans un échantillon (110), notamment en utilisant un procédé selon l'une quelconque des revendications précédentes, le dispositif (190) comprenant au moins un élément d'essai (50) comprenant au moins une chimie d'essai (102), le dispositif (190) étant conçu pour détecter au moins une réaction de la chimie d'essai (102) avec l'analyte (117), le dispositif (190) comprenant au moins un marqueur (104, 104'), le marqueur (104, 104') étant différent de l'analyte (117), le marqueur (104, 104') comprenant au moins un composé chimique, le dispositif (190) étant conçu pour détecter au moins une diffusion du marqueur (104, 104') dans l'échantillon (110) ou au moins un constituant de l'échantillon (110), la détection de la réaction de l'analyte (117) ayant lieu de manière électrochimique et/ou optique, et/ou en ce que la détection de la diffusion du marqueur (104, 104') a lieu de manière électrochimique et/ou optique, le dispositif (190) étant conçu pour générer au moins une information de correction à partir de la diffusion du marqueur (104, 104'), le dispositif (190) étant en outre conçu pour déterminer la concentration de l'analyte (117) à partir de la réaction de la chimie d'essai (102) avec l'analyte (117) en prenant en compte l'information de correction, l'information de correction étant conçue pour prendre en compte un effet de la température de l'échantillon (110) et/ou de l'élément d'essai (50) lors de la détermination de la concentration de l'analyte (117), l'information de correction comprenant au moins une information relative à au moins une grandeur de perturbation de l'échantillon (110), la grandeur de perturbation comprenant une température de l'échantillon (110) et/ou de l'élément d'essai (50), le dispositif (190) étant conçu pour déterminer l'information de correction en utilisant les étapes de procédé suivantes :

- lors d'au moins une mesure d'étalonnage, une relation générale entre la grandeur de perturbation et la diffusion du marqueur (104, 104') est mesurée ;
- dans l'échantillon (110) dans lequel la concentration de l'analyte (117) doit être déterminée, aussi bien la réaction de la chimie d'essai (102) avec l'analyte (117) que la diffusion du marqueur (104, 104') sont détectées ;
- l'information de correction est déterminée à partir de la diffusion détectée du marqueur (104, 104') dans l'échantillon (110) et de la relation générale entre la grandeur de perturbation et la diffusion du marqueur (104, 104').

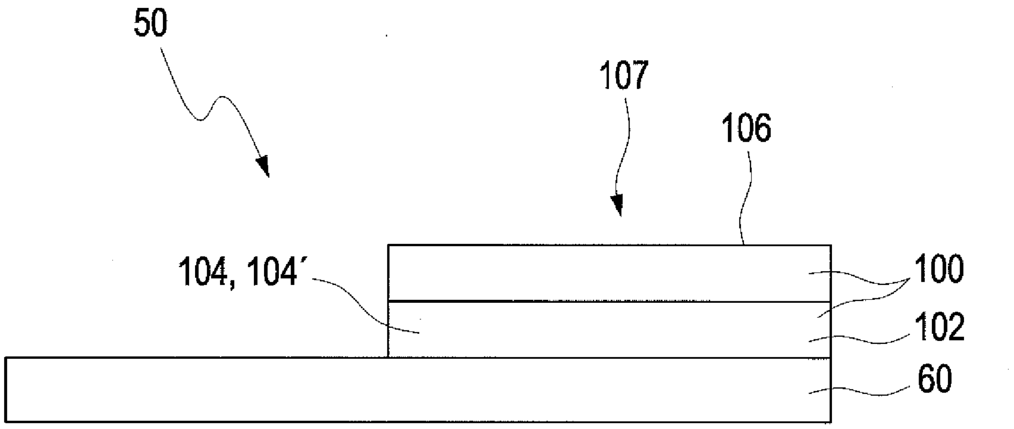
50
107
106
104, 104´
100
102
60

Fig. 1 a

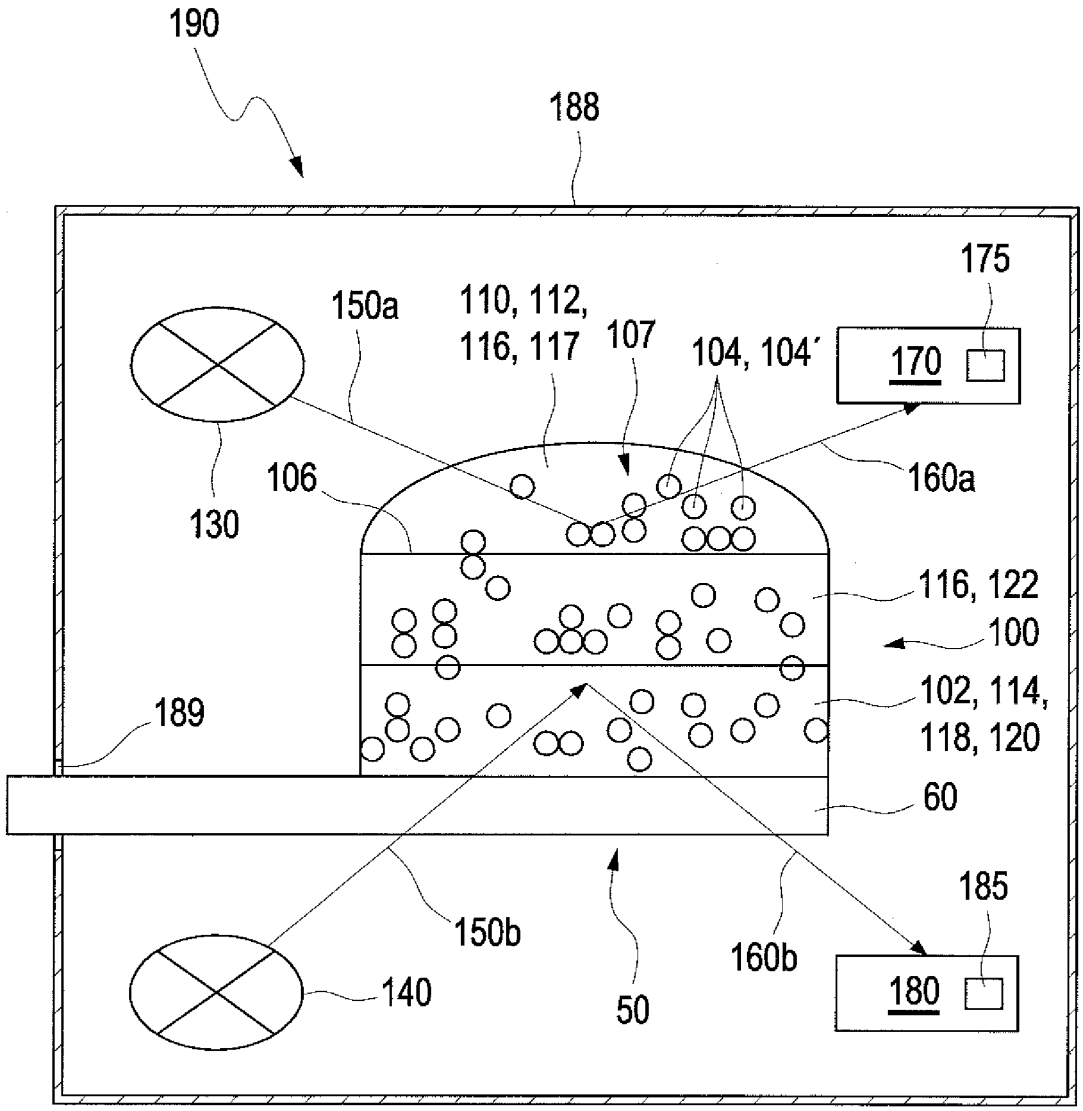
190
188
175
150a
110, 112,
116, 117
107
104, 104´
170
160a
106
130
116, 122
100
102, 114,
118, 120
189
60
185
150b
160b
140
50
180

Fig. 1 d

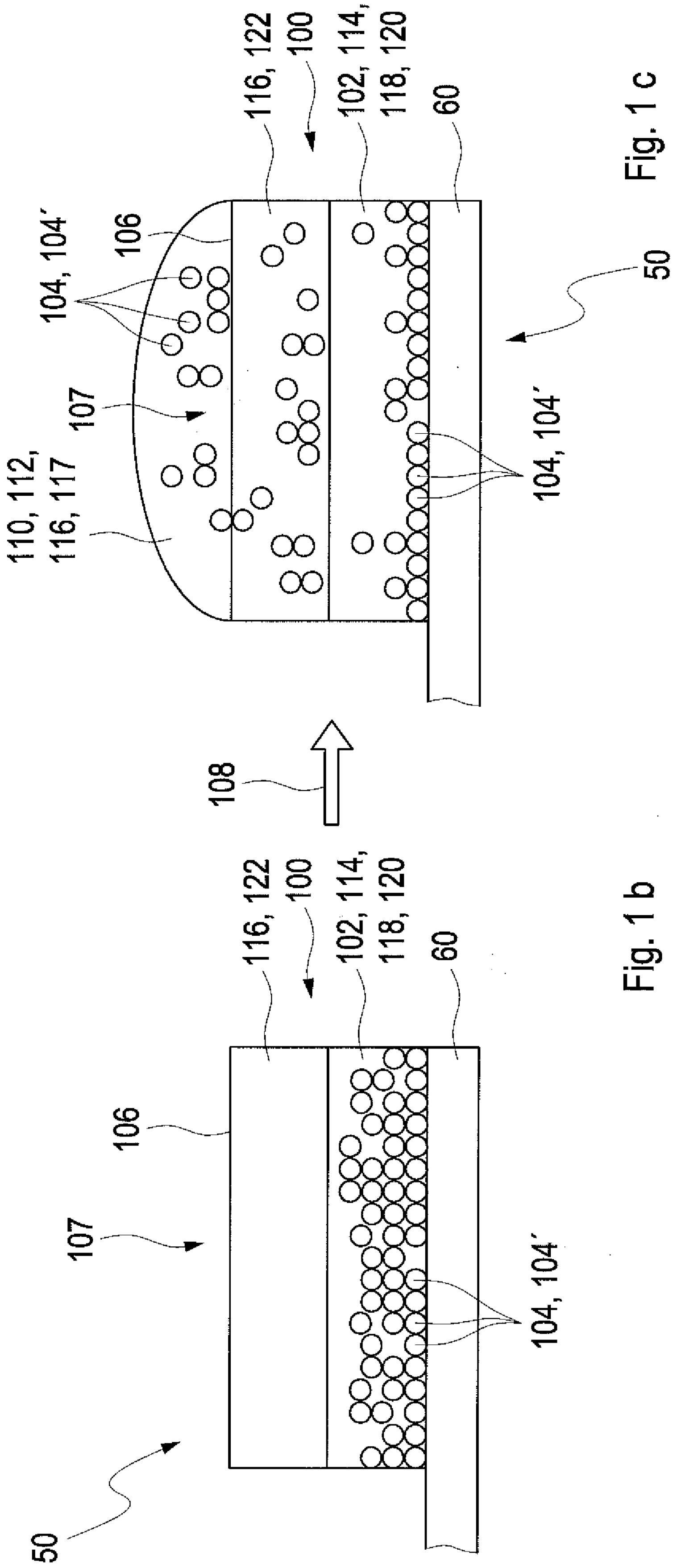
116, 122
100
102, 114,
118, 120
60
104, 104´
106
104, 104´
107
110, 112,
116, 117
50
104, 104´
108
116, 122
100
102, 114,
118, 120
60
106
107
104, 104´
50

Fig. 1 c

Fig. 1 b

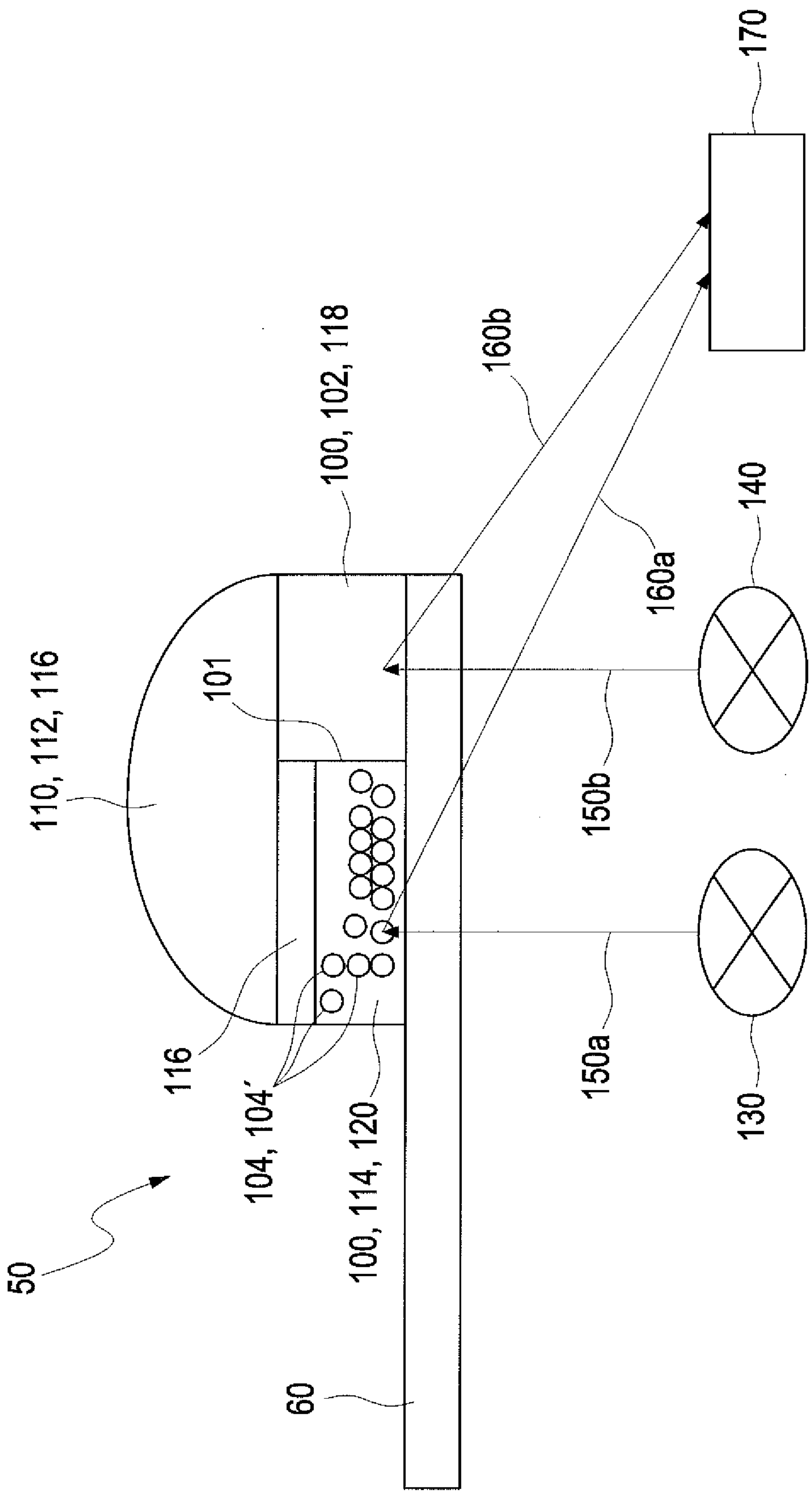
170
160b
100, 102, 118
110, 112, 116
101
160a
140
150b
116
104, 104′
100, 114, 120
150a
130
50
60

Fig. 1 e

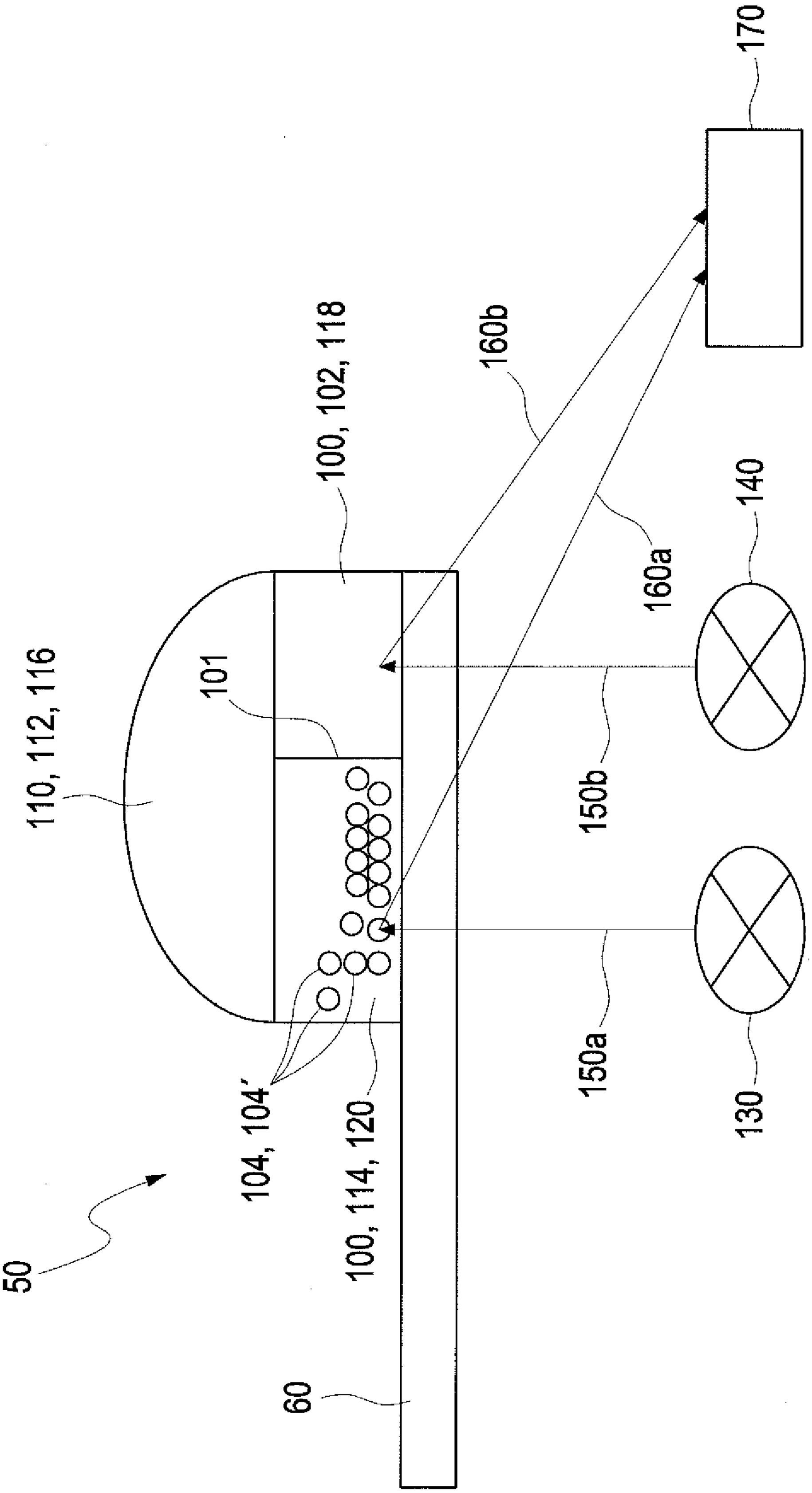
50
60
100, 114, 120
104, 104'
110, 112, 116
101
100, 102, 118
160b
160a
150b
150a
130
140
170

Fig. 1 f

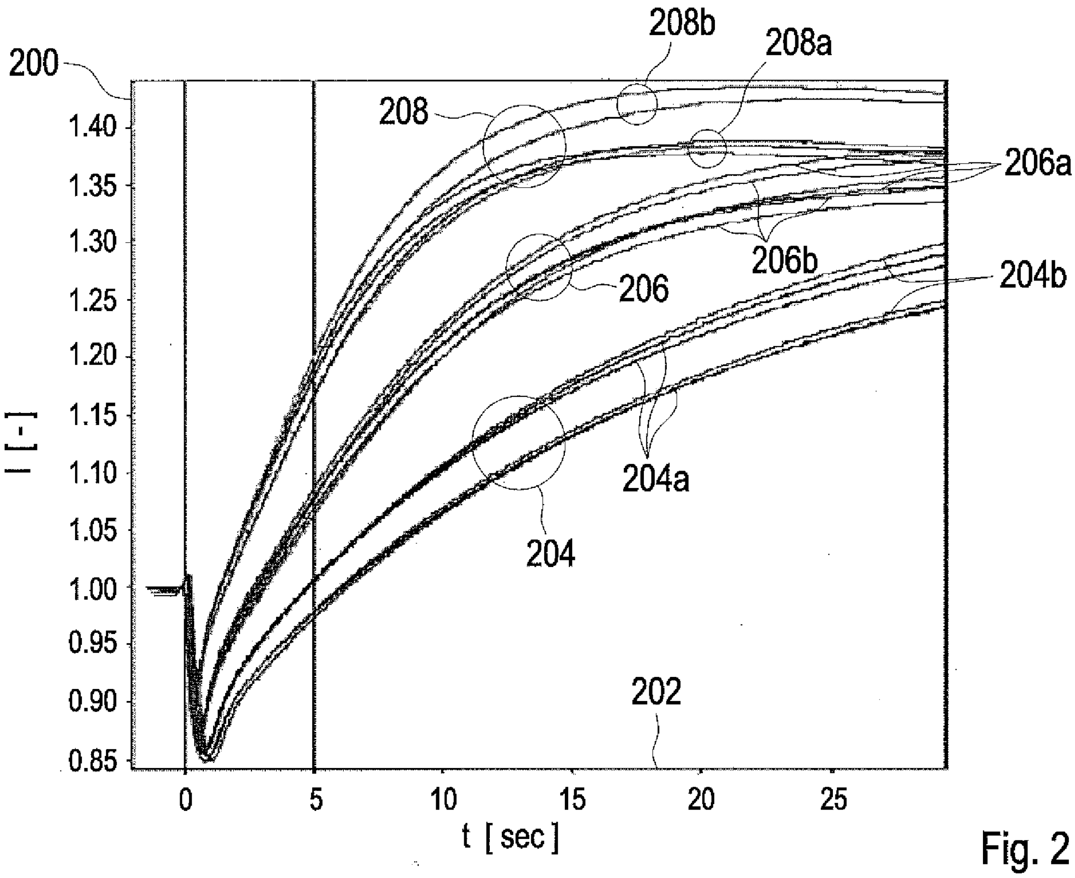
200
208b
208a
208
206a
206b
206
204b
204a
204
202
1.40
1.35
1.30
1.25
1.20
1.15
1.10
1.05
1.00
0.95
0.90
0.85
I [-]
0
5
10
15
20
25
t [sec]

Fig. 2

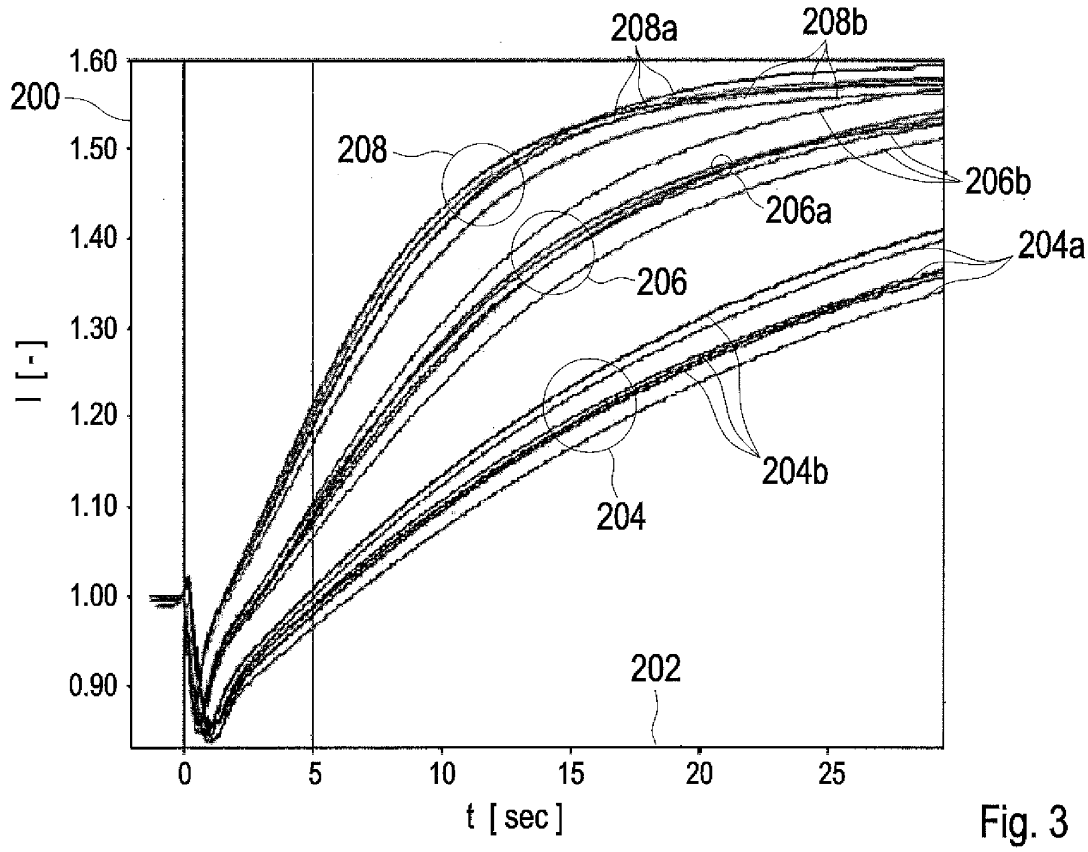
208a
208b
200
208
206b
206a
206
204a
204b
204
202
1.60
1.50
1.40
1.30
1.20
1.10
1.00
0.90
I [-]
0
5
10
15
20
25
t [sec]

Fig. 3

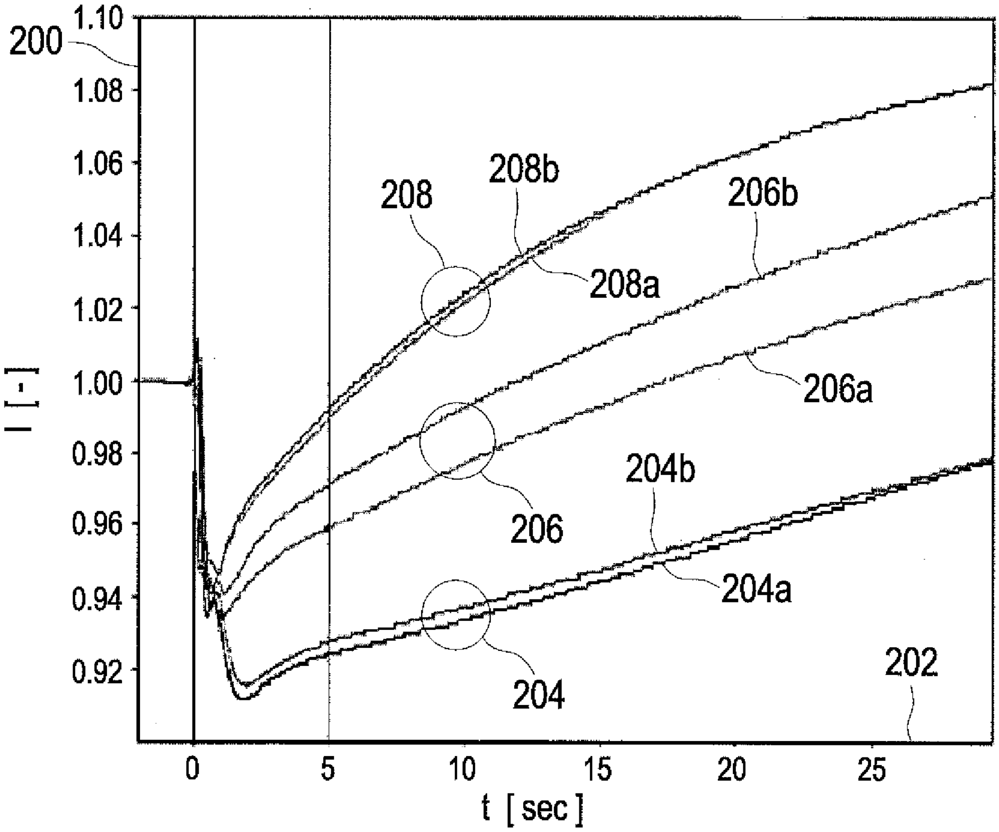
200
1.10
1.08
1.06
1.04
1.02
1.00
0.98
0.96
0.94
0.92
I [-]
208
208b
208a
206b
206a
206
204b
204a
204
202
0
5
10
15
20
25
t [sec]

Fig. 4

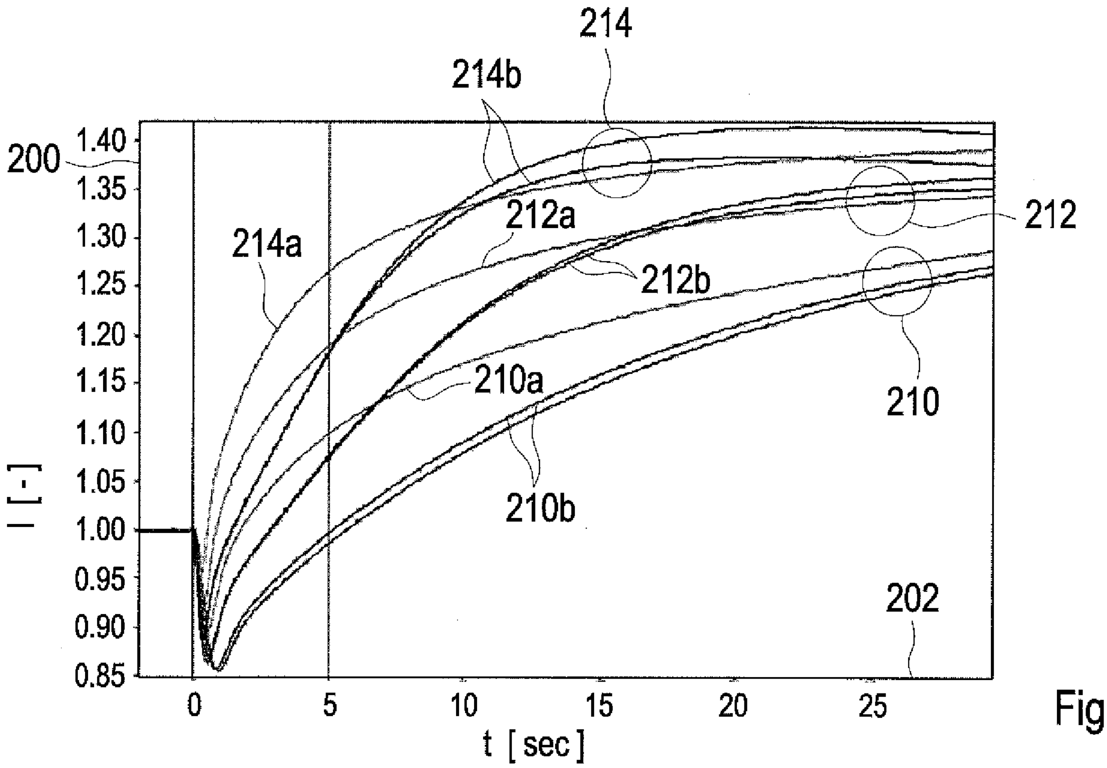
214
214b
200
1.40
1.35
1.30
1.25
1.20
1.15
1.10
1.05
1.00
0.95
0.90
0.85
I [-]
214a
212a
212
212b
210a
210
210b
202
0
5
10
15
20
25
t [sec]

Fig. 5

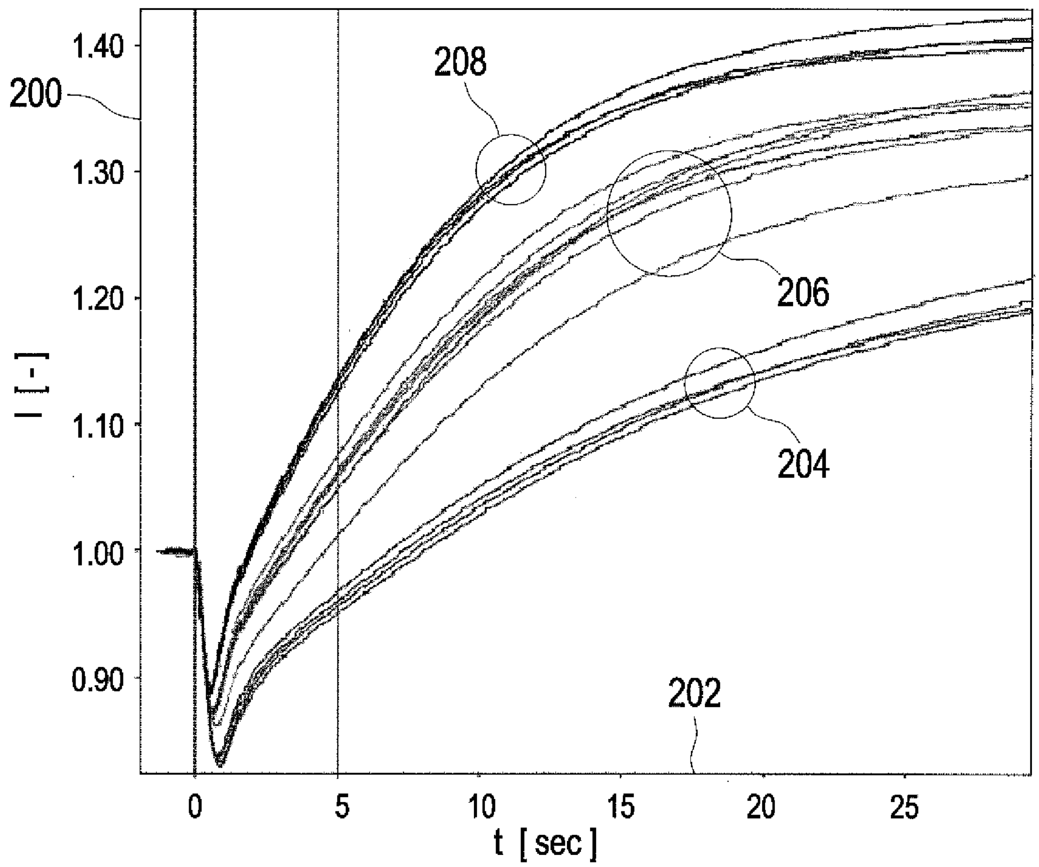
1.40
1.30
1.20
1.10
1.00
0.90
I [-]
200
208
206
204
202
0
5
10
15
20
25
t [sec]

Fig. 6

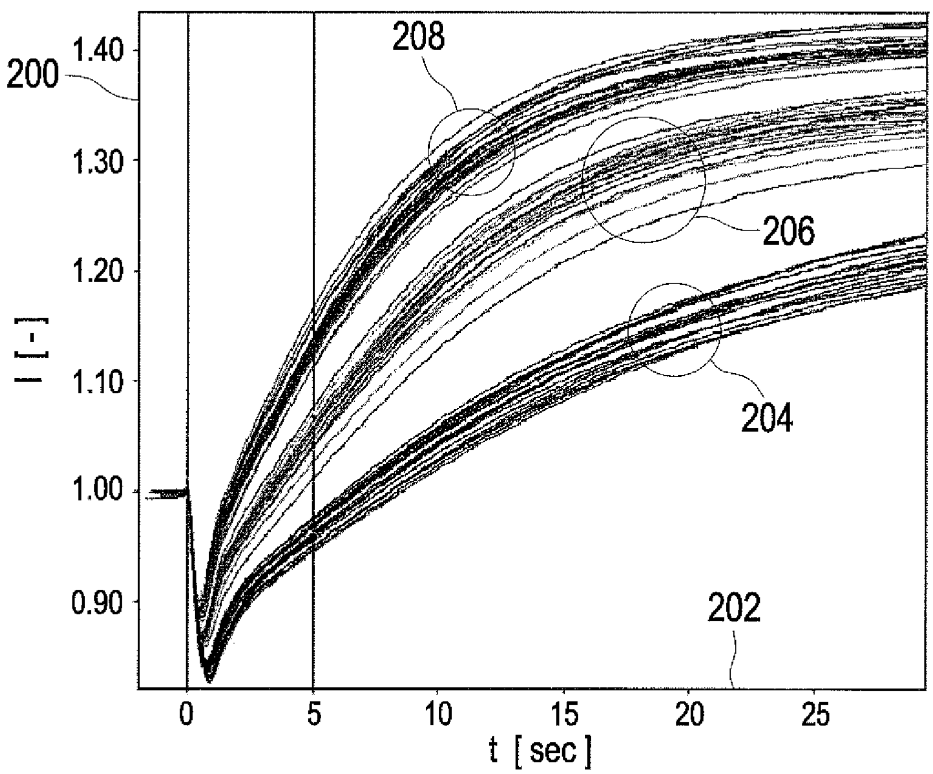
1.40
1.30
1.20
1.10
1.00
0.90
I [-]
200
208
206
204
202
0
5
10
15
20
25
t [sec]

Fig. 7

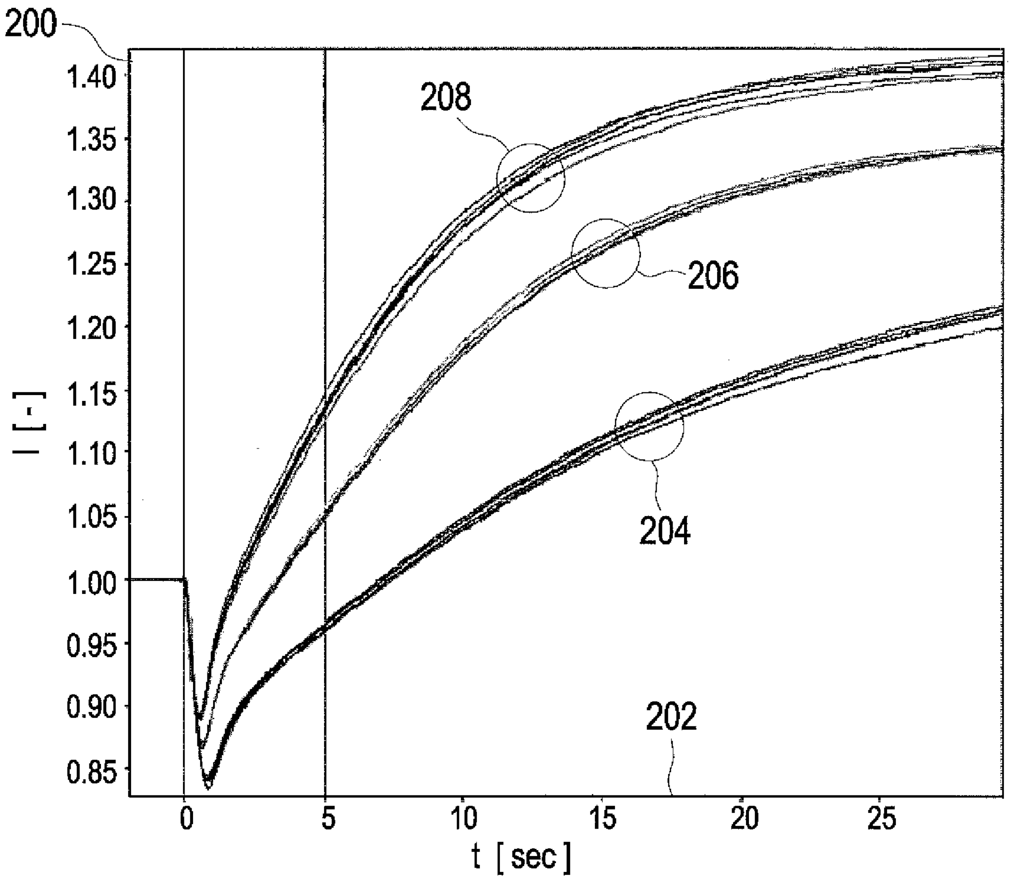
200
1.40
1.35
1.30
1.25
1.20
1.15
1.10
1.05
1.00
0.95
0.90
0.85
I [-]
208
206
204
202
0
5
10
15
20
25
t [ sec ]

Fig. 8

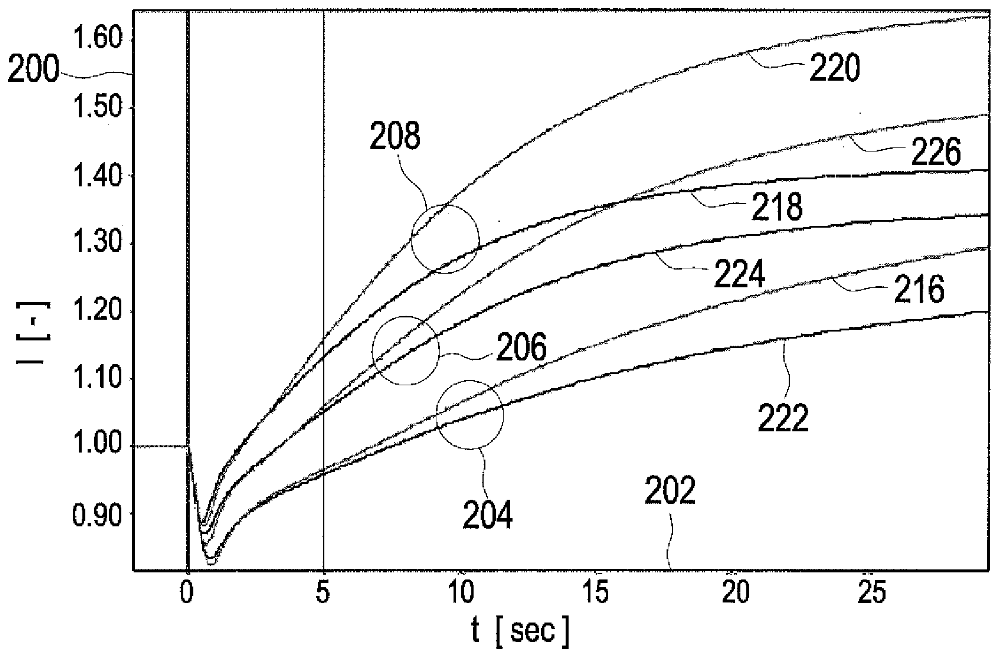
1.60
200
1.50
1.40
1.30
1.20
1.10
1.00
0.90
I [-]
220
226
208
218
224
216
206
222
204
202
0
5
10
15
20
25
t [ sec ]

Fig. 9

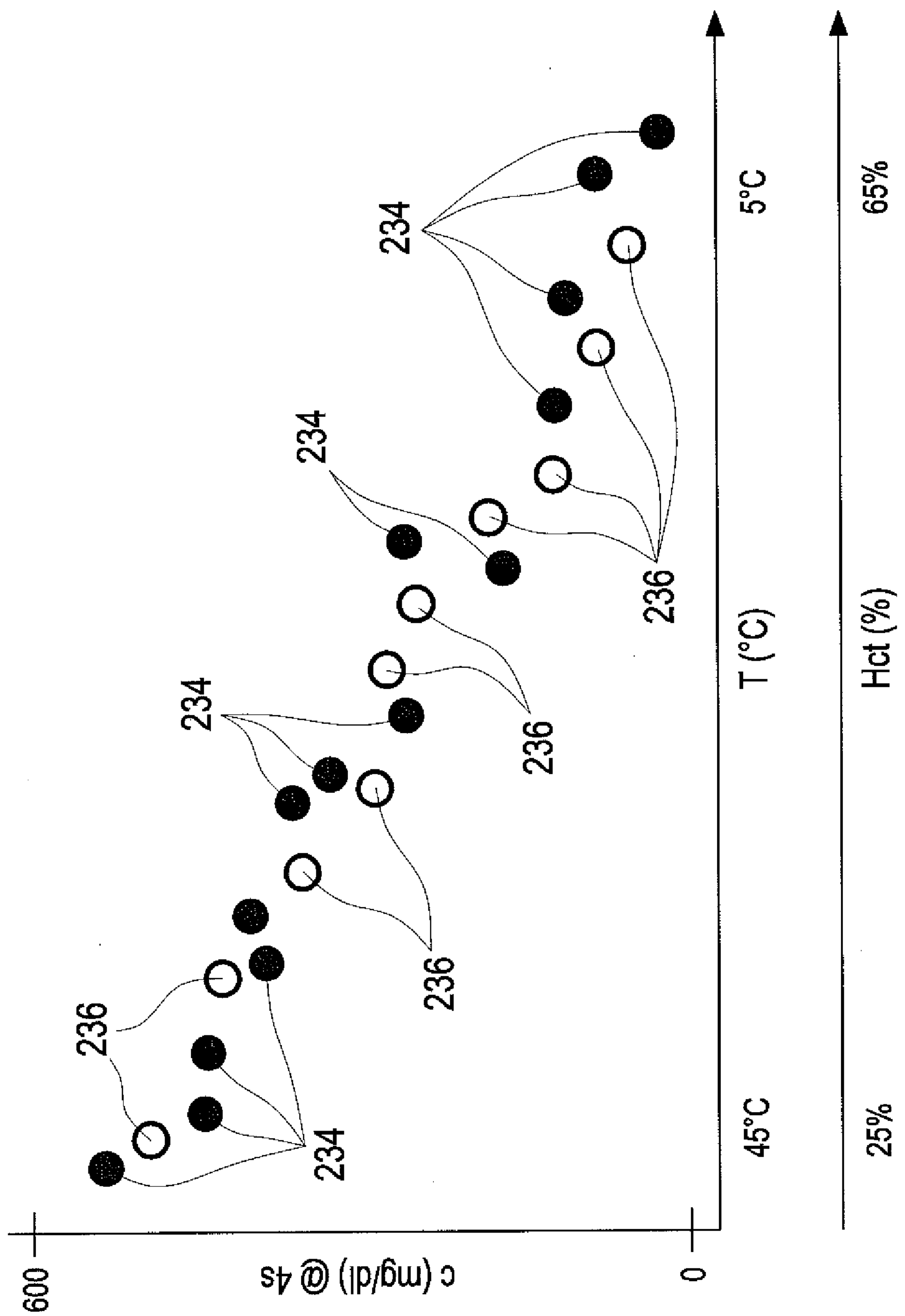


Fig. 10

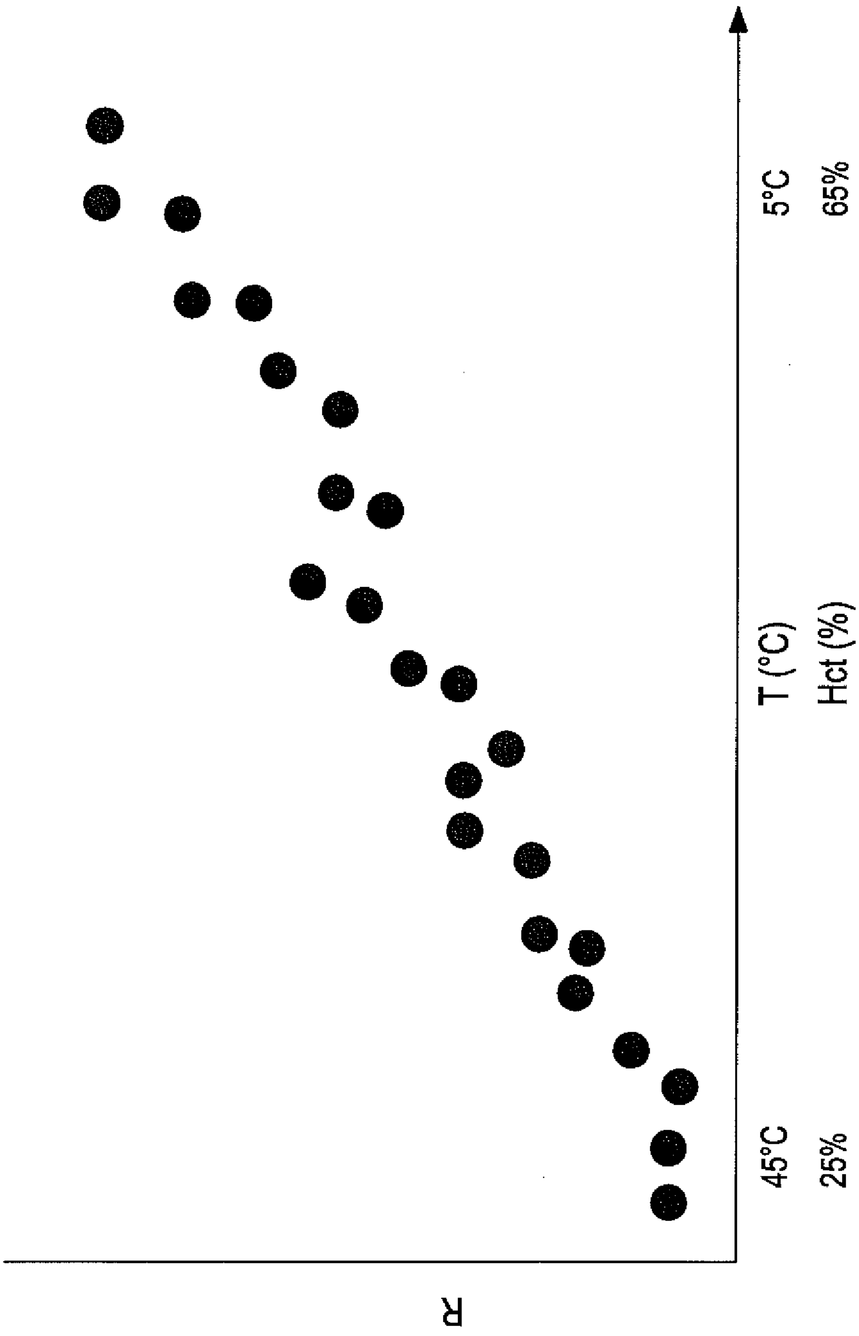


Fig. 11

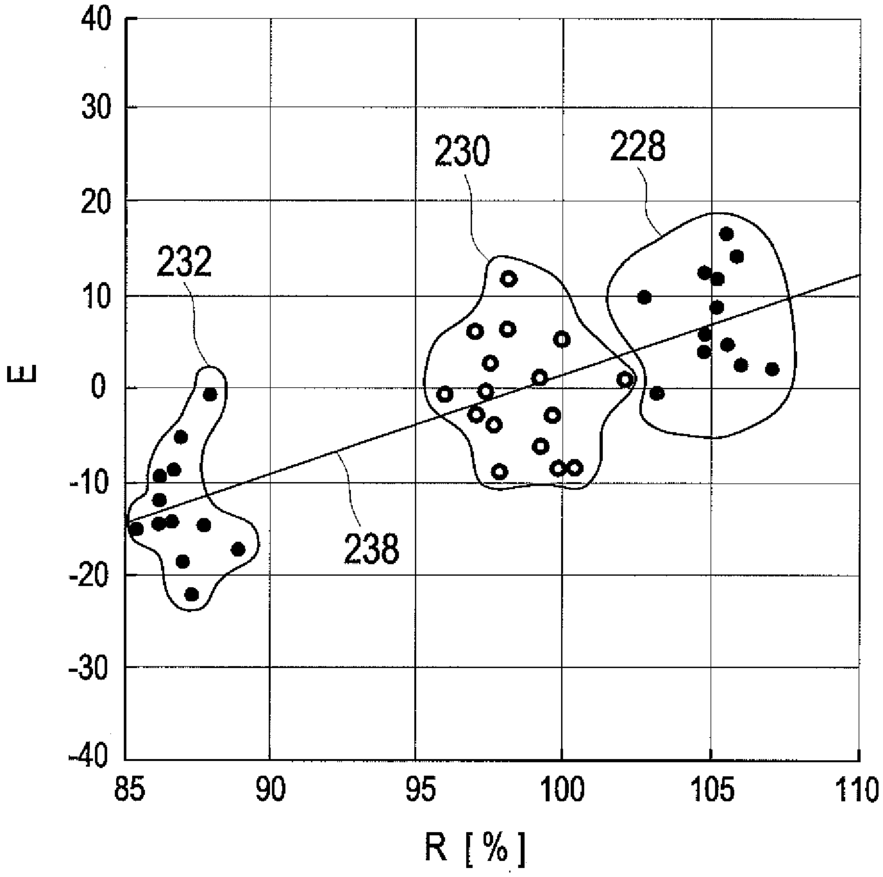


Fig. 12

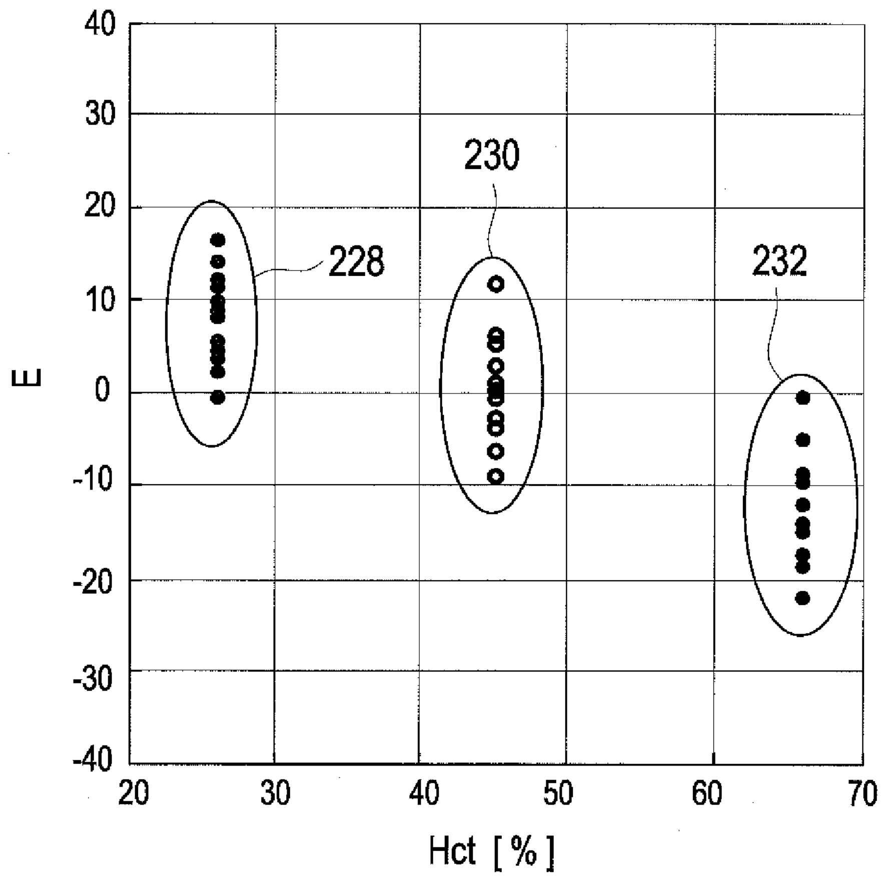


Fig. 13

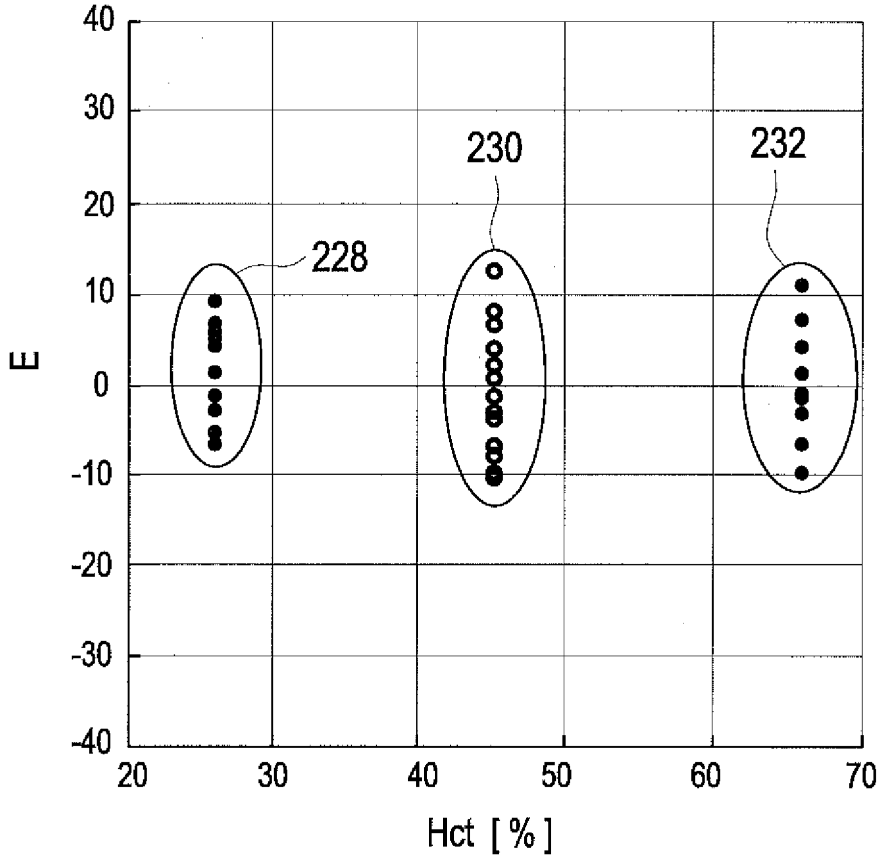


Fig. 14

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2010052306 A
- WO 2010052307 A
- US 4250257 A
- US 7548773 B
- EP 0821234 B1